(19) Europäisches Patentamt
European Patent Office
Office européen des brevets

(11) EP 3 052 647 B1

(12) **EUROPEAN PATENT SPECIFICATION**

(45) Date of publication and mention of the grant of the patent:
**11.07.2018 Bulletin 2018/28**

(21) Application number: **14781645.8**

(22) Date of filing: **03.10.2014**

(51) Int Cl.:
***C12Q 1/68*** *(2018.01)*

(86) International application number:
**PCT/GB2014/052994**

(87) International publication number:
**WO 2015/049537 (09.04.2015 Gazette 2015/14)**

# (54) CANCER BIOMARKERS AND USES THEREOF

KREBSBIOMARKER UND VERWENDUNGEN DAVON

BIOMARQUEURS DE CANCER ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.10.2013 GB 201317622**

(43) Date of publication of application:
**10.08.2016 Bulletin 2016/32**

(73) Proprietors:
- **F-Star Biotechnology Ltd.**
  **Cambridge, Cambridgeshire CB22 3AT (GB)**
- **F-Star Biotechnologische Forschungs- und Entwicklungsges.m.b.H**
  **1030 Vienna (AT)**

(72) Inventors:
- **LEUNG, Kinmei**
  **Cambridge**
  **Cambridgeshire CB22 3AT (GB)**
- **SUN, Haijun**
  **Cambridge**
  **Cambridgeshire CB22 3AT (GB)**
- **BATEY, Sarah**
  **Cambridge**
  **Cambridgeshire CB22 3AT (GB)**
- **ROWLAND, Robert**
  **Cambridge**
  **Cambridgeshire CB22 3AT (GB)**

(74) Representative: **Keirstead, Tanis Evelyne et al**
**Mewburn Ellis LLP**
**City Tower**
**40 Basinghall Street**
**London EC2V 5DE (GB)**

(56) References cited:
**WO-A1-2009/132876 WO-A1-2012/007167**

- **ESTEVA FRANCISCO J ET AL: "Molecular predictors of response to trastuzumab and lapatinib in breast cancer", NATURE REVIEWS. CLINICAL ONCOLOGY,, vol. 7, no. 2, 1 February 2010 (2010-02-01), pages 98-107, XP009130024,**
- **LEI WANG ET AL: "Retargeting T Cells for HER2-Positive Tumor Killing by a Bispecific Fv-Fc Antibody", PLOS ONE, vol. 8, no. 9, 23 September 2013 (2013-09-23), page e75589, XP055160755, DOI: 10.1371/journal.pone.0075589**
- **WOZNIAK-KNOPP G ET AL: "Introducing antigen-binding sites in structural loops of immunoglobulin constant domains: Fc fragments with engineered HER2/neu-binding sites and antibody properties", PROTEIN ENGINEERING, DESIGN AND SELECTION, OXFORD JOURNAL, LONDON, GB, vol. 23, no. 4, 1 April 2010 (2010-04-01), pages 289-297, XP009137665, ISSN: 1741-0126, DOI: 10.1093/PROTEIN/GZQ005 [retrieved on 2010-02-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **"F-star Alpha: A new asset centric company", , 11 February 2014 (2014-02-11), XP055160760, Retrieved from the Internet: URL:http://www.onenucleus.com/media/Events/LSLS/11%20feb%202014/Jane%20Dancer.pdf [retrieved on 2015-01-08]**

## Description

### Field of the invention

The present invention relates to the use of Human Epidermal Growth Factor Receptor 2 (HER2) gene copy number (GCN) and HER2 mRNA levels as biomarkers. Specifically, the present invention relates to the use of HER2 gene copy number and HER2 mRNA levels as biomarkers to identify cancers which will respond to treatment with a specific binding member comprising a HER2 antigen binding site engineered into a structural loop region of a constant domain, e.g. CH3 domain, of the specific binding member, and specific binding members which compete with such a binding member for binding to HER2. The present invention also relates to specific binding members comprising a HER2 antigen binding site engineered into a structural loop region of a CH3 domain of the specific binding member and uses thereof.

### Background to the invention

Human Epidermal Growth Factor Receptor 2 (also referred to as HER2, HER2/neu or ErbB-2) is an 185kDa cytoplasmic transmembrane tyrosine kinase receptor. It is encoded by the c-erbB-2 gene located on the long arm of chromosome 17q and is a member of the HER family (Ross *et al.,* 2003). The HER family normally regulates cell growth and survival, as well as adhesion, migration, differentiation, and other cellular responses (Hudis, C, 2007). Overexpression and amplification of HER2 is observed in the development of a variety of solid cancers including breast (Yarden, Y, 2001), gastric (Gravalos *et al.,* 2008), stomach (Rüschoff et al., 2010), colorectal (Ochs *et al.,* 2004), ovarian (Lanitis *et al.,* 2012), pancreatic (Lei *et al.,* 1995), endometrial (Berchuk *et al.,* 1991) and non-small cell lung cancers (Brabender *et al.,* 2001).

Breast, colorectal, and gastric cancers accounted for 30% of all diagnosed cancer cases and 24% of all cancer deaths in 2008 (CRUK and WHO World Cancer Report). Breast cancer, in particular, is a leading cause of cancer death among women. HER2 is overexpressed or amplified in 15 to 30% of breast cancers and is associated with poor prognosis, shorter periods of disease-free and overall survival, as well as a more aggressive cancer phenotype (Vinatzer *et al.,* 2005). In breast cancer, about 20% of patients will develop tumours that harbour a genomic alteration involving the amplification of an amplicon on chromosome 17 that contains the HER2 proto-oncogene (Ross, J, 2009; and Hicks *et al.,* 2005). Such tumours represent a more aggressive subtype of breast cancer that over-contributes to the mortality of the disease (Hudis C, 2007).

A number of HER2 targeting therapies have been approved for treatment of HER2 positive tumours.

Herceptin™ (trastuzumab) is approved for the treatment of metastatic breast cancer in combination with Taxol™ (paclitaxel) and alone for the treatment of HER2 positive breast cancer in patients who have received one or more chemotherapy courses for metastatic disease. As trastuzumab also enhances the efficacy of adjuvant chemotherapy (paclitaxel, docetaxel and vinorelbine) in operable or locally advanced HER2 positive tumours, it is considered standard of care for patients with early or advanced stages of HER2-overexpressing breast cancer. Trastuzumab has also been approved for treatment of HER2 positive metastatic cancer of the stomach or gastroesophageal junction cancer, in combination with chemotherapy (cisplatin and either capecitabine or 5-fluorouracil) in patients who have not received prior treatment for their metastatic disease (Genentech, About Herceptin. [online] Available at: http://www.herceptin.com/about [accessed on 17 September 2013]).

Perjeta™ (pertuzumab) has also been approved for the treatment of HER2 positive metastatic breast cancer in combination with trastuzumab and docetaxel (Genentech, PERJETA Can Help Strengthen Your Treatment. [online] Available at: http://www.perjeta.com/patient/about [accessed on 17 September 2013]). Pertuzumab targets a different domain of HER2 and has a different mechanism of action than trastuzumab. Specifically, pertuzumab is a HER2 dimerisation inhibitor, which prevents HER2 from pairing with other HER receptors (EGFR/HER1, HER3 and HER4). Kadcyla™ (ado-trastuzumab emtansine, T-DM1) is an antibody-drug conjugate, which comprises trastuzumab linked to the cytotoxic agent mertansine (DM1), which disrupts the assembly of microtubules in dividing cells resulting in cell death, and is approved for the treatment of metastatic breast cancer in patients who have received prior treatment with trastuzumab and a taxane chemotherapy (Genentech, How Kadcyla™ (ado-trastuzumab emtansine) is Believed to Work (Proposed Mechanism of Action). [online] Available at: http://www.gene.com/media/product-information/kadcyla-moa [accessed on 17 September 2013]).

Tykerb™ (lapatinib) is a small molecule kinase inhibitor that blocks the catalytic action of both HER2 and EGFR. It has been approved in combination with Femara™ (letrozole) for treatment of HER2 positive, hormone receptor positive, metastatic breast cancer in postmenopausal women, and in combination with Xeloda™ (capecitabine) for the treatment of advanced or metastatic HER2 positive breast cancer in patients who have received prior therapy including an anthracycline, a taxane, and Herceptin™ (U.S. Food and Drug Administration, Highlights of Prescribing Information. [online] Available at: http://www.accessdata.fda.gov/drugsatfda_docs/label/2010/022059s007lbl.pdf [accessed on 28 September 2013]). Further drugs are in clinical development.

Although the approved HER2-specific therapies have improved the standard of care of HER2-positive breast and gastric cancers, a significant unmet medical need exists due to intrinsic or acquired resistance to these drugs. Despite trastuzumab's standard of care status for HER2 positive breast cancer, 20-50% of patients from adjuvant settings and around 70% of patients from monotherapy settings go on to develop resistance to trastuzumab (Wolff *et al.,* 2007; and Harris *et al.,* 2007).

There is an emerging trend in cancer therapy towards the selection of patients for treatment based on the assessment of underlying genetic or molecular mechanisms of cancer, as biomarkers. Diagnostic tests based on biomarkers found to be relevant in particular cancers have been approved by the FDA to identify patients susceptible to treatment with specific cancer therapies. By May 2013, 15 such diagnostic tests, also known as companion diagnostics, had been approved by the FDA (Genetic Engineering and Biotechnology News, 2013, Companion Diagnostics: 52 Pick-Up. [online] Available at: http://www.genengnews.com/insight-and-intelligenceand153/companion-diagnostics-52-pick-up/77899813/ [accessed on 28 September 2013]) and various others are in development. For example, the therascreen™ KRAS test is an EGFR immunohistochemistry test which identifies patients having EGFR positive metastatic colorectal cancer with wild-type KRAS genes to be treated with Erbitux™ (cetuximab). The DAKO C-kit PharmDx immunohistochemistry test identifies patients with c-kit positive gastrointestinal stromal tumours susceptible to treatment with Gleevec (imatinib). A number of diagnostic tests have also been approved for identification of HER2 positive tumours for treatment with Herceptin™ (trastuzumab) (Hamburg and Collins, 2010), such as the immunohistochemistry test Herceptest™ and Her2 FISH PharmDx Kit™, which are commonly used together. Further commercially available kits for immunohistochemistry of HER2 positive tumours include Oracle (Leica Biosystems) and Pathway (Ventana). Preclinical and clinical research efforts to identify biomarkers predictive of the clinical response to treatment also have the potential to identify additional patients with "non-traditional" HER2+ cancers, including colorectal cancer, ovarian cancer and others, which are likely to benefit from HER2 targeting therapies (Gun *et al.,* 2013).

As a result of the aggressive nature of tumours which over-express HER2 or amplify the HER2 gene, the HER2 amplification status has become an increasingly important predictor of disease outcome and is widely tested for. HER2 positive tumours which may be susceptible to treatment with a HER2 specific biological therapeutic are usually identified in the first instance by using immunohistochemistry (IHC). Samples from patients are assessed in a semi-quantitative manner using a scoring system which reflects the intensity of staining alongside the percentage of stained tumour cells in the sample. The level of HER2 expression or over-expression is measured and a score is attributed whereby a strong complete membrane staining is given a score of 3+ and is considered strongly positive or unequivocal. A weak to moderate complete membrane staining is observed in greater than 10% of tumour cells is attributed a score of 2+ and is considered weakly positive or equivocal. Where the membrane staining is faint, which is the case in less than 10% of tumour cells, or is incomplete, the sample is attributed a score of 1+/0 and is considered HER2 negative. Those tumours which score 3+ in the IHC analysis, i.e. where there is strong overexpression of the HER2 protein on the tumour cell surface, are reported to a clinician for possible treatment with a HER2 specific therapy.

As IHC is only semi-quantitative, and measures cell surface over-expression of the HER2 protein, it can be inexact and does not always provide an accurate reading. In particular, it can be difficult to correctly identify tumours which are weakly positive for HER2 expression by IHC, i.e. tumours which achieve a 2+ score in the analysis. For clarification of the HER2 status of such tumours, samples are subjected to further testing, for example by Fluorescence In Situ Hybridisation (FISH), in order to determine the level of HER2 gene amplification in the tumour sample. Various methods are known for performing FISH. In methods where the HER2 gene copy number is compared to an internal control such as CEP17, where the FISH score is less than two, in other words the HER2/CEP17 ratio is less than 2, the HER2 gene status is said to be non-amplified and the tumour is considered HER2 negative. Where the FISH score is equal to or greater than 2, the HER2 gene status is said to be amplified, and the tumour is considered HER2 positive and is reported to a clinician for possible treatment with a HER2 specific therapy.

However, despite these multiple layers of testing for HER2 positive tumours, not all tumours identified as HER2 positive are responsive to current HER2 specific therapies. Therefore, there remains a need in the art for treatments for patients with HER2 positive tumours, as well as means for identifying patients likely to benefit from such therapies. In addition, as mentioned above, acquired resistance to known therapies for HER2, such as trastuzumab, means that there is a need in the art for therapies suitable for treating such resistant HER2 positive cancers.

Antigen-binding Fc fragments (also referred to as Fcab™ [Fc fragment with Antigen Binding]) comprising e.g. a modified IgG1 Fc domain which binds to HER2 with high affinity are described in WO 2009/132876 A1 and WO 2009/000006 A1. Such Fcabs have been shown to have favourable properties, such as half-lives of approximately 60 hours in mice.

## Statements and detailed description of the invention

The present inventors have discovered that a specific binding member comprising a HER2 antigen binding site engineered into a structural loop region of a constant domain, e.g. CH3 domain, of the specific binding member can be used to treat cancers with a HER2 gene copy number of greater than or equal to 10 per tumour cell. The present inventors have also discovered that such specific binding members can be used to treat cancers with a high HER2 mRNA levels. In particular, such specific binding members can be used to treat cancers resistant to treatment with known HER2 specific treatments, such as trastuzumab monotherapy, or treatment with trastuzumab in combination with pertuzumab.

As explained above, HER2 is known to be expressed in a number of different cancers. More data is available in breast cancer than in other cancers known to express HER2, as this is perhaps the most well studied HER2 positive cancer. Most data is available for tumours having an IHC score of 3+. Hoff *et al.* (2002) determined that 8% of all breast cancer patients had gene copy number greater than or equal to 10. In the largest adjuvant breast cancer trial of trastuzumab (the HERA trial), gene copy number of the tumours was assessed in 2071 (61%) patients by FISH. Of those patients, a gene copy number of greater than or equal to 10 was identified in about 65% of the patients. This represents about 13% of all breast cancer patients (Dowsett *et al.,* 2009). While the number of copies of the HER2 gene has been studied in some clinical trials, little information is available on the corresponding level of HER2 mRNA in these cancers. However, it is known that an increase in gene copy number in a cell often results in greater expression of that gene, and hence in an increased level of mRNA encoded by that gene. Consequently, patients with cancers with a high HER2 gene copy number per tumour cell (i.e. equal to or greater than 10), are also expected to have high HER2 mRNA levels.

Thus, there are a significant number of patients suffering from cancers with a HER2 GCN of greater than or equal to 10 per tumour cell, which would benefit from treatment with a specific binding member comprising a HER2 antigen binding site engineered into a structural loop region of a constant domain, e.g. CH3 domain, of the specific binding member, or a specific binding member which competes with such a specific binding member for binding to HER2. The same applies with respect to patients suffering from cancers with high HER2 mRNA levels.

The present inventors have shown that gene copy number is predictive for treatment with a specific binding member as disclosed herein, and is not predictive for treatment with the previously approved HER-2-specific treatment trastuzumab. Described herein is a specific binding member comprising a HER2 antigen binding site engineered into a structural loop region of a constant domain, e.g. CH3 domain, of the specific binding member, as well as a specific binding member which competes with such a specific binding member for binding to HER2, for use in a method of treating cancer in a patient, wherein the cancer has a HER2 gene copy number of greater than or equal to 10 per tumour cell.

Also described herein is a specific binding member comprising a HER2 antigen binding site engineered into a structural loop region of a constant domain, e.g. CH3 domain, of the specific binding member, as well as a specific binding member which competes with such a specific binding member for binding to HER2, for use in a method of treating cancer in a patient, wherein said cancer has a high HER2 mRNA level.

In one aspect, the present invention provides a specific binding member which binds to HER2 for use in a method of treating cancer in a patient, wherein said cancer has a HER2 gene copy number, e.g. an average HER2 gene copy number, of greater than or equal to 10 per tumour cell, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

A patient, as referred to herein, is a human patient.

A tumour sample obtained from said patient may have been determined to have a HER2 gene copy number, e.g. an average HER2 gene copy number, of greater than or equal to 10 per tumour cell. Thus, the present invention provides a specific binding member which binds to HER2 for use in a method of treating cancer in a patient, wherein a tumour sample obtained from said patient has been determined to have an average HER2 gene copy number of greater than or equal to 10 per tumour cell, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Alternatively, the method may comprise:

(i) determining the gene copy number, e.g. the average gene copy number, of the HER2 gene per tumour cell in a tumour sample obtained from the patient; and
(ii) treating said patient with said specific binding member if the HER2 gene copy number, e.g. the average HER2 gene copy number, is greater than or equal to 10 per tumour cell.

The present invention therefore also provides a specific binding member which binds to HER2 for use in a method of treating cancer in a patient, wherein the method comprises:

(i) determining the gene copy number, e.g. the average gene copy number, of the HER2 gene per tumour cell in a tumour sample obtained from the patient; and
(ii) treating said patient with said specific binding member if the HER2 gene copy number, e.g. the average HER2 gene copy number, is greater than or equal to 10 per tumour cell;

wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

As set out above, the term "HER2 gene copy number", as used herein may refer to the average HER2 gene copy number per tumour/cancer cell. The HER2 gene copy number may be greater than or equal to 10. Alternatively, the HER2 gene copy number may be greater than or equal to 11, greater than or equal to 12, greater than or equal to 13, greater than or equal to 14, greater than or equal to 15, greater than or equal to 16, greater than or equal to 17, greater than or equal to 18, greater than or equal to 19, greater than or equal to 20, greater than or equal to 21, greater than or equal to 22, greater than or equal to 23, greater than or equal to 24, or greater than or equal to 25. In one example, the HER2 gene copy number is greater than 10. In another example, the HER2 gene copy number is greater than or equal to 11. In another example, the HER2 gene copy number is greater than or equal to 12. In another example, the HER2 gene copy number is greater than or equal to 13. In another example, the HER2 gene copy number is greater than or equal to 14. In a further example, the HER2 gene copy number is greater than or equal to 15. In another example, the HER2 gene copy number is greater than or equal to 16. In another example, the HER2 gene copy number is greater than or equal to 17. In another example, the HER2 gene copy number is greater than or equal to 18. In another example, the HER2 gene copy number is greater than or equal to 19. In another example, the HER2 gene copy number is greater than or equal to 20. In a further example, the HER2 gene copy number is greater than or equal to 23. In a yet further example, the HER2 gene copy number is greater than or equal to 25.Preferably, the HER2 gene copy number, e.g. the average HER2 gene copy number, is greater than or equal to 15.

Preferably, the HER2 gene copy number, e.g. the average HER2 gene copy number, is greater than or equal to 10, wherein the gene copy number is determined by quantitative polymerase chain reaction (qPCR).

Preferably, the HER2 gene copy number, e.g. the average HER2 gene copy number, is greater than or equal to 18, wherein the gene copy number is determined by FISH.

In another aspect, the present disclosure provides a specific binding member which binds to HER2 for use in a method of treating cancer in a patient, wherein said cancer has a high HER2 mRNA level, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

A tumour sample obtained from said patient may have been determined to have a high HER2 mRNA level. Thus, the present disclosure provides a specific binding member which binds to HER2 for use in a method of treating cancer in a patient, wherein a tumour sample obtained from said patient have been determined to have a high HER2 mRNA level, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Alternatively, the method may comprise:

(i) determining the HER2 mRNA level in a tumour sample obtained from the patient; and
(ii) treating said patient with said specific binding member if the HER2 mRNA level is high.

The present disclosure therefore also provides a specific binding member which binds to HER2 for use in a method of treating cancer in a patient, wherein the method comprises:

(i) determining the HER2 mRNA level in a tumour sample obtained from the patient; and
(ii) treating said patient with said specific binding member if the HER2 mRNA level is high;

wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Levels of mRNA encoding a specific protein in a sample may be measured indirectly through quantifying, by quantitative PCR, the copies of cDNA derived from the mRNA in the sample by reverse transcription PCR (RT-PCR), relative to the cDNA copies of a reference, preferably a housekeeping gene. The reference gene has a normal profile across diverse tissue/sample types and is used as a standard. The HER2 mRNA level in a tumour/cancer or tumour sample, as referred to herein, preferably refers to the HER2 mRNA level as reflected by the HER2 cDNA copy number in the tumour/cancer or tumour sample, determined by RT-PCR followed by quantitative PCR, relative to the mRNA level of a reference (e.g. housekeeping) gene, as reflected by the number of cDNA copies of a reference gene, determined by RT-PCR followed by quantitative PCR, in the a tumour/cancer or tumour sample, respectively. The cDNA copy number may be determined from quantitative PCR data using CopyCaller™ Software version 2.0 (Life Technologies), or another equivalent software which would be known to the skilled person. CopyCaller™ Software performs a comparative cycle threshold relative quantitation analysis of the number of copies of the HER2 cDNA relative to the number of copies of the cDNA of a housekeeping gene. Optionally, the cDNA copy number may be normalised by reference to a reference cDNA sample, such as a cDNA sample obtained from a pool of 10 human cell lines which have or are expected to have two copies of the HER2 gene per cell (LifeTechnologies, CopyCaller Software - User Manual).

A high HER2 mRNA level in a tumour/cancer or tumour sample, as referred to herein, thus preferably refers to a high HER2 mRNA level as reflected by a high HER2 cDNA copy number in the tumour/cancer or tumour sample, as determined by RT-PCR followed by quantitative PCR, relative to the mRNA level of a reference gene, as reflected by the number of cDNA copies of the reference gene, as determined by RT-PCR followed by quantitative PCR, in the tumour/cancer or tumour sample, respectively. Specifically, reverse transcription PCR is used to transcribe the HER2 or reference gene mRNA in a tumour sample to cDNA, which is then quantified using qPCR. The reference gene is preferably a housekeeping gene. In a preferred example, the reference gene is TATA-binding protein (TBP) but other suitable reference genes are also known in the art. The HER2 mRNA level in tumour/cancer or tumour sample may be normalized relative to the HER2 mRNA level in a control cell sample known to have two copies of the HER2 gene per cell. In particular, the HER2 mRNA level in tumour/cancer or tumour sample, as reflected by the HER2 cDNA copy number in the tumour/cancer or tumour sample, determined by RT-PCR followed by quantitative PCR, may be normalized relative to the HER2 mRNA level as reflected by the HER2 cDNA copy number, determined by RT-PCR followed by quantitative PCR, in a control cell sample known to have two copies of the HER2 gene per cell. The HER2 mRNA level of the control sample is preferably the HER2 mRNA level as reflected by the cDNA copy number in the control sample, as determined by RT-PCR followed by quantitative PCR, relative to the mRNA level of a reference gene, as reflected by the number of cDNA copies of the reference gene, as determined by RT-PCR followed by quantitative PCR, in the control sample. The reference gene is preferably the same reference gene as used to determine the HER2 mRNA level in the tumour/cancer or tumour sample, e.g. TBP.

A high HER2 mRNA level in a tumour/cancer or tumour sample, as referred to herein, thus preferably refers to a high HER2 mRNA level as reflected by a high HER2 cDNA copy number in the tumour/cancer or tumour sample determined by RT-PCR followed by quantitative PCR, relative to the mRNA level of a reference gene, as reflected by the number of cDNA copies of the reference gene determined by RT-PCR followed by quantitative PCR, in the tumour/cancer or tumour sample, respectively. A high HER2 mRNA level in a tumour/cancer or tumour sample may refer or correspond to a HER2 cDNA copy number greater than or equal to 75, greater than or equal to 80, greater than or equal to 90, greater than or equal to 100, greater than or equal to 110, greater than or equal to 120, greater than or equal to 130, greater than or equal to 140, greater than or equal to 150, greater than or equal to 160, greater than or equal to 168, greater than or equal to 170, greater than or equal to 180, greater than or equal to 190, greater than or equal to 200, greater than or equal to 210, greater than or equal to 220, greater than or equal to 229, or greater than or equal to 248 in a sample of said cancer/tumour relative to the cDNA copy number of a reference gene in said sample, wherein the cDNA copy number of HER2 and the reference gene in the sample is determined by RT-PCR followed by quantitative PCR. A high HER2 mRNA level in a tumour/cancer or tumour sample may refer or correspond to a HER2 cDNA copy number greater than or equal to 168 in a sample of said cancer/tumour relative to the cDNA copy number of a reference gene in said sample, wherein the cDNA copy number of HER2 and the reference gene in the sample is determined by RT-PCR followed by quantitative PCR. A high HER2 mRNA level in a tumour/cancer or tumour sample may refer or correspond to a HER2 cDNA copy number greater than or equal to 248 in a sample of said cancer/tumour relative to the cDNA copy number of a reference gene in said sample, wherein the cDNA copy number of HER2 and the reference gene in the sample is determined by RT-PCR followed by quantitative PCR. Preferably, a high HER2 mRNA level in a tumour/cancer or tumour sample may refer or correspond to a HER2 cDNA copy number greater than or equal to 200 in a sample of said cancer/tumour relative to the cDNA copy number of a reference gene in said sample, wherein the cDNA copy number of HER2 and the reference gene in the sample is determined by RT-PCR followed by quantitative PCR. More preferably, a high HER2 mRNA level in a tumour/cancer or tumour sample may refer or correspond to a HER2 cDNA copy number greater than or equal to 229 in a sample of said cancer/tumour relative to the cDNA copy number of a reference gene in said sample, wherein the cDNA copy number of HER2 and the reference gene in the sample is determined by RT-PCR followed by quantitative PCR.

In addition to the lower limit set out above, a high HER2 mRNA level in a tumour/cancer or tumour sample may refer or correspond to a HER2 cDNA copy number of less than or equal to 820, less than or equal to 850, less than or equal to 900, less than or equal to 950, less than or equal to 1000, less than or equal to 1100, less than or equal to 1200, less than or equal to 1300, less than or equal to 1400, less than or equal to 1500, less than or equal to 1600, less than or equal to 1700, less than or equal to 1800, or less than or equal to 1900 in a sample of said cancer/tumour relative to the cDNA copy number of a reference gene in said sample, wherein the cDNA copy number of HER2 and the reference gene in the sample is determined by RT-PCR followed by quantitative PCR. Preferably, a high HER2 mRNA level in a tumour/cancer or tumour sample may refer or correspond to a HER2 cDNA copy number of less than or equal to 820 in a sample of said cancer/tumour relative to the cDNA copy number of a reference gene in said sample, wherein the cDNA copy number of HER2 and the reference gene in the sample is determined by RT-PCR followed by quantitative PCR.

Preferably, a high HER2 mRNA level in a tumour/cancer or tumour sample may refer or correspond to a HER2 cDNA copy number greater than or equal to 200 and less than or equal to 820 in a sample of said cancer/tumour relative to the cDNA copy number of a reference gene in said sample, wherein the cDNA copy number of HER2 and the reference gene in the sample is determined by RT-PCR followed by quantitative PCR. More preferably, a high HER2 mRNA level in a tumour/cancer or tumour sample may refer or correspond to a HER2 cDNA copy number greater than or equal to 229 and less than or equal to 820 in a sample of said cancer/tumour relative to the cDNA copy number of a reference gene in said sample, wherein the cDNA copy number of HER2 and the reference gene in the sample is determined by RT-PCR followed by quantitative PCR.

A cancer as referred to herein may be a gastric cancer, breast cancer, colorectal cancer, ovarian cancer, pancreatic cancer, lung cancer (for example, non-small cell lung cancer), stomach cancer, or endometrial cancer. All of these cancers have been shown to overexpress HER2. Preferably, the cancer is gastric cancer, breast cancer, or colorectal cancer. More preferably, the cancer is gastric cancer or breast cancer. In one preferred embodiment, the cancer is gastric cancer. Gastric cancer, as referred to herein, includes oesophageal cancer. In another preferred embodiment, the cancer is breast cancer. The HER2 gene copy number of the cancer is as set out above. Such a cancer may be referred to as HER2 positive (HER2+) or as overexpressing HER2. Thus, a cancer, as referred to herein, may be HER2 positive. In addition, or alternatively, a cancer as referred to herein may overexpress HER2. Whether a cancer is HER2 positive or overexpresses HER2 may, for example, be determined initially using immunohistochemistry (IHC), optionally followed by methods such as qPCR as outlined above.

The present inventors have discovered that a specific binding member comprising a HER2 antigen binding site engineered into a structural loop region of a constant domain, e.g. CH3 domain, of the specific binding member can be used to treat cancers which are resistant or refractory for treatment with trastuzumab and/or trastuzumab plus pertuzumab. The cancer may be intrinsically resistant or refractory for treatment with trastuzumab and/or trastuzumab plus pertuzumab, or may have acquired resistance or refractoriness to treatment with trastuzumab and/or trastuzumab plus pertuzumab. The cancer may be a gastric cancer which is resistant or refractory for treatment with trastuzumab and/or trastuzumab plus pertuzumab. Alternatively, the cancer may be a breast cancer which is resistant or refractory for treatment with trastuzumab and/or trastuzumab plus pertuzumab. Methods for determining whether a cancer is resistant or refractory for treatment with trastuzumab and/or trastuzumab plus pertuzumab are well known in the art and would be apparent to the skilled person. For example, a breast cancer which is resistant to treatment with trastuzumab may show progression at the first radiological reassessment at 8-12 weeks or within 3 months after first-line trastuzumab treatment with or without chemotherapy in the metastatic setting or new recurrences diagnosed during or within 12 months after adjuvant trastuzumab. A breast cancer which is refractory to trastuzumab may show disease progression after two or more lines of trastuzumab-containing regimens that initially achieved disease response or stabilization at the first radiological assessment (Wong *et al.,* 2011).

In addition, or alternatively, a patient as referred to herein may have exhibited an inadequate response to trastuzumab and/or trastuzumab plus pertuzumab treatment. An inadequate response to trastuzumab and/or trastuzumab plus pertuzumab may refer to a lack of tumour growth retardation, or insufficient tumour growth retardation, when the patient was treated with trastuzumab and/or trastuzumab plus pertuzumab. Alternatively, a patient who exhibited an inadequate response to trastuzumab and/or trastuzumab plus pertuzumab may refer to a patient who had to discontinue treatment with trastuzumab and/or trastuzumab plus pertuzumab due to adverse effects or adverse events resulting from the treatment with trastuzumab and/or trastuzumab plus pertuzumab, such as side effects, in particular serious side effects. Preferably, an inadequate response to trastuzumab and/or trastuzumab plus pertuzumab refers to a lack of tumour growth retardation, or insufficient tumour growth retardation, when the patient was treated with trastuzumab and/or trastuzumab plus pertuzumab. A medical practitioner will have no difficulty in determining whether a given patient has exhibited an inadequate response to trastuzumab and/or trastuzumab plus pertuzumab treatment, as medical practitioners are experienced in judging whether a given cancer treatment was or was not successful in the case of a particular patient, and hence should or should not be continued. For example, a long period of stable disease may be considered a favourable clinical state if a patient's disease burden and clinical symptoms are light at the start of treatment, whereas if the disease burden is high and clinical symptoms are significant at the start of treatment, then stable disease may be considered an inadequate response.

The term "specific binding member" describes one member of a pair of molecules that bind specifically to one another. A specific binding member may be natural or partly or wholly synthetically produced. A specific binding member normally comprises a molecule having an antigen-binding site. For example, a specific binding member may be an antibody molecule, or fragment thereof, that comprises an antigen-binding site. A specific binding member may also be a non-antibody protein scaffold that binds specifically to an antigen, such as a molecule comprising a modified immunoglobulin like fold, e.g., a fibronectin domain ($^{10}$Fn3 domain). Various methods are available in the art for obtaining antibodies, and antibody fragments, against a target antigen. An antibody may be a monoclonal antibody, especially a human monoclonal antibody, which can be obtained according to the standard methods well known to the person skilled in the art.

Antibody fragments that comprise an antigen-binding site include, but are not limited to, Fab fragments (consisting of VL, VH, CL and CH1 domains); F(ab')2 fragments (bivalent fragment comprising two linked Fab fragments); single chain Fv molecules (scFv) (consisting of a VH domain and a VL domain linked by a peptide linker which allows the two domains to associate to form an antigen binding site; Bird et al. [1988] Science, 242, 423-426; Huston et al. [1988] PNAS USA, 85, 5879-5883); bispecific single chain Fv dimers (PCT/US92/09965); Fv fragments (consisting of the VL and VH domains of a single antibody); dAb fragments (consisting of a VH or a VL domain; Ward et al. [1989] Nature 341, 544-546; McCafferty et al., [1990] Nature, 348, 552-554; Holt et al. [2003] Trends in Biotechnology 21, 484-490); Fd fragments (consisting of the VH and CH1 domains); diabodies (multivalent or multispecific fragments constructed by gene fusion; WO94/13804; Holliger et al. [1993a], Proc. Natl. Acad. Sci. USA 90 6444-6448); and antigen-binding Fc fragments.

Antigen-binding Fc fragments comprise an antigen-binding site engineered into one or more structural loop regions of a constant domain of the Fc fragment, e.g. the CH2 or CH3 domain. The preparation of antigen-binding Fc fragments is described in WO 2006/072620 and WO2009/132876. A specific binding member for use in the present invention preferably is, or comprises, an antigen-binding Fc fragment, also referred to as Fcab™ in the art. More preferably, a specific binding member for use in the present invention is an antigen-binding Fc fragment. The specific binding member may be an IgA1, IgA2, IgD, IgE, IgG1, IgG2, IgG3, IgG4 or IgM antigen-binding Fc fragment. Most preferably, a specific binding member as referred to herein is an IgG1 antigen-binding Fc fragment.

Antigen-binding Fc fragments may be incorporated into immunoglobulin molecules. Thus, a specific binding member for use in the present invention may be an IgA1, IgA2, IgD, IgE, IgG1, IgG2, IgG3, IgG4 or IgM molecule comprising an antigen-binding site in the Fc region. Such molecules preferably comprise a second CDR-based binding site in the variable region of the molecule, and hence are bispecific. In particular, the specific binding member for use in the present invention may be an IgG1 molecule comprising an antigen-binding site in the Fc region.

A specific binding member as referred to herein preferably has a molecular weight (MW) of 60 kD or less, more preferably of 55 kD or less, 54 kD or less, or 53 kD or less. Specific binding member H561-4 disclosed herein has a MW of approximately 53 kD. Specific binding member H561-4 is also known as FS102.

A specific binding member, as referred to herein, may comprise a HER2 antigen binding site engineered into a structural loop region of a CH3 domain of the specific binding member, wherein the HER2 antigen binding site comprises or contains the amino acid sequences FFTYW (SEQ ID NO: 12), and DRRRWTA (SEQ ID NO: 14). Alternatively, a specific binding member may comprise a HER2 antigen binding site engineered into a structural loop region of a CH3 domain of a specific binding member, wherein the HER2 antigen binding site contains the amino acid sequences FFTYW (SEQ ID NO: 12), NGQPE (SEQ ID NO: 13), and DRRRWTA (SEQ ID NO: 14).

A specific binding member, as referred to herein, may comprise a HER2 antigen binding site engineered into a structural loop region of a CH3 domain of the specific binding member, wherein the HER2 antigen binding site comprises the amino acid sequence FFTYW (SEQ ID NO: 12) and the amino acid sequence DRRRWTA (SEQ ID NO: 14). In particular, a specific binding member, as referred to herein, may comprise a HER2 antigen binding site engineered into a structural loop region of a CH3 domain of the specific binding member, wherein the HER2 antigen binding site comprises the amino acid sequence FFTYW (SEQ ID NO: 12) in the AB loop, and amino acid sequence DRRRWTA (SEQ ID NO: 14) in the EF loop of the CH3 domain. Preferably, a specific binding member, as referred to herein, comprises a HER2 antigen binding site engineered into a structural loop region of a CH3 domain of the specific binding member, wherein the HER2 antigen binding site comprises the amino acid sequences FFTYW (SEQ ID NO: 12) and DRRRWTA (SEQ ID NO: 14), wherein SEQ ID NO: 12 is located in residues 14-18 of the AB loop of the CH3 domain, and SEQ ID NO: 14 is located in residues 92-98 of the EF loop of the CH3 domain, and wherein the residues are numbered according to the IMGT (ImMunoGeneTics) numbering scheme. In further preferred embodiment, a specific binding member, as referred to herein, comprises a HER2 antigen binding site engineered into a structural loop region of a CH3 domain of the specific binding member, wherein the HER2 antigen binding site comprises the amino acid sequences FFTYW (SEQ ID NO: 12) and DRRRWTA (SEQ ID NO: 14), wherein SEQ ID NO: 12 is located in residues 381-385, and SEQ ID NO: 14 is located in residues 440-450 of the EF loop of the CH3 domain, and wherein the residues are numbered according to KABAT (Kabat et al., 1987, Sequences of Proteins of Immunological Interest. 4th Edition. US Department of Health and Human Services, and updates thereof, now available on the Internet at immuno.bme.nwu.edu or find "Kabat" using any internet search engine).

A specific binding member, as referred to herein, preferably comprises the CH3 domain of SEQ ID NO: 11. A specific binding member may further comprise the CH2 domain of SEQ ID NO: 10. A specific binding member as referred to herein preferably comprises the sequence of SEQ ID NO: 8. More preferably, a specific binding member as referred to herein is a dimer of a polypeptide of SEQ ID NO: 8. For example, the specific binding member may be a dimer formed by two polypeptides of SEQ ID NO: 8, such as a dimer consisting of two polypeptides, wherein each polypeptide consists of the sequence shown in SEQ ID NO: 8 (such an specific binding member is also referred to as H561-4 herein).

Loss of the CH3 domain C-terminal lysine (K) or C-terminal lysine and adjacent lysine (GK) residues has been previously reported to occur during manufacture of immunoglobulins. This so-called 'C-terminal clipping' is believed to be common phenomenon which does not have functional implications for these microvariants (Beck et al. 2013). It is expected that C-terminal clipping may occur during the manufacture of H561-4. The H561-4 CH3 domain, including the C-terminal lysine and glycine residues which may be subject to C-terminal clipping is shown in SEQ ID NO: 11, and is also present for example in SEQ ID NO: 8 as indicated in the informal sequence listing below.

It therefore follows that a specific binding member, as referred to herein may comprise the CH3 domain of SEQ ID NO: 11, or the CH3 domain of SEQ ID NO: 11 minus one or two C-terminal residues (e.g. the C-terminal lysine or C-terminal lysine and glycine residue). A specific binding member, as referred herein, may comprise the sequence of SEQ ID NO: 8, or the sequence of SEQ ID NO: 8 minus one or two C-terminal residues (e.g. the C-terminal lysine or lysine and glycine residues). Any reference to SEQ ID NOs 8 and 11 as used herein should be interpreted as also applying to these sequences lacking one or two of the C-terminal amino acid residues (e.g. the C-terminal lysine or lysine and glycine residues). Reference to a CH3 domain as referred herein encompasses a CH3 domain which is missing one or two C-terminal amino acids residues (e.g. the C-terminal lysine or lysine and glycine residues).

Variants of such specific binding members may also be employed in the present invention. For example, a specific binding member, as referred to herein, may comprise a HER2 antigen binding site engineered into a structural loop region of a CH3 domain of the specific binding member, wherein the HER2 antigen binding site comprises or contains the amino acid sequences FFTYW (SEQ ID NO: 12), and DRRRWTA (SEQ ID NO: 14) with 3, 2 or 1 amino acid substitutions, deletions or additions. An amino acid substitution may be a conservative amino acid substitution. Alternatively, a specific binding member may comprise a HER2 antigen binding site engineered into a structural loop region of a CH3 domain of a specific binding member, wherein the HER2 antigen binding site contains the amino acid sequences FFTYW (SEQ ID NO: 12), NGQPE (SEQ ID NO: 13), and DRRRWTA (SEQ ID NO: 14) with 3, 2 or 1 amino acid substitutions, deletions or additions. Variants also include those comprising an amino acid sequence that is at least 90%, 95%, 96%, 97%, 98%, or 99% identical to SEQ ID NO: 8. The alterations normally do not result in loss of function, so a specific binding member comprising a thus-altered amino acid sequence may retain the ability to bind HER2. For example, it may bind HER2 with same affinity as a specific binding member in which the substitution is not made.

A specific binding member, which competes with a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14), or a specific binding member as set out above, for binding to HER2, preferably comprises a HER2 antigen binding site engineered into one or more, preferably two, structural loop regions of a constant domain of the specific binding member. More preferably, such a specific binding member comprises a HER2 antigen binding site engineered into one or more, preferably two, structural loop regions of a CH3 domain of the specific binding member. The structural loops in the CH3 domain are located at residues 7-21 (AB loop), 25-39 (BC loop), 41-81 (CD loop), 83-85 (DE loop), 89-103 (EF loop) and 106-117 (FG loop), wherein the residues are numbered according to IMGT (ImMunoGeneTics) numbering scheme (WO 2006/072620 A1). Yet more preferably, such a specific binding member comprises a HER2 antigen binding site engineered into structural loop regions AB and EF of a CH3 domain of the specific binding member. In particular, such a specific binding member may comprise a HER2 antigen binding site engineered into residues 14-18 and 92-98 of the CH3 domain of the specific binding member, wherein the residues are numbered according to the IMGT numbering scheme. Residues 14-18 are located in the AB loop and residues 92-98 are located in the EF loop of the CH3 domain. Such a specific binding member may comprise a HER2 antigen binding site engineered into residues 381-385 and 440-450 of the specific binding member, wherein the residues are numbered according to KABAT. Residues 381-385 are located in the AB loop and residues 440-450 are located in the EF loop of the CH3 domain. Alternatively, such a specific binding member may comprise a HER2 antigen binding site engineered into structural loop regions AB, CD, and EF of a CH3 domain of the specific binding member. The specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14), with which the specific binding member competes for binding to HER2, is preferably a dimer of a polypeptide of SEQ ID NO: 8.

Suitable methods for determining whether two specific binding members compete for binding to the same target, in this case HER2, are well known in the art and would be apparent to the skilled person. Such methods include competition methods using surface plasmon resonance (SPR), enzyme-linked immunosorbent assays (ELISA), fluorescence activated cell sorting (FACS), or immunocytochemistry. Surface plasmon resonance (SPR) methods include BIAcore. Thus, a specific binding member which competes with a second specific binding member for binding to HER2, may compete with the second binding member for binding to HER2, as determined using surface plasmon resonance (SPR) (e.g. BIAcore), an enzyme-linked immunosorbent assay (ELISA), fluorescence activated cell sorting (FACS), or immunocytochemistry. Preferably, a specific binding member which competes with a second specific binding member for binding to HER2, competes with the second binding member for binding to HER2, as determined using surface plasmon resonance (SPR), e.g. BIAcore. As shown in Example 1 herein, trastuzumab and pertuzumab do not compete with specific binding member H561-4, which comprises a HER2 antigen binding site engineered into a structural loop region of its CH3 domain containing the amino acid sequences FFTYW (SEQ ID NO: 12), and DRRRWTA (SEQ ID NO: 14), for binding to HER2.

For the competition methods listed above, the HER2 is immobilised on a chip surface (BIAcore), on a plate (ELISA), or displayed on a cell surface (FACS and immunohistochemistry). One of the two specific binding members to be compared is then incubated with the immobilized HER2 for a time and at a concentration that leads to saturation of the HER2 antigen, thereby blocking all of the HER2 epitopes to which this specific binding member binds. The second specific binding member is then incubated with the immobilized HER2. Binding of the second specific binding member to the HER2 indicates that the specific binding members bind to different epitopes on HER2, whereas if the second binding does not bind to the immobilized HER2, this indicates that the second specific binding member competes with the first specific binding member for binding to HER2, and that the two specific binding members bind to the same (or overlapping) epitopes on HER2.

In certain embodiments, a binding member inhibits the binding of H561-4 to HER2 by at least 50%, 60%, 70%, 80%, 90%, 95% or more, as determined, e.g., by Biacore, ELISA or FACS. In certain embodiments, H561-4 inhibits the binding of the binding member to HER2 by at least 50%, 60%, 70%, 80%, 90%, 95% or more, as determined, e.g., by Biacore, ELISA or FACS. In certain embodiments, a binding member inhibits the binding of H561-4 to HER2 by at least 50%, 60%, 70%, 80%, 90%, 95% or more, and H561-4 inhibits the binding of the binding member to HER2 by at least 50%, 60%, 70%, 80%, 90%, 95% or more, as determined, e.g., by Biacore, ELISA or FACS (i.e., the competition is both ways).

In certain embodiments, a binding member competes for binding to HER2 with H561-4, but not with trastuzumab or pertuzumab. In certain embodiments, a binding member does not inhibit the binding of trastuzumab or pertuzumab to HER2 by more than 50%, 40%, 30%, 20%, 10% or less, as determined, e.g., by Biacore, ELISA or FACS. In certain embodiments, trastuzumab or pertuzumab does not inhibit the binding of H561-4 to HER2 by more than 50%, 40%, 30%, 20%, 10% or less, as determined, e.g., by Biacore, ELISA or FACS. In certain embodiments, a binding member inhibits the binding of H561-4 to HER2 and/or H561-4 inhibits the binding of the binding member by at least 50%, 60%, 70%, 80%, 90%, 95% or more, but does not inhibit the binding of trastuzumab or pertuzumab and/or the to HER2 and/or trastuzumab or pertuzumab does not inhibit the binding of H561-4 to HER2 by more than 50%, 40%, 30%, 20%, 10% or less, as determined, e.g., by Biacore, ELISA or FACS. For example, a binding member inhibits the binding of H561-4 to HER2 and/or H561-4 inhibits the binding of the binding member by at least 50%, but does not inhibit the binding of trastuzumab or pertuzumab to HER2 and/or trastuzumab or pertuzumab does not inhibit the binding of H561-4 to HER2 by more than 50%, as determined, e.g., by Biacore, ELISA or FACS. In another example, a binding member inhibits the binding of H561-4 to HER2 and/or H561-4 inhibits the binding of the binding member by at least 90%, but does not inhibit the binding of trastuzumab or pertuzumab to HER2 and/or trastuzumab or pertuzumab does not inhibit the binding of H561-4 to HER2 by more than 10%, as determined, e.g., by Biacore, ELISA or FACS.

A specific binding member as referred to herein may bind dimeric HER2. The specific binding member may bind dimeric HER2 with an affinity of 1 nM, or an affinity that is higher. The specific binding member may preferentially bind dimeric HER2 compared with monomeric HER2. Preferably, the specific binding member binds to same epitope on the HER2 extracellular domain as a dimer of a polypeptide of SEQ ID NO: 8. The epitope may be a conformational, i.e. non-linear, epitope. The epitope may span domains 1 and 3 of HER2. The epitope may comprise, or consist of, SEQ ID NOs 15, 16 and 17. The epitope may comprise, or consist of, amino acids 13 to 27, 31 to 45, and 420 to 475 of the HER2 extracellular domain. Alternatively, the epitope may be located within SEQ ID NOs 15, 16 and 17, or amino acids 13 to 27, 31 to 45, and 420 to 475 of the HER2 extracellular domain. The sequence of the HER2 extracellular domain is shown in SEQ ID NO: 18.

Methods for determining whether two specific binding members bind to the same epitope are well known in the art and would be apparent to the skilled person. Such methods include Pepscan, hydrogen/deuterium exchange mass spectrometry (HDX-MS), and X-ray Crystallography. In epitope mapping studies, an antigen, in this case HER2, may be divided into small regions, i.e. peptides or domains, and then tested for binding to the specific binding member. The regions which show binding can be used to infer the epitope bound by the specific binding member. Comparison of the regions which bind to two different specific binding members can be used to compare epitopes. Alternatively, mutations can be made in the antigen, here HER2, and then the binding to a specific binding member tested. Residue mutations which cause a loss in binding are indicated to be part of the epitope bound by the specific binding member. However care must be taken to ensure that a mutation does not cause a general loss in structure of the HER2. In X-ray crystallography, the antigen, here HER2, and the specific binding member are crystallised as a complex. The crystal data is then used to determine the epitope bound by the specific binding member. The HER2 epitopes bound by two different specific binding members can then be compared.

Methods for determining HER2 gene copy number are well known in the art and would be apparent to the skilled person. For example, HER2 gene copy number may be determined using fluorescence *in situ* hybridization (FISH), chromogenic *in situ* hybridisation (CISH), or quantitative polymerase chain reaction (qPCR). A previous study reported that the HER2 GCN determined by CISH and FISH is highly correlated as shown by linear regression analysis (Garcia-Caballero et al., 2010). Further studies demonstrated that the HER2 amplification status determined by the two methods also has a strong correlation (Arnould et al., 2012; Mollerup et al., 2012). Therefore the HER2 GCN determined by CISH is expected to be similar to the GCN determined by FISH. Preferably, HER2 gene copy number is determined using FISH. Methods for determining HER2 mRNA levels are similarly known in the art and would be apparent to the skilled person. For example, HER2 mRNA level may be determined using reverse transcription PCR (RT-PCR) followed by quantitative PCR (qPCR). Overexpression of the HER2 gene can be measured by immunohistochemistry (IHC) to determine the amount of HER2 expressed on the cell surface.

FISH employs fluorescence-tagged probes to detect particular DNA sequences in tissue samples by fluorescence microscopy. FISH is used to quantitatively determine HER2 gene amplification in formalin-fixed, paraffin-embedded (FFPE) cancer tissue specimens using specific probes which hybridise with HER2 sequences. Different FDA approved methods exist for performing FISH to determine HER2 GCN (Press et al, 2002). In one method, both HER2 and chromosome 17 numbers are measured and compared, to determine the average HER2 GCN per tumour cell in the samples. CEP17 probes (chromosome enumeration probes, which are directly labelled fluorescent DNA probes specific to the alpha satellite DNA sequence at the centromeric region of chromosome 17) may be used in this test as an internal control for chromosomal aneuploidy. The HER2 gene and CEP17 are labelled with different fluorophores and can be viewed using specific filters for the microscope. Measurement of HER2 gene amplification is based on the ratio between the number of HER2 copies to CEP17 copies counted by microscopy, thereby eliminating aneuploidy of chromosome 17 as a source of increased HER2 GCN. Where, for example, the ratio is equal to or greater than 10, the average HER2 GCN per tumour/cancer cell is greater than or equal to 10. A HER2, or average HER2, gene copy number per tumour/cancer cell, as referred to herein, may therefore exclude any increase in HER2 gene copy number due to chromosomal aneuploidy, in particular aneuploidy of chromosome 17. Alternatively, in a preferred method, overall HER2 gene copy number may be determined by direct counting of the HER2 signals without normalisation to CEP17 signal. This method is preferred because counting overall HER2 gene copy number can avoid false-positive or false-negative results due to loss or gain of the pericentromeric region of chromosome 17 which is more commonly observed than true polysomy (duplication of the entire chromosome) that can result in alterations in the HER2/CEP17 ratio (Wolff et al., 2013; Hanna et al., 2014). As a further alternative, FISH may be performed using only a HER2 probe on FFPE as an indirect method for localization of HER2 gene (Press et al, 2002).

The gene copy number obtained by FISH and qPCR for a particular tumor has been found to be highly correlative (see Example 9). Thus, conversion between GCN values obtained by different methods, (e.g. FISH and qPCR), is possible.

Also provided is the use of a specific binding member comprising a HER2 antigen binding site engineered into a structural loop region of a constant domain, e.g. CH3 domain, of the specific binding member, as well as a specific binding member which competes with such a specific binding member for binding to HER2, in the manufacture of a medicament for treating cancer in a patient, wherein the cancer has a HER2 gene copy number, e.g. an average HER2 gene copy number, of greater than or equal to 10 per tumour cell, and/or wherein the cancer has a high HER2 mRNA level.

The present invention further provides the use of a specific binding member which binds to HER2 in the manufacture of a medicament for treating cancer in a patient, wherein said cancer has a HER2 gene copy number, e.g. an average HER2 gene copy number, of greater than or equal to 10 per tumour cell, and/or the cancer has a high HER2 mRNA level, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Also provided is the use of a specific binding member which binds to HER2 in the manufacture of a medicament for treating cancer in a patient, wherein a tumour sample obtained from said patient has been determined to have an average HER2 gene copy number of greater than or equal to 10 per tumour cell, and/or wherein a tumour sample obtained from said patient has been determined to have a high HER2 mRNA level, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Further provided is the use of a specific binding member which binds to HER2 in the manufacture of a medicament for the treatment of cancer in a patient, the treatment comprising:

(i) determining the gene copy number, e.g. the average gene copy number, of the HER2 gene per tumour cell in a tumour sample obtained from the patient; and
(ii) treating said patient with said specific binding member if the HER2 gene copy number, e.g. the average HER2 gene copy number, is greater than or equal to 10 per tumour cell;

wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or (b) a specific binding member which competes with a specific binding member according to (a) for binding to HER2.

Also provided is the use of a specific binding member which binds to HER2 in the manufacture of a medicament for the treatment of cancer in a patient, the treatment comprising:

(i) determining the HER2 mRNA level in a tumour sample obtained from the patient; and
(ii) treating said patient with said specific binding member if the HER2 mRNA level is high; wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

In another aspect, the present invention provides a method of treating cancer in a patient, wherein said cancer has HER2 gene copy number, e.g. an average HER2 gene copy number, of greater than or equal to 10 per tumour cell, and wherein the method comprises administering to the patient a therapeutically effective amount of:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

A tumour sample obtained from said patient may have been determined to have a HER2 gene copy number, e.g. an average HER2 gene copy number, of greater than or equal to 10 per tumour cell. Thus, the present invention provides a method of treating cancer in a patient, wherein a tumour sample obtained from said patient has been determined to have an average HER2 gene copy number of greater than or equal to 10 per tumour cell, and wherein the method comprises administering to the patient a therapeutically effective amount of:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Alternatively, the method may comprise:

(i) determining the gene copy number, e.g. the average gene copy number, of the HER2 gene per tumour cell in a tumour sample obtained from the patient; and
(ii) treating said patient with said specific binding member if the HER2 gene copy number, e.g. the average HER2 gene copy number, is greater than or equal to 10 per tumour cell.

The present invention therefore also provides a method of treating cancer in a patient, wherein the method comprises:

(i) determining the gene copy number, e.g. the average gene copy number, of the HER2 gene per tumour cell in a tumour sample obtained from the patient; and
(ii) administering a therapeutically effective amount of the specific binding member to the patient if the HER2 gene copy number, e.g. the average gene copy number, is greater than or equal to 10 per tumour cell, wherein the specific binding member is:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

In another aspect, the present invention provides a method of treating cancer in a patient, wherein said cancer has a high HER2 mRNA level, and wherein the method comprises administering to the patient a therapeutically effective amount of:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

In another aspect, the present invention provides a specific binding member which binds to HER2 for use in a method of treating cancer in a patient, wherein said cancer has a high HER2 mRNA level, and wherein the specific binding member is:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

Tumour samples obtained from said patient may have been determined to have high HER2 mRNA levels. Thus, the present invention provides a method of treating cancer in a patient, wherein a tumour sample obtained from said patient has been determined to have a high HER2 mRNA level, and wherein the method comprises administering to the patient a therapeutically effective amount of:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or

(b) a specific binding member which competes with a specific binding member according to (a) for binding to HER2.

Alternatively, the method may comprise:

(i) determining the HER2 mRNA level in a tumour sample obtained from the patient; and
(ii) treating said patient with said specific binding member if the HER2 mRNA levels are high.

The present invention therefore also provides a method of treating cancer in a patient, wherein the method comprises:

(i) determining the HER2 mRNA level in a tumour sample obtained from the patient; and
(ii) administering a therapeutically effective amount of the specific binding member to the patient if the HER2 mRNA level is high, wherein the specific binding member is:

a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2.

The specific binding members described herein are designed to be used in methods of treatment of patients, preferably human patients. Specific binding members will usually be administered in the form of a pharmaceutical composition, which may comprise at least one component in addition to the specific binding member. Thus, pharmaceutical compositions described herein, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be by injection, e.g. intravenous or subcutaneous. The specific binding member may be administered intravenously, or subcutaneously.

Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be employed, as required. Many methods for the preparation of pharmaceutical formulations are known to those skilled in the art. See e.g. Robinson ed., Sustained and Controlled Release Drug Delivery Systems, Marcel Dekker, Inc., New York, 1978.

A composition comprising a conjugate according to the present invention may be administered alone or in combination with other treatments, concurrently or sequentially or as a combined preparation with another therapeutic agent or agents, dependent upon the condition to be treated. For example, a specific binding member for use in the invention may be used in combination with an existing therapeutic agent for the disease to be treated. For example, as specific binding member may be administered in combination with abitaterone acetate (Zytiga), afatinib, aflibercept, anastrozole, bevacizumab, bicalutamide, BRAF inhibitors, carboplatin, capecitabine, cetuximab, cisplatin, crizotinib, cyclophosphoamide, docetaxel, pegylated liposomal doxorubicin, enzalutamide (XTANDI), epirubicin, eribulin mesylate, erlotinib, etoposide, everolimus, exemestane, FOLFIRI, FOLFOX, fluoracil, 5-fluorouracil, flutamide, fulvestrant, gefitinib, gemcitabine, goserelin, hedgehog pathway inhibitors, irenotecan, ixabepilone, lapatinib, letrozole, leucovorin, leuprorelin, methotrexate, mitoxantrone, oxaliplatin, paclitaxel, albumin-bound nano particle paclitaxel, panitumumab, pemetrexed, pertuzumab, prednisone, radium Ra 223 dichloride (Xofigo Injection), ramucirumab, regorafenib, S-1, tamoxifen, topotecan, trastuzumab DM-1, triptorelin, or vinorelbine.

Administration may be in a "therapeutically effective amount", this being sufficient to show benefit to a patient. Such benefit may be at least amelioration of at least one symptom. Thus "treatment" of a specified disease refers to amelioration of at least one symptom. The actual amount administered, and rate and time-course of administration, will depend on the nature and severity of what is being treated, the particular patient being treated, the clinical condition of the individual patient, the cause of the disorder, the site of delivery of the composition, the type of conjugate, the method of administration, the scheduling of administration and other factors known to medical practitioners. Prescription of treatment, e.g. decisions on dosage etc., is within the responsibility of general practitioners and other medical doctors, and may depend on the severity of the symptoms and/or progression of a disease being treated. Appropriate doses of antibody are well known in the art (Ledermann et al. (1991) Int. J. Cancer 47: 659-664; and Bagshawe et al. (1991) Antibody, Immunoconjugates and Radiopharmaceuticals 4: 915-922). Specific dosages indicated herein, or in the Physician's Desk Reference (2003) as appropriate for the type of medicament being administered, may be used. A therapeutically effective amount or suitable dose of a specific binding member can be determined by comparing its *in vitro* activity and *in vivo* activity in an animal model. Methods for extrapolation of effective dosages in mice and other test animals to humans are known. The precise dose will depend upon a number of factors, including whether the size and location of the area to be treated, and the precise nature of the specific binding member. Treatments may be repeated at daily, twice-weekly, weekly or monthly intervals, at the discretion of the physician. Treatment may be given before, and/or after surgery, and may be administered or applied directly at the anatomical site of surgical treatment.

In another aspect, the present invention provides a method of identifying a cancer in a patient which is susceptible to treatment with:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2; the method comprising:

(i) determining the gene copy number, e.g. the average gene copy number, of the HER2 gene per tumour cell in a tumour sample obtained from the patient, wherein a gene copy number, e.g. an average gene copy number, of greater than or equal to 10 per tumour cell indicates that the cancer is susceptible to treatment with the specific binding member.

A method of identifying a cancer in a patient which is susceptible to treatment may further comprise (ii) selecting said patient for treatment with the specific binding member if the HER2 gene copy number, e.g. the average HER2 gene copy number, is greater than or equal to 10 per tumour cell.

In another aspect, the present invention provides a method of predicting the response of a cancer to treatment with:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2; the method comprising:

(i) determining the HER2 gene copy number, e.g. the average HER2 gene copy number, of the HER2 gene per tumour cell in a tumour sample obtained from a patient, wherein a gene copy number, e.g. the average HER2 gene copy number, of greater than or equal to 10 per tumour cell indicates that the cancer is susceptible to treatment with the specific binding member, and wherein a gene copy number, e.g. the average HER2 gene copy number, of less than 10 per tumour cell indicates that the cancer is not susceptible to treatment with the specific binding member.

A method of predicting the response of a cancer to treatment may further comprise:

(ii) selecting said cancer for treatment with the specific binding member if the HER2 gene copy number, e.g. the average HER2 gene copy number, is greater than or equal to 10 per tumour cell.

In another aspect, the present invention provides a method of identifying a cancer in a patient which is susceptible to treatment with:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2; the method comprising:

(i) determining the HER2 mRNA level in a tumour sample obtained from the patient, wherein a high HER2 mRNA level indicates that the cancer is susceptible to treatment with the specific binding member.

A method of identifying a cancer in a patient which is susceptible to treatment may further comprise (ii) selecting said patient for treatment with the specific binding member if the HER2 mRNA level of the tumour sample is high.

In another aspect, the present invention provides a method of predicting the response of a cancer to treatment with:

(a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
(b) a specific binding member which competes with a specific binding member according to (a) for binding to HER2; the method comprising:

(i) determining the HER2 mRNA level in a tumour sample obtained from a patient, wherein a high HER2 mRNA level in the tumour sample indicates that the cancer is susceptible to treatment with the specific binding member.

A method of predicting the response of a cancer to treatment may further comprise:

(ii) selecting said cancer for treatment with the specific binding member if the HER2 mRNA level in the tumour sample is high.

In another aspect, the present invention provides a specific binding member which binds to HER2, wherein the specific binding member is a dimer of a polypeptide of SEQ ID NO: 8. For example, the specific binding member may be a dimer formed by two polypeptides of SEQ ID NO: 8, such as a dimer consisting of two polypeptides, wherein each polypeptide consists of the sequence shown in SEQ ID NO: 8 (such an specific binding member is also referred to as H561-4 herein).

Also provided is a pharmaceutical composition comprising the specific binding member of the invention. The pharmaceutical composition may comprise the specific binding member of the invention and a pharmaceutically acceptable excipient. A nucleic acid encoding the specific binding member of the invention is also provided. Preferably, the nucleic acid comprises or consists of the sequence of SEQ ID NO: 1. This nucleotide sequence has been optimized for expression in Chinese Hamster Ovary (CHO) cells but the skilled person would have no difficulty in designing other nucelotide sequences encoding the specific binding member of the invention. Also provided is a vector comprising a nucleic acid encoding the specific binding member of the invention, as is a host cell comprising a vector or nucleic acid of the invention.

The present invention further provides a method of producing the specific binding memberof the invention, comprising culturing the recombinant host cell of the invention under conditions for production of the specific binding member. The method may further comprise isolating and/or purifying the specific binding member. The method may also comprise formulating the specific binding member into a pharmaceutical composition.

## Brief Description of the Figures

**Figure 1** shows an alignment of the antigen-binding Fc fragment H561-4 amino acid sequence (H561-4) with the wild-type IgG1 amino acid sequence (WT) in the same region. The amino acids are numbered according to Kabat - Kabat numbering is shown above the aligned sequences (Note: Kabat numbering does not have residues 293-294, 297-298, 315-316, 356,362, 380, 403-404, 409, 412-413, 429, 431-431 in IgG1). Part of the AB loop is shown in bold, and part of the EF loop is shown in bold and doubly underlined.

**Figure 2** demonstrates that H561-4 binds to a different epitope on HER2 than trastuzumab or pertuzumab. **Figure 2A** shows a surface plasmon resonance (SPR) analysis of epitope competition of trastuzumab and H561-4 using BIAcore. A CM5 BIAcore chip coated with 1000 RU of HER2 ECD was saturated with trastuzumab (TR) by injection of 10 μg/ml of trastuzumab for 3 min followed by a second injection of trastuzumab at 10 μg/ml showing that the HER2 chip is saturated with trastuzumab after the first injection (dotted black line). A CM5 BIAcore chip coated with 1000 RU of HER2 ECD was saturated with trastuzumab by injection of 10 μg/ml of trastuzumab for 3 min followed by a second injection of a mixture of H561-4 (10 μg/ml) and trastuzumab (10 μg/ml) showing when a HER2 chip is saturated with trastuzumab H561-4 is still able to bind (solid black line). A CM5 BIAcore chip coated with 1000 RU of HER2 ECD was saturated with H561-4 by injection of 10 μg/ml of H561-4 for 3 min followed by a second injection of H561-4 (10 μg/ml) showing that the HER2 chip is saturated with H561-4 after the first injection (dashed black line). A CM5 BIAcore chip coated with 1000 RU of HER2 ECD was saturated with H561-4 by injection of 10 μg/ml of H561-4 for 3 min followed by a second injection of a mixture of trastuzumab (10 μg/ml) and H561-4 (10 μg/ml) showing when a HER2 chip is saturated with H561-4 trastuzumab is still able to bind (wide dashed black line). **Figure 2B** shows a SPR analysis of epitope competition of pertuzumab (PE) and H561-4 using Octet. A streptavidin coated tip was loaded by incubation in biot-HER2 for 30 min and then washed for 1 minute in buffer. HER2 was saturated with pertuzumab by incubation of 325 nM pertuzumab for 30 min followed by a second incubation of pertuzumab at 325 nM showing that the HER2 coated tip is saturated with pertuzumab after the first incubation (grey line). A streptavidin coated tip was loaded by incubation in biot-HER2 for 30 min, and then washed for 1 minute in buffer. HER2 was saturated with pertuzumab by incubation of 325 nM pertuzumab for 30 min followed by a second incubation of H561-4 at 325 nM and pertuzumab at 325 nM showing that a HER2 coated tip saturated with pertuzumab is still able to bind H561-4 (black line). **Figure 2C** shows the predicted binding region of H561-4 (black labelling spanning domain 1 and 3) on the extracellular domain of HER2. The binding epitope of H561-4 was predicted using Pepscan CLIPS (Chemical Linkage of Peptides onto Scaffolds) technology. HER2 domains are indicated by roman numerals. The table in Figure 2C indicates the predicted linear sequences for each of the binding epitopes of H561-4, that together from the binding pocket spanning domains 1 and 3.

**Figure 3** demonstrates that H561-4 induces apoptosis and leads to HER2 internalization and degradation. **Figure 3A** shows that H561-4 treatment of SKBr3 cells caused a reduction in the total number of cells and in the percentage of viable cells. PI and Annexin V staining showed an increase in late apoptotic and necrotic cells after H561-4 treatment. **Figure 3B** shows that H561-4 treatment of SKBr3 cells caused a reduction in total HER2 and in phosphorylated HER2. **Figure 3C** shows that H561-4 treatment of SKBr3 cells caused a reduction in cell surface HER2 and total HER2. No such reduction in Her2 was seen in SKBr3 cells treated with trastuzumab, wild-type (WT) antigen-binding Fc fragment (SEQ ID NO: 19), or an IgG1 control (IgG), or untreated cells. **Figure 3D** shows that caspase 3/7 activity is induced by H561-4 treatment of SKBr3 cells but not by treatment of SKBr3 cells with trastuzumab, wt antigen-binding Fc fragment, or and IgG1 control (ctrl).

**Figure 4** demonstrates efficacy of H561-4 treatment human patient derived tumor xenograft (PDX) models with HER2 gene copy numbers greater than or equal to 10. **Figure 4A** shows *In vivo* tumour response data from gastric tumour (GXF281) with a high HER2 gene copy number (HER2 GCN = 15 copies per cell). The arrows indicate the treatment days. **Figure 4B** shows *in vivo* tumour response data from gastric tumour (GXA3039) with high HER2 gene copy number (HER2 GCN= 25 copies per cell). The arrows indicate the treatment days. **Figure 4C** shows *in vivo* tumour response data from colorectal tumour (CXF1991) with high HER2 gene copy number (HER2 GCN= 32 copies per cell). The arrows indicate the treatment days. **Figure 4D** shows *in vivo* tumour response data from colorectal tumour (CXF2102) with high HER2 gene copy number (HER2 GCN= 35 copies per cell). The arrows indicate the treatment days. **Figure 4E** shows *in vivo* tumour response data from gastric tumour (GXA3054) with high HER2 gene copy number (HER2 GCN= 76 copies per cell). The arrows indicate the treatment days. **Figure 4F** shows *in vivo* tumour response data from gastric tumour (GXA3038) with high HER2 gene copy number (HER2 GCN= 29 copies per cell). The arrows indicate the treatment days. **Figure 4G** shows *in vivo* tumour response data from breast tumour (HBCx-13B) with high HER2 gene copy number (HER2 GCN= 23 copies per cell). The arrows indicate the treatment days. **Figure 4H** shows *in vivo* tumour response data from Gastric tumour (GA0060) with high HER2 gene copy number (HER2 GCN= 43 copies per cell). The arrows indicate the treatment days. The error bars in Figures 4A-H represent the standard error of the mean (SEM).

**Figure 5** shows that H561-4 overcomes trastuzumab plus pertuzumab (TR+PE) resistance in GXF281 gastric PDX model (HER2 GCN per cell = 25). Mice were treated intravenously (i.v.) with compounds listed in the legend ("/" separates the dosing compounds of the first treatment cycle from those of the second cycle). Arrows indicate the dosing schedule. After implanted tumours reached the mean volume of 100 $mm^3$, they were treated with 4 weekly doses of TR+PE combination therapy (or 4 weekly doses of H561-4 as a control). The TR+PE treated tumours continued to grow albeit at a slower rate. A 33-day interval was implemented to allow the clearance of these antibodies before the second dosing cycle. After the interval, if the mean tumour volume reached above 500 $mm^3$, mice were re-dosed weekly with TR+PE or with H561-4. The error bars represent the standard error of the mean (SEM).

**Figure 6** shows a scatter plot of the T/C values of H561-4 and trastuzumab treatment in PDX models with HER2 gene copy numbers (GCN) greater than or equal to 10, compared with PDX models with GCN of less than 10. P values were calculated by unpaired T-test using Graphpad prism software.

**Figure 7** shows a scatter plot of the T/C values of H561-4 and trastuzumab treatment in PDX models with HER2 mRNA levels (mRNA) greater than or equal to 200, compared with PDX models with mRNA levels of less than 200. P values were calculated by unpaired T-test using Graphpad prism software.

**Figure 8** shows a scatter plot of the T/C values of H561-4 and trastuzumab treatment in PDX models with HER2 GCN greater than 10 compared to PDX models with GCN less than 10. The T/C values were calculated using the updated T/C equation. P values were calculated by unpaired T-test using Graphpad prism software.

**Figure 9** shows a scatter plot of the T/C values of H561-4 and trastuzumab treatment in PDX models with HER2 mRNA level greater than or equal to 200 compared to PDX models with HER2 mRNA level of less than 200. The T/C values were calculated using the updated T/C equation. P values were calculated by unpaired T-test using Graphpad prism software.

**Figure 10** shows a scatter plot of the T/C values of H561-4 and trastuzumab treatment in PDX models with HER2 GCN greater than 18 as determined by FISH compared to PDX models with GCN less than 18. P values were calculated by unpaired T-test using Graphpad prism software.

**Figure 11** shows a scatter plot of the gene copy number determined by FISH and qPCR in each of 17 PDX tumour models. The GCN determined by the two methods are highly correlative based on the correlation analysis (Pearson $r$= 0.90; $p$ < 0.0001; 95% CI = 0.74-0.96).

**Figure 12** shows a scatter plot of the T/C values of H561-4 and trastuzumab treatment in PDX models with HER2 protein overexpression positive compared to PDX models with HER2 protein overexpression negative. P values were calculated by unpaired T-test using Graphpad prism software.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above.

## Examples

### Example 1 - H561-4 binds to a different epitope on HER2 than trastuzumab or pertuzumab

Surface Plasmon resonance (SPR) was used to determine if H561-4 competes with the known anti-HER2 antibodies, trastuzumab and pertuzumab, for binding to HER2.

*Competition with trastuzumab*

A BIAcore 3000 (GE healthcare) was used to determine if H561-4 could bind to a human HER2 coated chip that was saturated with trastuzumab (TR) and vice versa. A CM5 chip was coated with 1000 RU of extracellular domain (ECD) of human HER2 (BenderMed Systems) using standard amine coupling, experiments were carried out using a flow rate of 20 μl/min in HBS-P buffer (GE Healthcare), and the HER2 surface was regenerated using 4 M $MgCl_2$. The first HER2 binding compound (H561-4 or trastuzumab) was injected at 10 μg/ml for 3 min and then the second HER2 binding compound (trastuzumab or H561-4) was injected for 3 min (the second injection was performed in the presence of 10 μg/ml of the first compound in order to eliminate the dissociation of compound 1 during the second injection) followed by dissociation in buffer (Figure 2A). In the case where injections 1 and 2 were the same compound (trastuzumab followed by trastuzumab or H561-4 followed by H561-4) little or no binding was observed at the second injection, showing that the HER2 binding surface was saturated in both cases. Table 1 below shows the response (RU) observed with the different combinations of injections:

Table 1 shows that the binding response of H561-4 on a HER2 chip saturated with trastuzumab (246 RU) is similar to that of H561-4 binding on a naked HER2 surface (253-254 RU), indicating that H561-4 does not compete with trastuzumab for binding to HER2.

| Injection 1 | Response (RU) | Injection 2 | Additional Response (RU) |
|---|---|---|---|
| Trastuzumab | 794 | Trastuzumab | 20 |
| Trastuzumab | 771 | H561-4 (+Trastuzumab) | 246 |
| H561-4 | 254 | H561-4 | 0 |
| H561-4 | 253 | Trastuzumab (H561-4) | 746 |

The same result was seen when the injection series was reversed in that the binding response of trastuzumab on a H561-4 saturated surface (746 RU) is similar to that when binding to a naked HER2 surface (771-794 RU).

*Competition with Pertuzumab*

An Octet (ForteBio) was used to determine if H561-4 could bind to a human HER2 coated tip that was saturated with pertuzumab (PE) and vice versa. All steps were carried out with at 1000 rpm in kinetics buffer (ForteBio). A Streptavidin coated tip (ForteBio) was coated by incubation in 50 μg/ml biot-HER2 for 30 mins, the tip was then washed in buffer for 1 min then incubated in 325 nM pertuzumab for 30 mins to saturate all the pertuzumab binding sites then immediately transferred into a mixture of H561-4 (325 nM) and pertuzumab (325 nM) and incubated for 30 mins before dissociation in buffer for 10 mins. As a control to ensure that all pertuzumab binding sites were occupied, a fresh tip was then used to repeat the experiment using Pertuzumab (325 nM) in the first step and pertuzumab (325 nM) only in the second step (Figure 2B). In the case where both steps were pertuzumab, little or no additional binding was observed at the second step showing that the HER2 binding surface was saturated with pertuzumab. Table 2 below shows the response (RU) observed with the different combinations of injections:

Table 2 shows that the binding response of H561-4 on a HER2 chip saturated with pertuzumab (0.41 RU) is significantly greater than the additional binding response of pertuzumab (0.04 RU), indicating that H561-4 does not compete with pertuzumab for binding to HER2.

| Incubation 1 | Response (RU) | Incubation 2 | Additional Response (RU) |
|---|---|---|---|
| Pertuzumab | 1.15 | Pertuzumab | 0.04 |
| Pertuzumab | 1.01 | H561-4 (+Pertuzumab) | 0.41 |

Example 2 - Investigating binding of H561-4 to HER2

*Comparison of H561-4 binding to monomeric versus dimeric HER2*

H561-4 was found to preferentially bind to dimeric HER2 compared with monomeric HER2. ELISA based and SPR based methods were used to compare binding of H561-4 to monomeric HER2 (extracellular domain [ECD] of HER2 without tag) versus dimeric HER2 (ECD of HER2 with Fc tag, R&D biological). In the ELISA format, the HER2 was coated onto a plate surface and was therefore immobilised HER2, whereas in the BIAcore experiments, the H561-4 was captured and the HER2 was injected as the analyte and was therefore soluble HER2.

*ELISA based comparison of H561-4 binding to monomeric HER2 and dimeric HER2*

Monomeric HER2 (HER2 ECD untagged) and dimeric HER2 (HER2 ECD Fc-tag) were immobilised at 2 μg/ml on a 96-well maxisorp plate (Nunc) in PBS overnight at 4°C. The plates were blocked for 1 hour with 200 μl of 5% BSA in PBS. H561-4 and trastuzumab (Roche) were biotinylated using the Innova Bioscience biotinylation kit (#704-0010) following the manufactures instructions. A dilution series of biotinylated H561-4 or biotinylated trastuzumab was prepared in 5% BSA. The blocking solution was removed and 100 μl of the dilution series of H561-4 and trastuzumab were added to the plate and incubated at RT for 1 hour. The dilution series was removed and the plate was washed 3x with phosphate buffered saline with Tween 20 (PBST).

A 1:5000 dilution of Strep-HRP (Abcam ab) was prepared in 5% BSA and 100 μl was added to the plate and incubated for 1 hour at RT. The plate was then washed 3x with PBST before 100 μl of TMB (Roche) were added, and the plate incubated at RT for 2 mins. The reaction was stopped using 100 μl of 2 M $H_2SO_4$ and the plate was read at 450 nM.

The trastuzumab control had the same half maximal effective concentration ($EC_{50}$) (1.3 - 1.5 nM) on monomeric and dimeric HER2, whereas H561-4 had a 10-fold preference for the dimeric HER2 compared with monomeric HER2, as shown by the $EC_{50}$ values of 1.7 nM and 20.6 nM, respectively. $EC_{50}$ refers to the concentration of the binding member which induces a response halfway between the baseline and maximum after a specified exposure time.

*BIAcore based comparison of H561-4 binding to monomeric HER2 and dimeric HER2*

In order to capture the H561-4 in an orientation that allowed for binding to soluble HER2, an anti-EGFR-H561-4 $mAb^2$ (EGFR/H561-4) was used. This molecule is an anti-EGFR antibody with the Fc region modified to contain the binding loops of H561-4 in the CH3 domain. This EGFR/HER2 bispecifc molecule was then captured on a EGFR coated BIAcore chip via the EGFR binding specificity, which is in the Fab arms, thereby leaving the HER2 binding CH3 domain exposed to the analyte. In order to allow comparison with the anti-HER2 mAb trastuzumab, a similar bispecific was constructed, trastuzumab-EGFR antigen-binding Fc fragment (TR/EGFR). This bispecific comprises the anti-HER2 mAb trastuzumab with the Fc region modified to contain the binding loops of an anti-EGFR antigen-binding Fc fragment in the CH3 domain. Both the EGFR mAb and the EGFR antigen-binding Fc fragment have similar binding affinities for EGFR (1 nM). The TR/EGFR was captured on the EGFR chip via the EGFR antigen-binding Fc fragment Fc region thereby leaving the HER2 binding specificity in the Fab arms exposed to the analyte.

A BIAcore streptavidin chip was immobilised with 1000 RU of biot-EGFR. Experiments were carried out using a flow rate of 20 μl/min in HBS-P buffer (GE Healthcare). The EGFR/HER2 bispecific molecules were captured by injecting 60 μl of 100 mM of the $mAb^2$, and the EGFR surface was regenerated using 50 mM NaOH. Dilution series of monomeric (untagged HER2 ECD, in house) and dimeric (Fc-tagged HER2 ECD, R&D) were injected as the analyte. The data were fit using the Langmuir 1:1 binding model.

Table 3 below shows the $K_D$ values obtained by the 1:1 fits of the data. H561-4 shows only weak binding to soluble monomeric HER2 even at concentrations up to 1000 nM, whereas the affinity to dimeric HER2 is 1 nM. Trastuzumab binds to monomeric HER2 with an affinity of 1 nM and to dimeric HER2 with a sub-nM affinity.

| $K_D$ | $K_D$ monomeric HER2 | $K_D$ dimeric HER2 |
|---|---|---|
| **H561-4** | >1 μM | 1 nM |
| **Trastuzumab** | 1 nM | Sub nM (off rate outside of range that can be fitted using BIAcore 2000) |

*Prediction of H561-4 epitope on HER2 using Pepscan*

Using CLIPS technology (Pepscan), H561-4 was determined to bind across two domains of extracellular HER2. These domains were domains 1 and 3 of HER2, with binding epitopes mapped to amino acids 13 through to 27 (LPASPETHLDMLRHL) and amino acids 31 through to 45 (CQWQGNLELTYLPT) of domain 1. Domain 3 had a larger binding site of amino acids 420 through to 475 (SLTLQGLGISWLGLRSLRELGSGLALIHHNTHLCFVHTVPWDQLFRNPHQALLHTA). In a linear formation these three different epitopes seem random, however when viewed in the conformational format, a distinct binding pocket spanning these two domains is revealed (Figure 2C).

The linear and CLIPS (Chemical Linkage of Peptides onto Scaffolds) peptides were synthesized based on the amino acid sequence of extracellular HER2 using standard FMOC-chemistry and de-protected using trifluoric acid with scavengers. The constrained peptides were synthesized on chemical scaffolds in order to reconstruct conformational epitopes, using CLIPS technology (Timmerman et al., 2007, Functional reconstruction and synthetic mimicry of a conformational epitope using CLIPS™ technology. J MoL Recognit. 20:283-99). The side-chains of the multiple cysteines in the peptides were coupled to CLIPS templates by reacting onto credit-card format polypropylene PEPSCAN cards (455 peptide formats/card) with a 0.5 mM solution of CLIPS template (1,3- bis (bromomethyl) benzene in ammonium bicarbonate (20 mM, pH 7.9)/acetonitrile (1:1[v/v]). The cards were gently shaken in the solution for 30 to 60 mins while completely covered in solution. Finally, the cards were washed extensively with excess of $H_20$ and sonicated in disrupt-buffer containing 1 percent SDS/0.1 percent beta-mercaptoethanol in PBS (pH 7.2) at 70°C for 30 mins, followed by sonication in $H_20$ for another 45 mins. The binding of antibody to each peptide was tested in a PEPSCAN-based ELISA. The 455-well credit card format polypropylene cards containing the covalently linked peptides were incubated with primary antibody solution for example consisting of 1 micrograms/mL diluted in blocking solution, 5% horse serum, 5% ovalbumin (w/v) in PBS/1% Tween 80.

After washing, the peptides were incubated with a 1:1000 dilution of antibody peroxidase conjugate for one hour at 25° C. After washing, the peroxidase substrate 2,2'-azino-di-3-ethylbenzthiazoline sulfonate (ABTS) and 2 microlitres of 3 percent 11202 were added. After one hour, the colour development was measured. The colour development was quantified with a charge coupled device (CCD) - camera and an image processing system (as firstly described in Slootstra et al., 1996, Structural aspects of antibody-antigen interaction revealed through small random peptide libraries, Molecular Diversity, 1, 87-96).

The raw data were therefore optical values obtained by a CCD-camera. The values mostly range from 0 to 3000, a log scale similar to 1 to 3 of a standard 96-well plate ELISA-reader. In this experiment, the values obtained ranged from 0-380.

The CCD-camera first took a picture of the card before peroxidase colouring and then another picture after peroxidase colouring. These two pictures were subtracted from each other, which resulted in the raw-data. This was copied into the Peplabtm database. Then the values were copied to excel and this file was labelled as a raw-data file. One follow-up manipulation was allowed. Sometimes a well contains an air-bubble resulting in a false-positive value, the cards were manually inspected and any values caused by an air-bubble were scored as 0. Positive values were taken as a binding signal (the epitope map) and mapped to the sequence of HER2. Computer generated figures of the extracellular HER2 protein were generated and the positive binding signals mapped, indicating the areas of antigen-binding Fc fragment binding (Figure 2C).

Example 3 - H561-4 induces apoptosis and leads to unique and profound HER2 internalization and degradation

The HER2 overexpressing breast cancer cell line SKBr3 was used as a model system to determine the mechanism of action of H561-4. The SKBr3 cell line was obtained from ATCC (HTB-30) and was cultured in McCoy's 5a + GlutaMAX Medium (Invitrogen) containing 10% fetal bovine serum (FBS). Trastuzumab (Roche) was used as a control in all mechanism of action studies.

*H561-4 has anti-proliferative activity in SKBr3 cells and induces apoptosis*

SKBr3 cells were seeded at 7.5 x $10^3$ cells per well in 96 well tissue culture plates and incubated overnight at 37°C, 5% $CO_2$ with 100 μl per well. The next day 20 μl media containing the treatments (H561-4 or trastuzumab) were added to give a concentration dilution series of H561-4 or trastuzumab. The cells were incubated with the treatments for 5 days at 37°C, 5% $CO_2$ The cells were then stained with a mixture of 1 μg/ml final Hoechst stain (Sigma Aldrich, H6024), 2.5 μg/ml Propidium Iodide (PI) and 1:100 Alexa-488 Annexin V (Invitrogen, V13245) in Annexin V Binding Buffer (12.5 mM $CaCl_2$, 140 mM NaCl, 10 mM Hepes, pH 7.4) and incubated at room temperature (RT) for 2 hours. The cells were then read on the ImageXpress Micro (Molecular Devices). The cell numbers in each well were counted using the Hoechst staining at 4x magnification. Annexin V and PI staining was used to determine the percentage of viable cells (cells not stained with PI), early apoptotic cells (cells stained with Annexin V only), late apoptotic cells (cells stained with Annexin V and PI) and necrotic cells (cells stained with PI only) with a 40x magnification. At the highest concentration tested (300 nM) H561-4 caused a 37% reduction in cell number compared to a 22% reduction by trastuzumab. In addition, of the remaining H561-4 treated cells only 43% were viable compared with 79% for trastuzumab. This reduction in total cells and viable cells was associated with an increase in late apoptotic and necrotic cells (18% and 35%, respectively) for the H561-4 treated cells only. The results of this experiment are shown in Figure 3A.

*H561-4 causes internalisation and degradation of HER2*

SKBr3 cells were seeded at 3.3 x $10^5$ cells/ml with 3 ml/well in a 6 well plate and incubated overnight at 37°C, 5% $CO_2$. The next day the media were changed to media containing 0.1 % FBS and incubated for at least 2 hours at 37°C, 5% $CO_2$ before the media were removed and replaced with media containing 0.1 % FBS and the treatments (H561-4 or trastuzumab) at 200 nM. The cells were incubated at 37°C, 5% $CO_2$ for 24 h, 48 h or 72 h. The lysates were then collected on ice: the media were removed and 250 μl of the lysis buffer (10mM Tris pH7.5, 150mM NaCl, 1mM EDTA, 1:100 protease cocktail [Calbiochem, 539131], 1:100 phosphatase cocktails [Calbiochem, 524625]), was added. The cells were then scraped from the well surface and 250 μl were incubated on ice for 15 mins, centrifuged at 14,000 rpm for 15 min at 4°C and the supernatant was collected for analysis. The samples were mixed with NuPAGE loading buffer containing β-mercaptoethanol, incubated at 95°C for 10 mins then stored at -20°C until use. 20 μl of the samples were run on a 4-12 % Bis-Tris SDS PAGE and transferred to a nitrocellulose membrane, the membrane was blocked with 5 % Marvel/TBST and then probed for HER2 (Cell signalling, #2248), phosphor-HER2 (Cell Signalling, #2243) or α-β-actin as a loading control (Sigma A1978). The membranes were then washed and the appropriate secondary antibody conjugated to HRP (anti-mouse, Jackson ImmunoResearch, or anti-rabbit Jackson ImmunoResearch) was added. The membrane was developed using ECL reagents (GE Healthcare, RPN2105) and imaged. H561-4 treatment caused a reduction in total HER2 at 24 h, with a complete reduction at 48 h and 72 h (Figure 3B). In contrast, trastuzumab treatment did not detectably reduce HER2 levels at the time points studied (Figure 3B). H561-4 treatment also caused a concomitant reduction in pHER2. This reduction was evident at 24 h, with a further reduction at 48 h and no detectable pHER2 remaining at 72 h. Trastuzumab treatment only caused a slight reduction in pHER2 at the 72 h time point (Figure 3B).

Immunofluorescence imaging was then used to quantify the degradation of HER2. SKBr3 cells were seeded in a 96-well plate at $1x10^4$ cells/well and incubated overnight at 37°C, 5% $CO_2$. The next day, treatments (H561-4; trastuzumab [TR]) or the controls, (wild-type [WT] antigen-binding Fc fragment; or IgG1 isotype control, Sigma 15154 [IgG/IgG1 ctrl]) were added to the media to give a final concentration of 200 nM.

The cells were incubated for 24 h, 48 h or 72 h at 37°C, 5% $CO_2$, the supernatant removed, and the cells washed with PBS before being fixed with 4% formaldehyde in PBS for 20 mins. For cell surface imaging, half of the cells were then permeabilised in 0.1% TritonX100/Glucose 0.1 mg/ml in PBS for total protein imaging. The cells were then washed and blocked in 5% FBS/PBS for 1 hour at RT, before being incubated for 1 hour at RT with the primary antibody diluted 1:100 in PBS (Mouse Anti-Her2 MGR2, Enzo Life Sciences). The cells were then washed twice and before being incubated for 1 h at RT in the dark with the 5 μg/ml of secondary antibody (anti-mouse alexa647, Invitrogen A21237) and 1 μg/ml of Hoechst (Invitrogen, 33342). The cells were then washed and stored in 0.05 % NaN3 in PBS until they were read on the ImageXpress Micro (Molecular Devices). The analysis was performed with 40x imaging, the multiwave scoring function was used to count the cell nuclei and the number of cells with HER2 positive staining, and then the per cent of HER2 positive cells was calculated.

H561-4 treatment of SKBr3 cells at 200 nM resulted in a decrease in both cell surface and total HER2. The reduction in HER2 levels was detectable after 24 hours treatment in increased at increased time points (48 hours and 72 hours) (Figure 3C). Treatment with the control antibody, trastuzumab, did not result in a detectable decrease in HER2 levels (Figure 3C).

*H561-4 treatment causes caspase-dependent apoptosis*

SKBr3 cells were seeded at 7.5 x $10^4$ cells/ml in a 96 well plate with 100 μl per well and incubated overnight at 37°C and 5% $CO_2$. The next day treatments (H561-4, trastuzumab, wild-type (WT) antigen-binding Fc fragment or IgG1 isotype control) were added to the wells in media to give a final concentrations of 1000 nM, 100 nM, 10 nM, 1 nM, 0.1 nM and 0.01 nM, plus untreated controls. The cells were incubated for 5 days at 37°C and 5% $CO_2$ before the caspase 3/7 activity was measured using the Promega Caspase-Glo 3/7 assay. 50 μl of Caspase 3/7 Glo reagent was added into each well, mixed and then the plate was incubated at RT for 2 hours before luminescence was measured using the Synergy 4 microplate reader (BIOTEK).

H561-4 treatment of SKBr3 cells resulted in significant dose dependent induction of Caspase 3/7 activity (Figure 3D), Treatment with the control antibody, trastuzumab, did not result in a detectable increase in caspase 3/7 activity, nor did treatment with the WT antigen-binding Fc fragment or IgG1 isotype control.

In summary, the above results demonstrate that H561-4 treatment of SKBr3 cells induced apoptosis and resulted in internalisation and degradation of HER2.

Example 4 - In vivo efficacy studies: H561-4 treatment of human patient derived tumor xenograft (PDX) models with HER2 gene copy numbers greater than or equal to 10

The *in vivo* efficacy of H561-4 in tumour models that express HER2 with gene copy numbers greater than or equal to 10 were evaluated using mice bearing human patient derived xenograft tumours. Immunodeficient mice were used enabling the xenotransplantation and growth of human tumours. Mice were implanted with tumours at approximately 5-7 weeks old apart from the GA0060 PDX model where mice were implanted at approximately 6-8 weeks. The GA0060 work was carried out by Crown BioScience which uses a slightly different experimental procedure to the clinical research organisations used for the other PDX models. Any differences in the Crown BioScience experimental procedure are identified below. All experiments were approved by the local authorities and were conducted according to all applicable international, national and local laws and guidelines. Only animals with unobjectionable health were selected to enter testing procedures. Mice were allowed an acclimatization period of at least seven days after their arrival and prior to the start of any experiment. The tumour xenograft used as a test tumor in this study was derived from a surgical specimen from a patient which was directly implanted subcutaneously into nude mice, hence the designation "patient derived tumor xenograft" (PDX). Tumour fragments were obtained from xenografts in serial passage in nude mice. After removal from donor mice, tumours were cut into fragments (2-5 mm diameter or 2-4 mm in diameter for GA0060) and then used for unilateral subcutaneous implantation in to the flank of mice. At randomization, tumour-bearing animals were stratified into the various groups according to tumour volume. Only mice carrying a tumour of appropriate size (50 $mm^3$ to 250

$mm^3$, or 100 $mm^3$ to 300 $mm^3$ for GA0060) were considered for randomization.

Mice were randomized when the required number of mice qualified for randomization. The day of randomization was designated as day 0. The first day of dosing was also day 0. Animals were sacrificed if their tumour exceeded a volume of 2000 $mm^3$. If an animal was sacrificed due to tumour load, then the last observation carried forward (LOCF) was used for subsequent tumour volume values.

For all PDX models apart from the GA0060 PDX model, antibody samples were diluted to 1 mg/ml in PBS and were administered at 10 mg/kg. The dosing regimen was either once weekly or twice weekly depending on the study. The absolute tumor volume (ATV) was determined by two-dimensional measurement with a caliper one day after tumor implantation and then twice weekly (i. e. on the same days on which mice were weighed). Tumor volumes were calculated according to the formula: (a x $b^2$) x 0.5, where a represents the largest and b the perpendicular tumour diameter. Tumour inhibition for a particular day (T/C in %) was calculated from the ratio of the mean absolute tumour volume values (i.e mean growth of tumours) of test versus control groups multiplied by 100%.

$$\text{T/C (Dayx) [\%]} = \frac{\text{Mean tumor volume of the test group on Dayx - Mean tumor volume of the test group on Day0}}{\text{Mean tumor volume of the control group on Dayx - Mean tumor volume of the control group on Day0}} \times 100$$

Day x denotes any day where the minimum (optimal) T/C was observed. Day 0 was the first day of dosing.

For all PDX models apart from the GA0060 PDX model, the minimum (or optimum) T/C% value recorded for a particular test group during an experiment represents the maximum anti-tumour efficacy for the respective treatment. T/C values were calculated if at least 50% of the randomized animals in a group were alive on the day in question.

For the GA0060 PDX model, antibody samples were diluted to 6 mg/ml in PBS and were administered at 60 mg/kg. The dosing regimen was twice weekly depending on the study. The absolute tumor volume (ATV) was determined by two-dimensional measurement with a caliper one day after tumor implantation and then twice weekly. Tumor volumes were calculated according to the formula: (a x $b^2$) x 0.5 where a represents the largest and b the perpendicular tumour diameter. Tumour inhibition for a particular day (T/C in %) was calculated from the ratio of the mean absolute tumour volume values of test versus control groups multiplied by 100%. The difference between the two T/C equations is explained in Example 7.

$$\text{T/C (Day}_x\text{) [\%]} = \frac{\text{Mean absolute tumor volume of the test group on Day}_x}{\text{Mean Absolute volume of the control group on Day}_x} \times 100$$

where x is approximately one dose period after the final dose if at least 50% of the animals in a group were alive.

For simplicity, this equation will be referred to as the updated T/C equation.

*H561-4 efficacy in gastric PDX model GXF281*

GXF281 was isolated from a primary adenocarcinoma of the stomach from a 46 year old male patient and was studied in NMRI nude mice by Oncotest (Freiburg, Germany). H561-4 (10 mg/kg), trastuzumab (10 mg/kg) and PBS as a vehicle control (10ml/kg) were injected once a week for 5 weeks. Each group contained 6 mice. The tumour volume was measured twice a week for up to 81 days and the mean absolute tumour volume was plotted (Figure 4A). Five weekly treatments with H561-4 resulted in complete tumour regression (minimum T/C value of less than 0 %), whereas five weekly treatments with trastuzumab resulted in only moderate efficacy (minimum T/C of 30 %).

*H561-4 efficacy in gastric PDX model GXA3039*

GXA3039 was isolated from a primary adenocarcinoma of the stomach from a 65 year old male patient and was studied in NMRI nude mice by Oncotest (Freiburg, Germany). H561-4 (10 mg/kg), trastuzumab (10 mg/kg) and PBS as a vehicle control (10ml/kg) were injected once a week for 4 weeks. Each group contained 5 mice. The tumour volume was measured twice a week for up to 91 days and the mean absolute tumour volume was plotted (Figure 4B). Four weekly treatments with H561-4 resulted in complete tumour regression (minimum T/C value of less than 0 %), whereas four weekly treatments with trastuzumab resulted in only minor efficacy (minimum T/C of 88 %).

*H561-4 efficacy in Colorectal PDX model CXF1991*

CXF1991 was isolated from a primary adenocarcinoma of the colon from a 59 year old female patient and was studied in NMRI nude mice by Oncotest (Freiburg, Germany). H561-4(10 mg/kg), trastuzumab (10 mg/kg) and PBS as a vehicle control (10ml/kg) were injected once a week for 6 weeks. Each group contained 5 mice. The tumour volume was measured twice a week for up to 62 days and the mean absolute tumour volume was plotted (Figure 4C). Six weekly treatments with H561-4 resulted in complete tumour regression (minimum T/C value of less than 0 %), whereas six weekly treatments with trastuzumab resulted in only minor efficacy (minimum T/C of 57 %).

*H561-4 efficacy in Colorectal PDX model CXF2102*

CXF2102 was isolated from a liver metastasis of an adenocarcinoma of the colon from patient and was studied in NMRI nude mice by Oncotest (Freiburg, Germany), the age and gender of this patient are not known. H561-4 (10 mg/kg), trastuzumab (10 mg/kg) and PBS as a vehicle control (10ml/kg) were injected once a week for 4 weeks. Each group contained 5 mice. The tumour volume was measured twice a week for up to 58 days and the mean absolute tumour volume was plotted (Figure 4D). Four weekly treatments with H561-4 resulted in significant tumour regression (minimum T/C value of less than 0 %), whereas six weekly treatment with trastuzumab caused in no significant effect (minimum T/C of 76 %).

*H561-4 efficacy in gastric PDX model GXA3054*

GXA3054 was isolated from a primary tumour of an adenocarcinoma of the stomach from 65 year old male patient and was studied in NMRI nude mice by Oncotest (Freiburg, Germany). H561-4 (10 mg/kg), and PBS as a vehicle control (10ml/kg) were injected once a week for 5 weeks. There was a break in dosing for 4 weeks, then weekly dosing was resumed for 4 weeks. Each group contained 5 mice, a trastuzumab only group was not included in this model. The tumour volume was measured twice a week for up to 100 days and the mean absolute tumour volume was plotted (Figure 4E). The weekly treatments with H561-4 resulted in tumour regression (minimum T/C value of 17 %).

*H561-4 efficacy in gastric PDX model GXA3038*

GXA3038 was isolated from a primary tumour of an adenocarcinoma of the stomach from 63 year old male patient and was studied in NMRI nude mice by Oncotest (Freiburg, Germany). H561-4 (10 mg/kg), and PBS as a vehicle control (10ml/kg) were injected once a week for 5 weeks. There was a break in dosing for 1 week then weekly dosing was resumed for 4 weeks. Each group contained 5 mice, a trastuzumab only group was not included in this model. The tumour volume was measured twice a week for up to 77 days and the mean absolute tumour volume was plotted (Figure 4F). The weekly treatments with H561-4 resulted in minor tumour regression (minimum T/C value of 55 %).

*H561-4 efficacy in breast PDX model HBCx-13B*

HBCx-13B was isolated from a lymphoid metastasis carcinoma and was studied in athymic nude mice (Hsd;Athymic Nude-Fox1nu) by Xentech (Evry, France). H561-4 (10 mg/kg), trastuzumab (10 mg/kg) and PBS as a vehicle control (10ml/kg) were injected twice a week for 8 weeks. Each group contained 9 mice. The tumour volume was measured twice a week for up to 56 days and the mean absolute tumour volume was plotted (Figure 4G). Twice weekly treatments with H561-4 resulted in significant tumour regression (minimum T/C value of 7 %), whereas six weekly treatments with trastuzumab resulted in only minor efficacy (minimum T/C of 48 %).

*H561-4 efficacy in Gastric PDX model GA0060*

GA0060 was isolated from human gastric cancer and was studied in BALB/c nude mice by Crown BioScience (Beijing, China). H561-4 (30 and 60 mg/kg), trastuzumab (30 mg/kg) and PBS as a vehicle control (10ml/kg) were injected twice a week for 6 weeks each group contained 10 mice. The tumour volume was measured twice a week for up to 41 days and the mean absolute tumour volume was plotted (Figure 4H). The T/C values were calculated using the updated T/C equation. Six bi-weekly treatments with H561-4 resulted in minor tumour regression (T/C value of 49% and 60%, at 60 and 30 mg/kg respectively), whereas six bi-weekly treatment with trastuzumab resulted in tumour regression (T/C value of 25%). 60 mg/kg trastuzumab was not tested because it already caused tumour regression at 30 mg/kg.

Example 5 - H561-4 shows efficacy in a trastuzumab and pertuzumab resistance model

As shown in Example 4 above, H561-4 has an anti-tumour effect in patient derived xenograft (PDX) models with HER2 gene copy numbers greater than or equal to 10. It was also observed the H561-4 has a significantly superior anti-tumour effect to trastuzumab in these PDX models.

Trastuzumab is often used in combination with pertuzumab in the clinic to treat breast and gastric cancer. In the gastric PDX model GXF281, trastuzumab and pertuzumab combination treatment resulted in a reduction in tumour growth. However, the tumour eventually progressed. H561-4 treatment of tumours that had progressed on trastuzumab and pertuzumab combination treatment resulted in complete tumour regression. These results are shown in Figure 5.

Tumour fragments from the patient derived tumour GXF281, were implanted subcutaneously in anesthetised NMRI nu/nu mice. Mice with suitable tumour growth (50 $mm^3$ -250 $mm^3$ tumours) were randomised into 3 groups as follows:

- Group 1: 5 mice received 4 weekly i.v. injections of PBS at 10 ml/kg.
- Group 2: 20 mice received 4 weekly i.v. injections of trastuzumab at 10 mg/kg and pertuzumab at 10 mg/kg.
- Group 3: 5 mice received 4 weekly i.v. injections of H561-4 at 10 mg/kg

On day 53 when the average tumour volume of mice in group 2 had reached 500 $mm^3$, the mice from group 2 (the trastuzumab and pertuzumab combination group) were randomised into 4 groups as follows:

- Group 4: 5 mice received no further treatment.
- Group 5: 5 mice received 7 weekly i.v. injections of trastuzumab at 10 mg/kg and pertuzumab at 10 mg/kg.
- Group 6: 5 mice received 7 weekly i.v. injections of H561-4 at 10 mg/kg
- Group 7: 5 mice received 7 weekly i.v. injections of H561-4 at 3.6 mg/kg

The tumour volumes were monitored twice a week for 105 days (Figure 5). Group 3 showed complete tumour regression with no mice having a detectable tumour after day 46 of the study and no regrowth occurred up to the end of the study (day 105). Group 2 had slower tumour growth than the control group (group 1). Repeated dosing of mice with trastuzumab and pertuzumab did not slow tumour growth further (group 4 and group 5 had similar tumour growth). Treatment with H561-4 (at either 10 mg/kg or 3.6 mg/kg) in mice that had progressed on trastuzumab and pertuzumab combination treatment resulted in significant tumour regression, the average tumour size of group 6 was 19 $mm^3$ on day 105 compared to 716 $mm^3$ before the H561-4 treatment on day 53, and the average tumour size of group 7 was 37.5 $mm^3$ on day 105 compared to 659 $mm^3$ before the H561-4 treatment on day 53.

Example 6 - H561-4 selectively inhibits tumour growth in PDX models with HER2 gene copy numbers greater than or equal to 10

The *in vivo* activity of H561-4 was studied in 23 different patient derived xenograft (PDX) models, all of which were classified as HER2 positive by IHC according to the criteria used by Oncotest. The efficacy of the H561-4 treatment and trastuzumab treatment was assessed based on the minimum T/C value, expressed as a percentage. The T/C value was calculated using the following equation:

$$T/C\ (Day_x)\ [\%] = \frac{\text{Mean tumor volume of the test group on } Day_x \text{ - Mean tumor volume of the test group on } Day_0}{\text{Mean tumor volume of the control group on } Day_x \text{ - Mean tumor volume of the control group on } Day_0} \times 100$$

Day x denotes any day where the minimum (optimal) T/C was observed. Day 0 was the first day of dosing.

Table 4 shows the efficacy criteria as defined by the minimum T/C values (in %):

| | | |
|---|---|---|
| - | Inactive | T/C ≥ 65% |
| +/- | Borderline activity | 50% ≤ T/C < 65% |
| + | Moderate activity | 25% ≤ T/C < 50% |
| ++ | High activity | 10% ≤ T/C < 25% |
| +++ | Very high activity | 5% ≤ T/C < 10% |

(continued)

| | | |
|---|---|---|
| ++++ | Complete remission | T/C < 5% |

*Determination of HER2 Gene Copy Number in the PDX tumour models by quantitative PCR:*

DNA from each of the tumours was provided at an initial concentration of 100ng/μl and subsequently diluted to 5ng/μl in molecular grade water (SIGMA #W4502-1L). A master mix for the PCR reaction was created consisting of 2x Taqman Genotyping Master Mix (Invitrogen, #371355), 1μl HER2 taqman probe and primers set (Invitrogen, Gene Assay ID: Hs00817646 Cat No:4400291) and 1μl housekeeping RNAseP taqman probe and primers set (Invitrogen, #4403326). 20ng of sample DNA was added in dH20 resulting in a final reaction volume of 20ul. The quantitative PCR conditions were as follows: Hold at 95°C for 10 minutes followed by 40 cycles of 95°C for 15 seconds and then 60°C for 60 seconds. After acquisition of raw $C_T$ (Cycle Threshold) data, a manual $C_T$ of 0.2 and an auto-baseline were applied to the results.

All data obtained were normalized by a housekeeping gene (RNAseP taqman probe and primers set, Invitrogen, #4403326) of which it was known that there were 2 copies per cell. The results of the quantitative PCR were expressed in arbitrary units (AU, related to the copy number per cell). First, for each sample the difference between the $C_T$ value (Cycle Threshold) of a housekeeping gene and the HER2 gene was calculated, as follows:

$$\text{Target gene expression} = 2^{(C_T \text{ housekeeping gene (RNAse P)} - C_T \text{ HER2 gene})}$$

Results were analyzed for linear amplification and exported to Copy Caller™ Software version 2.0 (Invitrogen) for copy number determination of the HER2 gene. A DNA reference sample (Roche, #11691112001) found to have 2 copies of HER2 ($C_T$ values of HER2 gene and housekeeping gene were equal, indicating 2 copies per cell of HER2) was included in the assay to enable exact copy number determination and to allow for assay running adjustment. 2 AU was considered to be the normal value for HER2 gene in the reference genomic DNA. Where the copy number obtained for the human standard genomic DNA was not exactly 2 AU, a multiplier was applied to convert it to 2 AU. This multiplier was also applied to the results obtained for the tumour samples. The gene copy number for each sample is listed in Table 5.

Table 5 shows the efficacy as defined by the minimum T/C values (in %) of H561-4 and trastuzumab treated PDX models as well as gene copy number (GCN) in each of the PDX tumour samples tested:

| | **HER2 gene copy number** | **T/C (Treatment/Control tumour volume %)** | |
|---|---|---|---|
| | | H561-4 | Trastuzumab |
| **PDX tumour models with HER2 GCN≥10** | | | |
| HBCx-13B | 23 | 7 | 48 |
| Gastric GXF281 | 15 | <0 | 30 |
| Gastric GXA3039 | 25 | <0 | 88 |
| Gastric GXA 3054 | 76 | 17 | N/A |
| Gastric GXA3038 | 29 | 55 | N/A |
| Colon CXF2102 | 35 | <0 | 76 |
| Colon CXF1991 | 32 | <0 | 57 |
| **PDX tumour models with HER2 GCN<10** | | | |
| Breast MAXF583 | 2 | 65 | 60 |
| Breast MAXF508 | 1 | 147 | 126 |
| Colon CXF647 | 2 | 76 | 47 |
| Colon CXF1103 | 2 | 86 | 41 |
| Colon CXF975 | 6 | 98 | 170 |
| Colon CXF1729 | 2 | 127 | 65 |
| Colon CXF260 | 2 | 68 | 68 |

(continued)

| PDX tumour models with HER2 GCN<10 | | | |
|---|---|---|---|
| Colon CXF1034 | 2 | 155 | 56 |
| Gastric GXA3067 | 9 | 98 | N/A |
| Gastric GXA3005 | 2 | 106 | 47 |
| Lung LXFA983 | 4 | 71 | 86 |
| Lung LXFE690 | 2 | 121 | 59 |
| Ovarian OVXF1023 | 2 | 115 | 74 |
| Pancreatic PAXF736 | 3 | 62 | N/A |
| Pancreatic PAXF2005 | 2 | 96 | 69 |

H561-4 had efficacy in 6 out of 7 PDX models with a HER2 gene copy number (GCN) greater than or equal to 10, whereas there was only borderline activity in 2 of the PDX models with a HER2 GCN of less than 10. Trastuzumab had moderate activity in 2 PDX models with a GCN greater than or equal to 10 and borderline activity in another. In the PDX models with a GCN of less than 10, trastuzumab had borderline activity in 3 PDX models and moderate activity in another 3 models. Figure 6 shows a scatter plot of the T/C values of H561-4 and trastuzumab treatment in PDX models with a HER2 GCN greater than or equal to 10, compared with PDX models with GCN of less than 10. The scatter plot shows that H561-4 has statistically greater efficacy in cancers with GCN greater than or equal to 10 compared with cancers with GCN of less than 10. Trastuzumab activity is independent of HER2 gene copy number value.

*Determination of mRNA levels in the PDX tumour models by Reverse- Transcription (RT) PCR followed by quantitative PCR:*

mRNA from each of the tumours was provided at a concentration of 250ng/μl. mRNA was reverse transcribed using the High Capacity cDNA Reverse Transcription Kit (Invitrogen, #N8080234) as per the manufacturer's instructions. Transcribed cDNA was then quantified using a Nanodrop 2000 (Thermo Scientific) to determine cDNA concentration in ng/μl. The cDNA obtained was diluted to 25ng/μl in molecular grade water (SIGMA #W4502-1L). A master mix for the quantitative PCR reaction was created consisting of 2x Taqman Gene Expression Master Mix (Invitrogen, # 4369016), 1.25μl HER2 taqman probe and primers set (Invitrogen, Hs01001580_m1 #4331182) and 1.25μl housekeeping human TBP taqman probe and primers set (Invitrogen, Hs00427620_m1 #4331182). 50ng of sample cDNA was added along with $dH_20$ to produce a final reaction volume of 25μl. The quantitative PCR conditions were as follows: Hold at 95°C for 10 minutes followed by 50 cycles of 95°C for 15 seconds and then 61°C for 30 seconds. After acquisition of raw $C_T$ (Cycle Threshold) data, a manual $C_T$ of 0.2 and an auto-baseline were applied to the results.

A "human standard cDNA" reverse-transcribed from a "universal human reference RNA" (Agilent technologies, #740000) made by pooling 10 human cell lines, which are expected to have two copies of the HER2 gene per cell (as the cell lines are considered to have a normal profile) was used as a reference at the same concentration as the tumour sample cDNA. This human standard cDNA is considered to have a normal mRNA profile.

All data obtained were normalized by a housekeeping gene (housekeeping human TBP taqman probe and primers set (Invitrogen, Hs00427620_m1 #4331182) which is known to have a constant expression level across many tissue types. The results of the quantitative-PCR were expressed in arbitrary units (AU). First, for each sample the difference between the $C_T$ value (Cycle Threshold) of a housekeeping gene and the HER2 gene was calculated, as follows:

$$\text{HER2 expression} = 2^{(C_T\ \text{housekeeping gene(TBP)} - C_T\ \text{HER2 gene})}$$

Results were analyzed for linear amplification and exported to Copy Caller™ Software version 2.0 (Invitrogen). This software was used to determine the cDNA copy number of HER2 relative to the cDNA copy number of the housekeeping gene in the tumour samples, normalised to the HER2 cDNA copy number in the human standard cDNA sample.

Normalisation was performed by setting 2 AU to be the normal value for the HER2 cDNA copy number relative to the cDNA copy number of the housekeeping gene in the human standard cDNA. A multiplier (constant) was applied to convert it to 2 AU. This multiplier (constant) was also applied to the results obtained for the tumour samples and the resulting cDNA copy number is listed in Table 6 as the mRNA level.
i.e. HER2 expression of the standard cDNA x constant = 2

HER2 expression in the tumour sample x constant = cDNA copy number

**[0148]** Where constant is the multiplier used to normalised the HER2 expression in the standard cDNA to a value of 2.

Table 6 shows the efficacy as defined by the minimum T/C values (in %) of H561-4 and trastuzumab treated PDX models as well as the HER2 mRNA levels for each of the PDX tumour samples.

| | **HER2 mRNA level** | **T/C (Treatment/Control tumour volume %)** | |
|---|---|---|---|
| | | H561-4 | Trastuzumab |
| **PDX tumour models with HER2 mRNA>200** | | | |
| HBCx-13B | 550 | 7 | 48 |
| Gastric GXF281 | 392 | <0 | 30 |
| Gastric GXA3039 | 775 | <0 | 88 |
| Gastric GXA 3054 | 820 | 17 | N/A |
| Gastric GXA3038 | 1930 | 55 | N/A |
| Colon CXF2102 | 540 | <0 | 76 |
| Colon CXF1991 | 229 | <0 | 57 |
| **PDX tumour models with HER2 mRNA<200** | | | |
| Breast MAXF583 | 12 | 65 | 60 |
| Breast MAXF508 | 15 | 147 | 126 |
| Colon CXF647 | 11 | 76 | 47 |
| Colon CXF1103 | 10 | 86 | 41 |
| Colon CXF975 | 15 | 98 | 170 |
| Colon CXF1729 | 5 | 127 | 65 |
| Colon CXF260 | 3 | 68 | 68 |
| Colon CXF1034 | 14 | 155 | 56 |
| Gastric GXA3067 | 71 | 98 | N/A |
| Gastric GXA3005 | 16 | 106 | 47 |
| Lung LXFA983 | 6 | 71 | 86 |
| Lung LXFE690 | 7 | 121 | 59 |
| Ovarian OVXF1023 | 17 | 115 | 74 |
| Pancreatic PAXF736 | 26 | 62 | N/A |
| Pancreatic PAXF2005 | 8 | 96 | 69 |

H561-4 had efficacy in 6 out of 7 PDX models with a HER2 mRNA level ≥200 in the tumour, and efficacy in all PDX models with a HER2 mRNA level ≥200 and ≤ 820 in the tumour, whereas there was only borderline activity in 2 of the PDX models with a HER2 mRNA level <200 in the tumour. The HER2 mRNA level in those models which had mRNA levels ≤ 820 was 168, whereas for all models tested the HER2 mRNA level was 248. Trastuzumab had moderate activity in 2 PDX models with a HER2 mRNA level ≥200 and borderline activity in another. In the PDX models with an mRNA level <200, trastuzumab had borderline activity in 3 PDX models and moderate activity in another 3 models. Figure 7 shows a scatter plot of the T/C values of H561-4 and trastuzumab treatment in PDX models with a HER2 mRNA level greater than or equal to 200 in the tumour, compared with PDX models with an mRNA level of less than 200 in the tumour. This shows that H561-4 has statistically greater efficacy in cancers with a HER2 mRNA levels ≥200, compared with cancers with a HER2 mRNA level <200 . In particular, the data show that H561-4 has statistically greater efficacy in cancers with HER2 mRNA levels ≥200 and ≤820, compared with PDX models with a HER2 mRNA levels <200 and >820. Trastuzumab activity is independent of the HER2 mRNA level in the range tested.

Example 7 - H561-4 selectively inhibits tumour growth in PDX models with HER2 gene copy numbers greater than or equal to 10 using an updated measure of T/C values.

The efficacy of the H561-4 treatment and trastuzumab treatment was further assessed using a different equation for calculating T/C values.

The T/C values were calculated using the following equation:

$$T/C\ (Day_x)\ [\%] = \frac{\text{Mean absolute tumor volume of the test group on } Day_x}{\text{Mean absolute volume of the control group on } Day_x} \times 100$$

where x is approximately one dose period after the final dose if at least 50% of the animals in a group were alive.

As shown in Example 4 above, this equation is referred to as the updated T/C equation. This updated equation was designed to facilitate the comparison of data collected from different clinical research institutions.

Two changes were introduced in the updated T/C equation compared to the T/C equation used in Example 6: defining the time point for comparison as Day x and removing basal subtraction.

In the previously used T/C formula Day x denoted any day where the minimum (optimal) T/C observed. Therefore x could be any day over the entire period of each experiment. In the updated T/C equation, Day x becomes a fixed time point of approximately one dose period after the final dose (if at least 50% of the animals in a group were alive). Therefore the updated T/C formula has the advantage of standardising Day x, which allows us to compare the effect of treatments from different studies. Although the basal subtraction step was not included in the updated T/C equation, this was compensated by the ranked distribution of inoculated mice evenly to obtain equal mean tumour sizes across different test groups. Values from mice removed due to tumour load were considered beyond the day of sacrifice using the Last Observation Carried Forward methodology if this increased the group mean.

Using this updated equation, we re-assessed the T/C values of the 23 different patient derived xenograft (PDX) models set out in Tables 5 and 6, and summarised together with the HER2 gene copy number (GCN) in Table 7, and with the HER2 mRNA level in Table 8. T/C efficacy values were determined as shown in Table 4.

Gastric GXA 3054 and Gastric GXA3038 PDX models with trastuzumab and this information was also included in Tables 7 and 8.

Table 7 shows the efficacy as defined by the T/C values (%)(using the updated T/C formula) of H561-4 and trastuzumab treated PDX models.

| | **HER2 gene copy number By qPCR** | **T/C (Treatment/Control tumour volume %)** | |
|---|---|---|---|
| | | H561-4 | Trastuzumab |
| ***PDX tumour models with HER2 GCN≥10 by qPCR*** | | | |
| HBCx-13B | 23 | 14.0 | 63.6 |
| Gastric GXF281 | 15 | 1.9 | 54.0 |
| Gastric GXA3039 | 25 | 1.4 | 93.7 |
| Gastric GXA 3054 | 76 | 15.5* | 33.4 |
| Gastric GXA3038 | 29 | 57.0* | 48.8 |
| Colon CXF2102 | 35 | 11.1 | 58.3 |
| Colon CXF1991 | 32 | 8.8 | 77.6 |
| Gastric GA0060 | 43 | 49.2+ | 25.1* |
| ***PDX tumour models with HER2 GCN<10 by qPCR*** | | | |
| Breast MAXF583 | *2* | 85.3 | 68.5 |
| Breast MAXF508 | *1* | 150.7 | 141.1 |
| Colon CXF647 | *2* | 85.9 | 67.4 |
| Colon CXF1103 | *2* | 115.0 | 76.1 |
| Colon CXF975 | *6* | 90 | 147 |
| Colon CXF1729 | *2* | 129.9 | 67.6 |

(continued)

| | | | |
|---|---|---|---|
| ***PDX tumour models with HER2 GCN<10 by qPCR*** | | | |
| Colon CXF260 | *2* | 83.1 | 80.3 |
| Colon CXF1034 | *2* | 173.6 | 66.6 |
| Gastric GXA3067 | *9* | 95.9 | N/A |
| Gastric GXA3005 | *2* | 148.3 | 63.9 |
| Lung LXFA983 | *4* | 76.8 | 103.4 |
| Lung LXFE690 | *2* | 113.8 | 74.7 |
| Ovarian OVXF1023 | *2* | 112.7 | 78.9 |
| Pancreatic PAXF736 | *3* | 84.0 | N/A |
| Pancreatic PAXF2005 | *2* | 145.2 | 104.4 |
| *+These groups were treated with 30* and 60+ mg/kg H561-4 to reach maximal efficacy observed in the in vivo study. | | | |

H561-4 had efficacy in 7 out of 8 PDX models with HER2 gene copy number of greater than 10, 6 of which had high activity, whereas there was no activity in all of the PDX models with HER2 GCN less than 10. Trastuzumab had moderate activity in 3 PDX models with GCN greater than 10 and borderline activity in another 3, but none of them showed high activity. In the PDX models with GCN less than 10 trastuzumab had borderline activity in 1 model.

Figure 8 shows a scatter plot of the T/C values of H561-4 and trastuzumab treatment in PDX models with HER2 GCN greater than 10 compared to PDX models with GCN less than 10. The scatter plot shows that H561-4 has statistically greater efficacy in PDX models with GCN greater than 10 compared to PDX models with GCN less than 10. The mean activities of trastuzumab in PDX models with GCN greater than or less than 10 are both higher the 50%, supporting the notion that HER2 gene copy number value are not predictive to the trastuzumab efficacy.

In summary, the T/C values obtained using the updated T/C equation led to the same conclusion as before (which is described in Example 6).

Table 8 shows the efficacy as defined by the T/C values (%) (using the updated T/C formula) of H561-4 and trastuzumab treated PDX models.

| | **HER2 mRNA number By RT-PCR** | **T/C (Treatment/Control tumour volume %)** | |
|---|---|---|---|
| | | H561-4 | Trastuzumab |
| ***PDX tumour models with HER2 mRNA ≥200 by RT-PCR*** | | | |
| HBCx-13B | *550* | 14.0 | 63.6 |
| Gastric GXF281 | *392* | 1.9 | 54.0 |
| Gastric GXA3039 | *775* | 1.4 | 93.7 |
| Gastric GXA 3054 | *820* | 15.5* | 33.4 |
| Gastric GXA3038 | *1930* | 57.0* | 48.8 |
| Colon CXF2102 | *540* | 11.1 | 58.3 |
| Colon CXF1991 | *229* | 8.8 | 77.6 |
| Gastric GA0060 | *1045* | 49.2+ | 25.1* |
| ***PDX tumour models with HER2 mRNA <200 by RT-PCR*** | | | |
| Breast MAXF583 | *12* | 85.3 | ’ 68.5 |
| Breast MAXF508 | *15* | 150.7 | 141.1 |
| Colon CXF647 | *11* | 85.9 | 67.4 |
| Colon CXF1103 | *10* | 115.0 | 76.1 |
| Colon CXF975 | *15* | 90 | 147 |

(continued)

| PDX tumour models with HER2 mRNA <200 by RT-PCR | | | |
|---|---|---|---|
| Colon CXF1729 | *5* | 129.9 | 67.6 |
| Colon CXF260 | *3* | 83.1 | 80.3 |
| Colon CXF1034 | *14* | 173.6 | 66.6 |
| Gastric GXA3067 | *71* | 95.9 | N/A |
| Gastric GXA3005 | *16* | 148.3 | 63.9 |
| Lung LXFA983 | *6* | 76.8 | 103.4 |
| Lung LXFE690 | *7* | 113.8 | 74.7 |
| Ovarian OVXF1023 | *17* | 112.7 | 78.9 |
| Pancreatic PAXF736 | *26* | 84.0 | N/A |
| Pancreatic PAXF2005 | *8* | 145.2 | 104.4 |
| *+These groups were treated with 30* and 60+ mg/kg H561-4 to reach maximal efficacy observed in the in vivo study. | | | |

H561-4 had efficacy in 7 out of 8 PDX models with a HER2 mRNA level ≥ 200 in the tumour, and efficacy in all PDX models with a HER2 mRNA level ≥ 200 and ≤ 820 in the tumour, whereas there was no activity in all of the PDX models with a HER2 mRNA level < 200 in the tumour. Trastuzumab had moderate activity in 3 PDX models with a HER2 mRNA level ≥ 200 and borderline activity in another 3, but none of them showed high activity. In the PDX models with an mRNA level < 200, trastuzumab had borderline activity in 1 model.

Figure 9 shows a scatter plot of the T/C values of H561-4 and trastuzumab treatment in PDX models with a HER2 mRNA level greater than or equal to 200 in the tumour, compared with PDX models with an mRNA level of less than 200 in the tumour. The scatter plot shows that H561-4 has statistically greater efficacy in PDX models with a HER2 mRNA level ≥ 200 and ≤ 820 compared to PDX models with a HER2 mRNA levels < 200 and > 820. The mean activities of trastuzumab in PDX models in the groups of HER2 mRNA ≤ 200 and the group < 200 are both higher than 50%, supporting the notion that HER2 gene copy number value are not predictive to the trastuzumab efficacy.

The T/C values obtained using the updated T/C equation led to the same conclusion as before.

Example 8 - H561-4 selectively inhibits PDX models with HER2 gene copy numbers of greater than 18 where the gene copy number is determined by FISH

The HER2 gene copy numbers determined by FISH in relation to the *in vivo* activity of H561-4 was studied in 17 different patient derived xenograft (PDX) models, including breast, gastric and colon models. The efficacy of the H561-4 treatment and trastuzumab treatment was assessed based on the T/C value using the updated T/C equation:

$$T/C\ (Day_x)\ [\%] = \frac{\text{Mean absolute tumor volume of the test group on } Day_x}{\text{Mean Absolute volume of the control group on } Day_x} \times 100$$

The efficacy criteria as defined by the T/C values are set out in Table 4.

*Determination of HER2 Gene Copy Number in the PDX tumour models by Fluorescence In Situ Hybridization (FISH):*

Formalin-fixed, paraffin-embedded (FFPE) cancer tissue specimens were obtained from the PDX models. FISH assay was performed using the PathVysion HER-2 DNA probe kit II (Abbott Molecular #06N46-030). This assay used direct counting of the HER2 signals without normalisation to CEP17 signal. The tissue slides were pre-treated to be deparaffinised in xylene three times for 5 minutes, followed by rehydration in industrial methylated spirits (IMS) two times for 5 minutes and incubation of citrate buffer pH 6.0 at 95-99 °C for 30 minutes. After the pre-treatment, the slides were incubated at 37 °C for 20 minutes in pepsin protease buffer for protease digestion. For efficient marking of the target HER2 DNA sequence, the slides were heated to 73 °C for 5 minutes for DNA denaturation, followed by probe hybridisation at 37 °C for 14-18 hours. Unspecific hybridization events were washed away in the post-hybridization buffer at 72 °C for 2 minutes. The slides were then incubated with DAPI counterstain for 15 minutes in the dark until viewing using fluorescence microscopy for signal enumeration. Enumeration was evaluated based on 20-60 tumour cells per sample and an average of the HER2 gene copy number was taken. The HER2 gene copy number determined by FISH for each sample is listed in Table 9.

Table 9 shows the efficacy as defined by the T/C values (in %) of H561-4 and trastuzumab treated PDX models as well as gene copy number (GCN) in each PDX tumour tested:

| | HER2 gene copy number (by FISH) | T/C Treatment/Control tumour volume %) | |
|---|---|---|---|
| | | H561-4 | Trastuzumab |
| ***PDX tumour models with HER2 GCN≥18 (by FISH)*** | | | |
| HBCx-13B | 21 | 14.0 | 63.6 |
| Gastric GXF281 | 25 | 1.9 | 54.0 |
| Gastric GXA3039 | 30 | 1.4 | 93.7 |
| Gastric GXA 3054 | 40 | 15.5* | 33.4 |
| Gastric GXA3038 | 30 | 57.0* | 48.8 |
| Colon CXF2102 | 25 | 11.1 | 58.3 |
| Colon CXF1991 | 25 | 8.8 | 77.6 |
| Gastric GA0060 | 40 | 49.2+ | 25.1 |
| ***PDX tumour models with HER2 GCN<18 (by FISH)*** | | | |
| Gastric GXA3067 | 17 | 95.9 | N/A |
| Breast MAXF583 | 3 | 85.3 | 68.5 |
| Breast MAXF508 | 2 | 150.7 | 141.1 |
| Colon CXF647 | 2 | 85.9 | 67.4 |
| Colon CXF1103 | 2 | 115.0 | 76.1 |
| Colon CXF1729 | 2 | 129.9 | 67.6 |
| Colon CXF260 | 2 | 83.1 | 80.3 |
| Colon CXF1034 | 2 | 173.6 | 66.6 |
| Gastric GXA3005 | 2 | 148.3 | 63.9 |
| *+These groups are treated with 30* and 60+ mg/kg H561-4 to reach maximal efficacy observed in the in vivo study. | | | |

H561-4 had efficacy in 7 out of 8 PDX models with HER2 gene copy number of greater than 18 as determined by FISH, 6 of which had high activity, whereas there was no activity in all of the PDX models with HER2 GCN less than 18. Trastuzumab had moderate activity in 3 PDX models with GCN greater than 18 and borderline activity in another 3, but none of them showed high activity. In the PDX models with GCN less than 18 trastuzumab had borderline activity in 1 model.

Figure 10 shows a scatter plot of the T/C values of H561-4 and trastuzumab treatment in PDX models with HER2 GCN greater than 18 compared to PDX models with GCN less than 18. The scatter plot shows that H561-4 has statistically greater efficacy in PDX models with GCN greater than 18 compared to PDX models with GCN less than 18. Trastuzumab activity is independent of HER2 gene copy number value. The mean activities of trastuzumab in PDX models in the group with GCN greater than 18 or in the group with less than 18 are both higher than 50%, supporting the notion that HER2 gene copy number value are not predictive to the trastuzumab efficacy.

Example 9 - Determination of HER2 gene copy number by FISH and qPCR and their relationship

As shown in Example 6 and Example 8 above, the HER2 gene copy numbers were determined by qPCR and FISH. HER2 GCN in PDX tumours determined by the qPCR varied from 1 to 76 copies, while GCN in these tumors as determined by FISH varied from 2 to 40 copies. Tumours with high HER2 gene amplification determined by either method (GCN greater than or equal to 10 by qPCR or GCN greater than or equal to 18 by FISH) are statistically more likely to respond to H561-4 treatment. The relationship of the GCN determined by the two methods was assessed in 17 PDX tumours where data collected by both methods are available. The HER2 gene copy numbers determined by both methods are listed in Table 10.

Table 10 Summary of HER2 GCN in the PDX tumours determined by qPCR and FISH

| | **HER2 GCN (qPCR)** | **HER2 GCN (FISH)** |
|---|---|---|
| Gastric GXA3054 | *76* | 40 |
| Gastric GA0060 | *43* | 40 |
| Colon CXF2102 | *35* | 25 |
| Colon CXF1991 | *32* | 25 |
| Gastric GXA3038 | *29* | 30 |
| Gastric GXA3039 | *25* | 30 |
| HBCx-13B | *23* | 21 |
| Gastric GXF281 | *15* | 25 |
| Gastric GXA3067 | *9* | 17 |
| Breast MAXF583 | *2* | 3 |
| Colon CXF1034 | *2* | 2 |
| Colon CXF647 | *2* | 2 |
| Colon CXF1103 | *2* | 2 |
| Colon CXF1729 | *2* | 2 |
| Gastric GXA3005 | *2* | 2 |
| Colon CXF260 | *2* | 2 |
| Breast MAXF508 | *1* | 2 |

Correlation analysis was conducted to define the relationship between the HER2 GCN determined by qPCR and that by FISH. The scatter plot shown in Figure 11 shows the relationship of GCN determined by FISH and qPCR in each PDX tumour samples and the points lie closely to the diagonal line that denotes 1:1 correlation. The GCN determined by the two methods are highly correlative based on the correlation analysis (Pearson $r = 0.90$; $p < 0.0001$; 95% CI = 0.74-0.96).

Example 10 - Investigating variability of HER2 IHC scoring determined by different protocols.

HER2 amplification status has been used as a prognostic factor. Immunohistochemical (IHC) assay is a commonly used method for evaluating HER2 protein expression in tissue samples by antibodies that bind specifically to the antigen. As previously noted, the semi-quantitative nature of IHC means that it can be inexact and does not always provide an accurate reading. Furthermore, given that various assay protocols, HER2 antibodies and scoring systems are in use, variability in HER2 IHC results between providers of IHC assays could be a concern.

Although HercepTest has been approved by the FDA as a standardised IHC assay for determination of HER2 protein overexpression in breast and gastric cancers, it has been reported in some studies that most HercepTest HER2 positive samples lacked HER2 gene amplification as determined by FISH assay (Jacobs et al., 1999). On the other hand, breast cancers may exhibit HER2 protein overexpression in the absence of gene amplification, however this phenomenon previously was observed only in 7% of cases (Persons et al., 1997). These discrepancies contradict the current clinical practices to deem the HercepTest 3+ positive population to be HER2 amplified.

In this study, different IHC assays including HercepTest and the immunohistochemical assay developed by Oncotest were used to identify overexpression of HER2 protein in 22 formalin-fixed, paraffin-embedded PDX tumours. The aim was to investigate the reliability of IHC as a H561-4 biomarker.

For the HercepTest IHC assay, the manufacture protocol of HercepTest was used. Briefly, the formalin-fixed, paraffin-embedded cancer tissues were deparaffinised in xylene three times for 5 minutes, followed by rehydration in industrial methylated spirits (IMS) two times for 5 minutes. The slides were then incubated in Epitope Retrieval Solution for 40 minutes at 97 °C and socked in wash buffer for 20 minutes prior to staining. The slides were incubated with the primary rabbit anti-human HER2 antibody for 30 minutes followed by incubation with secondary goat anti-rabbit antibody linked to horseradish peroxidase for 30 minutes. DAB chromogen was applied for enzymatic conversion for signal visualisation.

The immunohistochemical assay developed by Oncotest was carried out by first deparaffinising and rehydration of the tissue. The slides were incubated in 10 mM TriSodium Citrate for antigen retrival for 20 minutes at 720 watt microwave. The samples were permeabilised by 0.2% triton X100 and blocked by 3% $H_2O_2$ to inactivate endogenous peroxidase. The slides were blocked for unspecific binding in 10% BSA and incubated with polyclonal rabbit anti-human HER2 antibody (Dako Cat# A0485) overnight at 4 °C. The slides were incubated the next day with biotinylated goat anti-rabbit IgG (Jackson Cat# 111-065-045) for 60 minutes at room temperature. The biotinylated secondary antibody was detect by incubation with an avidin/biotinylated enzyme complex (ABC complex, Vector Lab Cat#PK-4000) followed by signal development with DAB chromogen.

The American Society of Clinical Oncology (ASCO) scoring guideline was used by both methods. Briefly, HER2 protein overexpression is graded on a scale of 0 to 3+. The level of IHC staining of tumour tissue sections determined by the percentage of tumour expressing HER2 protein and the intensity of the HER2 stain is used to score the HER2 protein expression. The guidelines for the scoring of HER2 by IHC are shown in Table 11 below (in 2013, the assessment of IHC 2+ was updated from 'weakly positive' (as used in the 2007 guidelines) to 'equivocal')

Table 11: ASCO IHC scoring guideline for HER2 protein overexpression

| **Score to report** | **HER2 protein over expression assessment** | **Staining pattern** |
|---|---|---|
| 0 | Negative | No staining observed or membrane staining is observed in less than 10 % of the tumour cells |
| 1+ | Negative | A faint/barely perceptible membrane staining is detectable in more than 10 % of the tumour cells. The cells exhibit incomplete membrane staining. |
| 2+ | Weakly positive (Equivocal) | A weak to moderate complete membrane staining is observed in more than 10 % of tumour cells |
| 3+ | Strongly positive | A strong complete membrane staining is observed in more than 10 % of tumour cells. |

The HER2 IHC overexpression scores determined by HercepTest and by the IHC method developed by Oncotest for each PDX tumour tested in efficacy studies are listed in Table 12. The HER2 IHC results obtained by the two different staining methods showed wide variation, especially the tumours with HER2 GCN $\leq$ 10 by qPCR, despite the fact that both HercepTest and Oncotest use the same scoring guideline. Furthermore, even the repeat among the same method performed by Oncotest (n = 1 vs n = 2) showed inconsistency. Thus, IHC is shown to vary considerably between providers and within multiple assays by the same provider.

The number of HER2 negative cases are much higher by HercepTest compared to IHC performed by Oncotest (8 vs 2 cases for tumours tested by both methods). On the other hand, HercepTest was reported to result in higher positivity rate (Jacobs et al., 1999). Together, these results point toward the variation in HER2 IHC results. Previous studies have stated different issues that could lead to variability in the use of IHC assays for HER2 protein, including variability in tissue fixation and processing and variability in antibody sensitivity and specificity. However, in the current study the primary antibody specificity is unlikely to be the cause as the same antibody was used in both methods.

Comparing the overall HER2 IHC results to the HER2 GCN and HER2 mRNA results (Examples 7 and Example 8), the HER2 GCN determined by either FISH or qPCR methods or the HER2 mRNA are much more predictive in selecting a biomarker positive population to be the responsive patient population to H561-4 ($p < 0.0001$; unpaired t test).

Figure 12 shows a scatter plot of the T/C values of H561-4 and trastuzumab treatment in PDX models with HER2 protein overexpression positive compared to PDX models with HER2 protein overexpression negative determined by HercepTest. Although HercepTest was the FDA-approved companion test kit to select patients for Herceptin treatment, in the 16 samples tested trastuzumab activity was independent of HER2 protein overexpression status ($p = 0.09$; unpaired t test). The mean activities of trastuzumab in PDX models in both HER2 protein overexpression positive or negative are higher than 50%, suggesting the notion that HER2 protein overexpression is not robustly predictive to the trastuzumab efficacy.

Interestingly, the scatter plot shows that H561-4 has statistically greater efficacy in PDX models which are HER2 protein overexpression positive compared to PDX models with HER2 protein overexpression negative, when IHC is determined by HercepTest. These results demonstrate that the HER2 IHC status is more predictive in selecting the responsive population to H561-4 than trastuzumab.

Table 12 Efficacy as defined by the T/C values (in %) of H561-4 and trastuzumab treated PDX models as well as HER2 protein overexpression scoring determined by HercepTest and IHC by Oncotest in each PDX tumour tested:

| | **HER2 overexpression IHC score** | | | **T/C Treatment/Control tumour volume %)** | |
|---|---|---|---|---|---|
| | HercepT est | Oncotest n=1 | Oncotest n=2 | H561-4 | Trastuzumab |
| ***PDXtumour models with HER2 GCN≥10 (by RT-PCR)*** | | | | | |
| Breast HBCx-13B | 2+ | N.D | N.D | 14.0 | 63.6 |
| Gastric GXF281 | 3+ | 3+ | 3+ | 1.9 | 54.0 |
| Gastric GXA3039 | 3+ | 3+ | 3+ | 1.4 | 93.7 |
| Gastric GXA 3054 | 3+ | 3+ | 3+ | 15.5* | 33.4 |
| Gastric GXA3038 | 3+ | 3+ | 3+ | 57.0* | 48.8 |
| Colon CXF2102 | 3+ | 3+ | 3+ | 11.1 | 58.3 |
| Colon CXF1991 | 3+ | 3+ | 3+ | 8.8 | 77.6 |
| Gastric GA0060 | 3+ | N.D | N.D | 49.2$^+$ | 25.1 |
| ***PDX tumour models withHER2GCN<10 (byRT-PCR)*** | | | | | |
| Breast MAXF583 | 0 | 3+ | 2+ | 85.3 | 68.5 |
| Breast MAXF508 | 1+ | 3+ | 2+ | 150.7 | 141.1 |
| Colon CXF647 | 0 | 3+ | 2+ | 85.9 | 67.4 |
| Colon CXF1103 | 0 | 3+ | 1+ | 115.0 | 76.1 |
| Colon CXF1729 | 0 | 3+ | 2+ | 129.9 | 67.6 |
| Colon CXF260 | 0 | 2+ | 1+ | 83.1 | 80.3 |
| Colon CXF1034 | 1+ | 3+ | 3+ (2+) | 173.6 | 66.6 |
| Gastric GXA3067 | 3+ | 3+ | 2+ | 95.9 | N.D |
| Gastric GXA3005 | 0 | 3+ | 2+ | 148.3 | 63.9 |
| Lung LXFA983 | N.D | 2+ | 1+ | 76.8 | 103.4 |
| Lung LXFE690 | N.D | 3+ | 2+ | 113.8 | 74.7 |
| Ovarian OVXF1023 | N.D | 3+ | 2+ | 112.7 | 78.9 |
| Pancreatic PAXF736 | N.D | 2+ | 1+ | 84.0 | N.D |
| Pancreatic PAXF2005 | N.D | 2+ | 1+ | 145.2 | 104.4 |
| *$^+$These groups are treated with 30* and 60$^+$ mg/kg H561-4 to reach maximal efficacy observed in the in vivo study. | | | | | |

## Informal sequence listing

Nucleotide sequence of antigen-binding Fc fragment H561-4 (SEQ ID NO: 1)

ACCTGCCCCCCTTGTCCTGCCCCCGAGCTGCTGGGAGGCCCTTCCGTGTTTCTGTTCCCC
CCAAAGCCCAAGGACACCCTGATGATCTCCCGGACCCCCGAAGTGACCTGCGTGGTGGTGGACGTG
TCCCACGAGGACCCTGAAGTGAAGTTCAATTGGTACGTGGACGGCGTGGAAGTGCACAACGCCAAG
ACCAAGCCCAGAGAGGAACAGTACAACTCCACCTACCGGGTGGTGTCCGTGCTGACCGTGCTGCAC
CAGGACTGGCTGAACGGCAAAGAGTACAAGTGCAAGGTGTCCAACAAGGCCCTGCCTGCCCCCATC
GAAAAGACCATCTCCAAGGCCAAG*GGCCAGCCCCGCGAGCCCCAGGTGTACACCCTGCCCCCTAGC*
*CGGGACGAG**TTCTTCACCTACTGG**GTGTCCCTGACCTGCCTGGTCAAGGGCTTCTACCCCTCCGAT*
*ATCGCCGTGGAATGGGAGTCCAACGGCCAGCCCGAGAACAACTACAAGACCACCCCCCCTGTGCTG*
*GACTCCGACGGCTCATTCTTCCTGTACTCCAAGCTGACCGTG**GACCGGCGGAGATGGACCGCC**GGC*
*AACGTGTTCTCCTGCAGCGTGATGCACGAGGCCCTGCACAACCACTACACCCAGAAGTCCCTGTCC*
*CTGAGCCCCGGCAAG*TGATGAGAATTC

Sequence "ACCTGCCCCCCTTGTCCT" at the start of the H561-4 nucleotide sequence encodes part of the IgG1 hinge region. The sequence encoding the CH2 domain of H561-4 is underlined. The sequence encoding the CH3 domain of H561-4 is shown in italics. The sequence encoding the part of the AB structural loop of the CH3 domain believed to be involved in antigen binding is shown in bold, and the part of the sequence encoding the EF structural loop of the CH3 domain believed to be involved in antigen binding is shown in bold and doubly underlined.

Nucleotide sequence of the antigen-binding Fc fragment H561-4 hinge region (SEQ ID NO: 2)

ACCTGCCCCCCTTGTCCT

Nucleotide sequence of the antigen-binding Fc fragment H561-4 CH2 domain (SEQ ID NO: 3)

GCCCCCGAGCTGCTGGGAGGCCCTTCCGTGTTTCTGTTCCCC
CCAAAGCCCAAGGACACCCTGATGATCTCCCGGACCCCCGAAGTGACCTGCGTGGTGGTGGACGTG
TCCCACGAGGACCCTGAAGTGAAGTTCAATTGGTACGTGGACGGCGTGGAAGTGCACAACGCCAAG
ACCAAGCCCAGAGAGGAACAGTACAACTCCACCTACCGGGTGGTGTCCGTGCTGACCGTGCTGCAC
CAGGACTGGCTGAACGGCAAAGAGTACAAGTGCAAGGTGTCCAACAAGGCCCTGCCTGCCCCCATC
GAAAAGACCATCTCCAAGGCCAAG

Nucleotide sequence of the antigen-binding Fc fragment H561-4 CH3 domain (SEQ ID NO: 4)

GAAAAGACCATCTCCAAGGCCAAGGGCCAGCCCCGCGAGCCCCAGGTGTACACCCTGCCCCCTAGC
CGGGACGAGTTCTTCACCTACTGGGTGTCCCTGACCTGCCTGGTCAAGGGCTTCTACCCCTCCGAT
ATCGCCGTGGAATGGGAGTCCAACGGCCAGCCCGAGAACAACTACAAGACCACCCCCCCTGTGCTG
GACTCCGACGGCTCATTCTTCCTGTACTCCAAGCTGACCGTGGACCGGCGGAGATGGACCGCCGGC
AACGTGTTCTCCTGCAGCGTGATGCACGAGGCCCTGCACAACCACTACACCCAGAAGTCCCTGTCC
CTGAGCCCCGGCAAG

Nucleotide sequence encoding the part of the H561-4 AB structural loop region believed to be involved in antigen binding (SEQ ID NO: 5)

TTCTTCACCTACTGG

Nucleotide sequence encoding part of the H561-4 CD structural loop region (SEQ ID NO: 6)

AACGGCCAGCCCGAG

Nucleotide sequence encoding the part of the H561-4 EF structural loop region believed to be involved in antigen binding (SEQ ID NO: 7)

GACCGGCGGAGATGGACCGCC

Amino acid sequence of antigen-binding Fc fragment H561-4 (SEQ ID NO: 8)

TCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAK*GQPREPQVYTLPPSRDE***FFT**
**YW***VSLTCLVKGFYPSDIAVEWES*NGQPE*NNYKTTPPVLDSDGSFFLYSKLTV***DRRRWTA***GNVFSCSVM*
*HEALHNHYTQKSLSLSPGK*

Sequence "TCPPCP" at the start of the H561-4 amino acid sequence represents part of the IgG1 hinge region. The CH2 domain of H561-4 is underlined. The CH3 domain of H561-4 is shown in italics. Part of the AB structural loop of the CH3 domain is shown in bold and part of the EF structural loop of the CH3 domain is shown in bold and doubly underlined. Cleavage of the C-terminal Lysine (*K*) or C-terminal lysine and the adjacent glycine (*GK*) of the CH3 domain has been reported. The C-terminal lysine and the adjacent glycine (also present in SEQ ID NO: 11, below) are shown boxed in the above sequence.

Amino acid sequence of the H561-4 hinge region (SEQ ID NO: 9)

TCPPCP

Amino acid sequence of the H561-4 CH2 domain (SEQ ID NO: 10)

APELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAK

Amino acid sequence of the H561-4 CH3 domain (SEQ ID NO: 11)

GQPREPQVYTLPPSRDE**FFTYW**VSLTCLVKGFYPSDIAVEWESNGQPENNYKTTPPVLDSDGSFFLYS KLTV**DRRRWTA**GNVFSCSVMHEALHNHYTQKSLSLSPGK

Amino acid sequence of the part of the H561-4 AB structural loop region believed to be involved in antigen binding (SEQ ID NO: 12)

FFTYW

Amino acid sequence of part of the H561-4 CD structural loop region (SEQ ID NO: 13)

NGQPE

Amino acid sequence of the part of the H561-4 EF structural loop region believed to be involved in antigen binding (SEQ ID NO: 14)

DRRRWTA

HER2 amino acid sequence bound by H561-4 as determined by Pepscan (SEQ ID NO: 15)

LPASPETHLDMLRHL

HER2 amino acid sequence bound by H561-4 as determined by Pepscan (SEQ ID NO: 16)

CQVVQGNLELTYLPT

HER2 amino acid sequence bound by H561-4 as determined by Pepscan (SEQ ID NO: 17)

SLTLQGLGISWLGLRSLRELGSGLALIHHNTHLCFVHTVPWDQLFRNPHQAL

Amino acid sequence of the HER2 extra cellular domain (SEQ ID NO: 18)

```
        10         20         30         40         50         60
TQVCTGTDMK LRLPASPETH LDMLRHLYQG CQVVQGNLEL TYLPTNASLS FLQDIQEVQG

        70         80         90        100        110        120
YVLIAHNQVR QVPLQRLRIV RGTQLFEDNY ALAVLDNGDP LNNTTPVTGA SPGGLRELQL

       130        140        150        160        170        180
RSLTEILKGG VLIQRNPQLC YQDTILWKDI FHKNNQLALT LIDTNRSRAC HPCSPMCKGS

       190        200        210        220        230        240
RCWGESSEDC QSLTRTVCAG GCARCKGPLP TDCCHEQCAA GCTGPKHSDC LACLHFNHSG
```

```
       250        260        270        280        290        300
ICELHCPALV TYNTDTFESM PNPEGRYTFG ASCVTACPYN YLSTDVGSCT LVCPLHNQEV

       310        320        330        340        350        360
TAEDGTQRCE KCSKPCARVC YGLGMEHLRE VRAVTSANIQ EFAGCKKIFG SLAFLPESFD

       370        380        390        400        410        420
GDPASNTAPL QPEQLQVFET LEEITGYLYI SAWPDSLPDL SVFQNLQVIR GRILHNGAYS

       430        440        450        460        470        480
LTLQGLGISW LGLRSLRELG SGLALIHHNT HLCFVHTVPW DQLFRNPHQA LLHTANRPED

       490        500        510        520        530        540
ECVGEGLACH QLCARGHCWG PGPTQCVNCS QFLRGQECVE ECRVLQGLPR EYVNARHCLP

       550        560        570        580        590        600
CHPECQPQNG SVTCFGPEAD QCVACAHYKD PPFCVARCPS GVKPDLSYMP IWKFPDEEGA

       610        620        630
CQPCPINCTH SCVDLDDKGC PAEQRASPLT
```

Amino acid sequence of wild-type Fc antigen binding fragment used as control in the experiments (SEQ ID NO: 19)

```
TCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVM
HEALHNHYTQKSLSLSPGK
```

References

Arnould et al., Accuracy of HER2 status determination on breast core-needle biopsies (immunohistochemistry, FISH, CISH and SISH vs FISH). Mod Path. 2012. 25: 675-682 Beck et al. Characterization of Therapeutic Antibodies and Related Products. Anal. Chem. 2013, 85, 715-736

Berchuck, A. et al., Overexprerssion of HER2/neu in endometrial cancer is associated with advanced stage disease. AJOG; 164 (1): 15-21, 1991.

Brabender, J et al., Epidermal Growth Factor Receptor and HER2-neu mRNA Expression in Non-Small Cell Lung Cancer is Correlated with Survival. Clin Cancer Res; 7: 1850, 2001. CRUK and WHO world cancer report. World Cancer Report 2008, Editor Boyle P, et al., WHO International Agency for Research on Cancer, ISBN 9789283204237

Dowsett, M et al., Disease-free survival according to degree of HER2 amplification for patients treated with adjuvant chemotherapy with or without 1 year of trastuzumab, the HERA trial, J Clin Oncol, 27(18):2962-9, 2009.

Garcia-Caballero et al., Determination of HER2 amplification in primary breast cancer using dual-color chromogenic in situ hybridization is comparable to fluorescence in situ hybridization: a European multicentre study involving 168 specimens. Histopathology. 2010. 56: 472-480

Gravalos, C and Jimeno, A. Her2 in gastric cancer: a new prognostic factor and a novel therapeutic target. Ann Oncol; 19 (9): 1523-1529, 2008.

Gun, S et al., Molecular cytogenetics as a clinical test for prognostic and predictive biomarkers in newly diagnosed ovarian cancer. J Ovarian Res 6:2, 2013.

Hamburg M.A and Collins, F.S. The path to personalised medicine,10.1056/NEJMp1006304, 15 June 2010.

Hanna et al., HER2 in situ hybridization in breast cancer: Clinical implications of polysomy 17 and genetic heterogeneity. Mod Pathol: 27: 4-18 (2014)

Harris L et al., Predictors of Resistance to Preoperative Trastuzumab and Vinorelbine for HER-2 Positive Early Breast Cancer. Clin Cancer Res, 13:1198-1207, 2007.

Hicks D, et al., Assessment of the HER2 status in breast cancer by Fluorescence In Situ Hybridisation: a technical review with interpretive guidelines. Hum Pathol, 36:250-261, 2005.

Hoff, E et al., HER2/neu amplification in breast cancer. Am J Clin Pathol, 117:916-921, 2002.

Hudis, C. Trastuzumab - Mechanism of Action and Use in Clinical Practice. N Engl J Med, 357:39-51, 2007

Jacobs, T.W. et al. - Specificity of HercepTest in Determining HER-2/neu Status of Breast Cancers Using the United States Food and Drug Administration-Approved Scoring System. Journal of Clinical Oncology, Vol 17, No 7 (July), 1999: pp 1983-1987.

Lanitis et al., Primary Human Ovarian Epithelial Cancer Cells Broadly Express HER2 at Immunologically-Detectable Levels. PLOSone;7 (11):e49829, 2012.

Lei et al., Overexpression of HER2/neu oncogene in pancreatic cancer correlated with shortened survival. International Journal of Pancreatology; 17 (1), 15-21, 1995

Mollerup et al., Dual color chromogenic in situ hybridization for determination of HER2 status in breast cancer: a large comparative study to current state of the art fluorescence in situ hybridization. BMC Clinical Path. 2012. 12:3

Ochs et al., Expression of Vascular Endothelial Growth Factor and HER2/neu in Stage II Colon Cancer and Correlation with Survival. Clinical Colorectal Cancer; 4 (4):262-267, 2004.

Persons et al., Quantitation of HER-2/neu and c-myc gene amplification in breast carcinoma using fluorescence in situ hybridization. Mod Pathol 10:720-727, 1997

Press et al., Evaluation of HER-2/neu gene amplification and overexpression: comparison of frequently used assay methods in a molecularly characterized cohort of breast cancer specimens. JCO, 20 (14):3095-3105, 2002.

Rüschoff et al., HER2 diagnostics in gastric cancer- guideline validation and development of standardized immunohistochemical testing. Vichows Arch; 457:299-307, 2010.

Ross J. Breast cancer biomarkers and HER2 testing after 10 years of anti-HER2 therapy. Drug News Perspect; 22:93-106, 2009

Ross J et a/., The HER2/neu Gene and Protein in Breast Cancer 2003: Biomarker and Target of Therapy. The Oncologist, 8:307-325, 2003.

Timmerman et al. (2007). Functional reconstruction and synthetic mimicry of a conformational epitope using CLIPS™ technology. J Mol Recognit; 20:283-99.

Slootstra et al. (1996). Structural aspects of antibody-antigen interaction revealed through small random peptide libraries. Molecular Diversity, 1:87-96.

Vinatzer et al., Expression of HER2 and the Coamplified Genes GRB7 and MLN64 in Human Breast Cancer: Quantatative Real-time Reverse Transcription PCR as a Diagnostic Alternative to Immunohistochemistry and Fluorescence In situ Hybridisation. Clin Cancer Res; 11 (23):8348-8357, 2005.

Wolff A et al., American Society of Clinical Oncology/College of American Pathologists Guideline Recommendations for Human Epidermal Growth Factors Receptor 2 Testing in Breast Cancer. J Clin Oncol; 25:118/145, 2007

Wolff et al., Recommendations for Human Epidermal Growth Factor Receptor 2 Testing in Breast Cancer: American Society of Clinical Oncology/College of American Pathologists Clinical Practice Guideline Update. J Clin Onco; 31(31):3997-4013, 2013.

Wong et al. (2011) Intergrating molecular and clinical evidence in the management of trastuzumab resistant or refractory HER-2+ Metastatic breast cancer, The Oncologist; 16:1535-1546.

Yarden, Y. Biology of HER2 and Its Importance in Breast Cancer. Oncology; 61 (2): 1-13, 2001.

## Claims

1. A specific binding member which binds to Human Epidermal Growth Factor Receptor 2 (HER2) for use in a method of treating cancer in a patient, wherein said cancer has an average HER2 gene copy number of greater than or equal to 10 per tumour cell, and wherein the specific binding member is:

   (a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
   (b) a specific binding member which competes with a specific binding member according to (a) for binding to HER2.

2. A specific binding member for use according to claim 1, wherein a tumour sample obtained from said patient has been determined to have an average HER2 gene copy number of greater than or equal to 10 per tumour cell.

3. A specific binding member for use according to claim 1 or 2, wherein the cancer has, or has been determined to have, a high HER2 mRNA level, wherein a high HER2 mRNA level corresponds to a HER2 cDNA copy number of greater than or equal to 200 in a tumour sample obtained from said patient relative to the cDNA copy number of a reference gene in said sample, wherein the cDNA copy number of HER2 and the reference gene in the sample is determined by RT-PCR followed by quantitative PCR or, wherein the cancer has, or has been determined to have, a high HER2 mRNA level, wherein a high HER2 mRNA level corresponds to a HER2 cDNA copy number of greater than or equal to 200 and less than or equal to 820 in a tumour sample obtained from said patient relative to the cDNA copy number of a reference gene in said sample, wherein the cDNA copy number of HER2 and the reference gene in the sample is determined by RT-PCR followed by quantitative PCR.

4. A specific binding member for use according to claim 1, wherein the method comprises:

   (i) determining the average gene copy number of the HER2 gene per tumour cell in a tumour sample obtained from the patient; and
   (ii) treating said patient with said specific binding member if the average HER2 gene copy number is greater than or equal to 10 per tumour cell.

5. A method of identifying a cancer in a patient which is susceptible to treatment with:

   (a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
   (b) a specific binding member which competes with a specific binding member according to (a) for binding to HER2; the method comprising:

      (i) determining the average gene copy number of the HER2 gene per tumour cell in a tumour sample obtained from the patient, wherein an average gene copy number of greater than or equal to 10 per tumour cell indicates that the cancer is susceptible to treatment with the specific binding member, optionally, wherein the method further comprises:
      (ii) selecting said patient for treatment with the specific binding member if the average HER2 gene copy number is greater than or equal to 10 per tumour cell.

6. A method of predicting the response of a cancer to treatment with:

   (a) a specific binding member comprising a HER2 antigen binding site engineered into a structural loop region of a CH3 domain of the specific binding member and containing the amino acid sequences FFTYW (SEQ ID NO: 12), and DRRRWTA (SEQ ID NO: 14); or
   (b) a specific binding member which competes with a specific binding member according to (a) for binding to HER2; the method comprising:

      (i) determining the average gene copy number of the HER2 gene per tumour cell in a tumour sample obtained from a patient, wherein a gene copy number of greater than or equal to 10 per tumour cell indicates that the cancer is susceptible to treatment with the specific binding member, and wherein a gene copy number of less than 10 per tumour cell indicates that the cancer is not susceptible to treatment with the specific binding member, optionally, wherein the method further comprises:
(ii) selecting said cancer for treatment with the specific binding member if the HER2 gene copy number is greater than or equal to 10 per tumour cell.

**7.** A specific binding member for use or a method according to any one of claims 1 to 6, wherein the HER2 gene copy number is greater than or equal to 11, greater than or equal to 12, greater than or equal to 13, greater than or equal to 14, greater than or equal to 15, greater than or equal to 16, greater than or equal to 17, or greater than or equal to 18.

**8.** A specific binding member for use or a method according to any one of claims 1 to 7, wherein the cancer is gastric cancer, breast cancer, colorectal cancer, ovarian cancer, pancreatic cancer, lung cancer, stomach cancer, or endometrial cancer.

**9.** A specific binding member for use or a method according to any one of claims 1 to 8, wherein the patient has exhibited an inadequate response to trastuzumab and/or trastuzumab plus pertuzumab and/or, wherein the cancer is refractory to treatment with trastuzumab and/or trastuzumab plus pertuzumab.

**10.** A specific binding member for use or a method according to any one of claims 1 to 9, wherein the specific binding member of claim 1, part (b), claim 5, part (b), or claim 6, part (b), comprises a HER2 antigen binding site engineered into a structural loop region of a CH3 domain of the specific binding member.

**11.** A specific binding member for use or a method according to claim 10, wherein the specific binding member comprises a HER2 antigen binding site engineered into structural loop regions AB and EF of a CH3 domain of the specific binding member.

**12.** A specific binding member for use or a method according to any one of claims 1 to 11, wherein the specific binding member of claim 1, part (a) and/or (b), claim 5, part (a) and/or (b), or claim 6, part (a) and/or (b), is, or comprises, an antigen-binding Fc fragment.

**13.** A specific binding member for use or a method according to any one of claims 1 to 12, wherein the specific binding member of claim 1, part (a) and/or (b), claim 5, part (a) and/or (b), or claim 6, part (a) and/or (b), comprises the CH3 domain of SEQ ID NO: 11, or the CH3 domain of SEQ ID NO: 11 minus one or two C-terminal amino acids.

**14.** A specific binding member for use or a method according to claim 13, wherein the specific binding member of claim 1, part (a) and/or (b), claim 5, part (a) and/or (b), or claim 6, part (a) and/or (b), further comprises the CH2 domain of SEQ ID NO: 10.

**15.** A specific binding member for use or a method according to any one of claims 1 to 14, wherein the specific binding member of claim 1, part (a), claim 5, part (a), or claim 6, part (a), is a dimer of a polypeptide of SEQ ID NO: 8, or a dimer of a polypeptide of SEQ ID NO: 8 minus one or two C-terminal amino acids.

**16.** A specific binding member for use or a method according to any one of claims 1 to 15, wherein the specific binding member of claim 1, part (b), claim 5, part (b), or claim 6, part (b), competes with a specific binding member according to claim 1, part (a), claim 5, part (a), or claim 6, part (a), respectively, for binding to HER2 as determined by surface plasmon resonance (SPR), or a competitive enzyme-linked immunosorbent assay (ELISA)), fluorescence activated cell sorting (FACS), or competition immunocytochemistry.

**17.** A specific binding member for use or a method according any one of claims 1 to 16, wherein the specific binding member of claim 1, part (b), claim 5, part (b), or claim 6, part (b), binds to same epitope on HER2 as a specific binding member according to claim 1, part (a), claim 5, part (a), or claim 6, part (a), respectively, and wherein the specific binding member according to claim 1, part (a), claim 5, part (a), or claim 6, part (a), is a dimer of a polypeptide of SEQ ID NO: 8, or a dimer of a polypeptide of SEQ ID NO: 8 minus one or two C-terminal amino acids.

**18.** A specific binding member for use or a method according to any one of claims 1 to 17, wherein the method comprises determining the HER2 mRNA level in a tumour sample obtained from the patient, wherein a high HER2 mRNA level corresponds to a HER2 cDNA copy number of greater than or equal to 200 in the tumour sample relative to the cDNA copy number of a reference gene in said sample, and wherein a high HER2 mRNA level indicates that the average HER2 gene copy number per tumour cell is greater than or equal to 10 and optionally, wherein the cDNA copy number of HER2 and the reference gene is determined using reverse transcription PCR followed by quantitative PCR.

## Patentansprüche

1. Spezifisches Bindungselement, das an Human-Epidermis-Wachstumsfaktor-Rezeptor 2 (HER2) bindet, zur Verwendung in einem Verfahren zur Behandlung von Krebs bei einem Patienten, wobei der Krebs eine mittlere HER2-Genkopiezahl von größer oder gleich 10 pro Tumorzelle aufweist und wobei das spezifische Bindungselement

   (a) ein spezifisches Bindungselement ist, das eine HER2-Antigen-Bindungsstelle umfasst, welche zu einer strukturellen Schleifenregion einer CH3-Domäne des spezifischen Bindungselements verändert wurde, und die Aminosäuresequenzen FFTYW (Seq.-ID Nr. 12) und DRRRWTA (Seq.-ID Nr. 14) enthält, oder
   (b) ein spezifisches Bindungselement ist, das mit einem spezifischen Bindungselement gemäß (a) um die Bindung an HER2 konkurriert.

2. Spezifisches Bindungselement zur Verwendung nach Anspruch 1, wobei bestimmt wurde, dass eine vom Patienten erhaltene Tumorprobe eine mittlere HER2-Genkopiezahl von größer oder gleich 10 pro Tumorzelle aufweist.

3. Spezifisches Bindungselement zur Verwendung nach Anspruch 1 oder 2, wobei der Krebs einen hohen HER2-mRNA-Wert aufweist oder bestimmt wurde, dass er einen hohen HER2-mRNA-Wert aufweist, wobei ein hoher HER2-mRNA-Wert einer HER2-cDNA-Kopiezahl von größer oder gleich 200 in einer von dem Patienten erhaltenen Tumorprobe in Bezug auf die cDNA-Kopiezahl eines Bezugsgens in der Probe entspricht, wobei die cDNA-Kopiezahl von HER2 und des Referenzgens in der Probe durch RT-PCR und anschließende quantitative PCR bestimmt wird, oder
   wobei der Krebs einen hohen HER2-mRNA-Wert aufweist oder bestimmt wurde, dass er einen hohen HER2-mRNA-Wert aufweist, wobei ein hoher HER2-mRNA-Wert einer HER2-cDNA-Kopiezahl von größer oder gleich 200 und kleiner oder gleich 820 in einer von dem Patienten erhaltenen Tumorprobe in Bezug auf die cDNA-Kopiezahl eines Bezugsgens in der Probe entspricht, wobei die cDNA-Kopiezahl von HER2 und des Referenzgens in der Probe durch RT-PCR und anschließende quantitative PCR bestimmt wird.

4. Spezifisches Bindungselement zur Verwendung nach Anspruch 1, wobei das Verfahren Folgendes umfasst:

   (i) Bestimmen der mittleren Genkopiezahl des HER2-Gens pro Tumorzelle in einer vom Patienten erhaltenen Tumorprobe; und
   (ii) Behandeln des Patienten mit dem spezifischen Bindungselement, wenn die mittlere HER2-Genkopiezahl größer oder gleich 10 pro Tumorzelle ist.

5. Verfahren zum Identifizieren von Krebs bei einem Patienten, der mit Folgendem behandelt werden kann:

   (a) einem spezifischen Bindungselement, das eine HER2-Antigen-Bindungsstelle umfasst, welche zu einer strukturellen Schleifenregion einer CH3-Domäne des spezifischen Bindungselements verändert wurde, und die Aminosäuresequenzen FFTYW (Seq.-ID Nr. 12) und DRRRWTA (Seq.-ID Nr. 14) enthält, oder
   (b) einem spezifischen Bindungselement, das mit einem spezifischen Bindungselement gemäß (a) um die Bindung an HER2 konkurriert; wobei das Verfahren Folgendes umfasst:

      (i) Bestimmen der mittleren Genkopiezahl des HER2-Gens pro Tumorzelle in einer vom Patienten erhaltenen Tumorprobe, wobei eine mittlere Genkopiezahl von größer oder gleich 10 pro Tumorzelle anzeigt, dass der Krebs mit dem spezifischen Bindungselement behandelt werden kann, und wobei das Verfahren gegebenenfalls ferner Folgendes umfasst:
      (ii) Behandeln des Patienten mit dem spezifischen Bindungselement, wenn die mittlere HER2-Genkopiezahl größer oder gleich 10 pro Tumorzelle ist.

6. Verfahren zum Vorhersagen der Antwort von Krebs auf eine Behandlung mit

   (a) einem spezifischen Bindungselement, das eine HER2-Antigen-Bindungsstelle umfasst, welche zu einer strukturellen Schleifenregion einer CH3-Domäne des spezifischen Bindungselements verändert wurde, und die Aminosäuresequenzen FFTYW (Seq.-ID Nr. 12) und DRRRWTA (Seq.-ID Nr. 14) enthält, oder

(b) einem spezifischen Bindungselement, das mit einem spezifischen Bindungselement gemäß (a) um die Bindung an HER2 konkurriert; wobei das Verfahren Folgendes umfasst:

(i) Bestimmen der mittleren Genkopiezahl des HER2-Gens pro Tumorzelle in einer vom Patienten erhaltenen Tumorprobe, wobei eine Genkopiezahl von größer oder gleich 10 pro Tumorzelle anzeigt, dass der Krebs mit dem spezifischen Bindungselement behandelt werden kann, und wobei eine Genkopiezahl von kleiner oder gleich 10 pro Tumorzelle anzeigt, dass der Krebs mit dem spezifischen Bindungselement nicht behandelt werden kann, wobei das Verfahren gegebenenfalls ferner Folgendes umfasst:
(ii) Auswählen des Krebses für die Behandlung mit dem spezifischen Bindungselement, wenn die HER2-Genkopiezahl größer oder gleich 10 pro Tumorzelle ist.

**7.** Spezifisches Bindungselement zur Verwendung oder Verfahren nach einem der Ansprüche 1 bis 6, wobei die HER2-Genkopiezahl größer oder gleich 11, größer oder gleich 12, größer oder gleich 13, größer oder gleich 14, größer oder gleich 15, größer oder gleich 16, größer oder gleich 17 oder größer oder gleich 18 ist.

**8.** Spezifisches Bindungselement zur Verwendung oder Verfahren nach einem der Ansprüche 1 bis 7, wobei der Krebs Magenkrebs, Brustkrebs, Kolorektalkrebs, Eierstockkrebs, Pankreaskrebs, Lungenkrebs, Magenkrebs oder Endometriumkrebs ist.

**9.** Spezifisches Bindungselement zur Verwendung oder Verfahren nach einem der Ansprüche 1 bis 8, wobei der Patient eine unangemessene Antwort auf Trastuzumab und/oder Trastuzumab plus Pertuzumab aufgewiesen hat und/oder wobei der Krebs nicht auf eine Behandlung mit Trastuzumab und/oder Trastuzumab plus Pertuzumab anspricht.

**10.** Spezifisches Bindungselement zur Verwendung oder Verfahren nach einem der Ansprüche 1 bis 9, wobei ein spezifisches Bindungselement nach Anspruch 1, Teil (b), Anspruch 5, (Teil b), oder Anspruch 6, Teil (b), eine HER2-Antigen-Bindungsstelle umfasst, welche zu einer strukturellen Schleifenregion einer CH3-Domäne des spezifischen Bindungselements verändert wurde.

**11.** Spezifisches Bindungselement zur Verwendung oder Verfahren nach Anspruch 10, wobei das spezifische Bindungselement eine HER2-Antigen-Bindungsstelle umfasst, welche zu den strukturellen Schleifenregionen AB und EF einer CH3-Domäne des spezifischen Bindungselements verändert wurde.

**12.** Spezifisches Bindungselement zur Verwendung oder Verfahren nach einem der Ansprüche 1 bis 11, wobei ein spezifisches Bindungselement nach Anspruch 1, Teil (a) und/oder (b), Anspruch 5, Teil (a) und/oder (b), oder Anspruch 6, Teil (a) und/oder (b), ein Antigen-bindendes Fc-Fragment ist oder ein solches umfasst.

**13.** Spezifisches Bindungselement zur Verwendung oder Verfahren nach einem der Ansprüche 1 bis 12, wobei ein spezifisches Bindungselement nach Anspruch 1, Teil (a) und/oder (b), Anspruch 5, Teil (a) und/oder (b), oder Anspruch 6, Teil (a) und/oder (b), die CH3-Domäne von Seq.-ID Nr. 11 oder die CH3-Domäne von Seq.-ID Nr. 11 minus eine oder zwei C-terminale Aminosäuren umfasst.

**14.** Spezifisches Bindungselement zur Verwendung oder Verfahren nach Anspruch 13, wobei ein spezifisches Bindungselement nach Anspruch 1, Teil (a) und/oder (b), Anspruch 5, Teil (a) und/oder (b), oder Anspruch 6, Teil (a) und/oder (b), weiters die CH2-Domäne von Seq.-ID Nr. 10 umfasst.

**15.** Spezifisches Bindungselement zur Verwendung oder Verfahren nach einem der Ansprüche 1 bis 14, wobei ein spezifisches Bindungselement nach Anspruch 1, Teil (a), Anspruch 5, Teil (a), oder Anspruch 6, Teil (a), ein Dimer eines Polypeptids von Seq.-ID Nr. 8 oder ein Dimer eines Polypeptids nach Seq.-ID Nr. 8 minus eine oder zwei C-terminale Aminosäuren ist.

**16.** Spezifisches Bindungselement zur Verwendung oder Verfahren nach einem der Ansprüche 1 bis 15, wobei jeweils ein spezifisches Bindungselement nach Anspruch 1, Teil (b), Anspruch 5, Teil (b), oder Anspruch 6, Teil (b), mit einem spezifischen Bindungselement nach Anspruch 1, Teil (a), Anspruch 5, Teil (a), oder Anspruch 6, Teil (a), um die Bindung an HER2 konkurriert, wie durch Oberflächenplasmonresonanz (SPR) oder kompetitiven Enzym-gebundenen Immunosorptionstest (ELISA), fluoreszenzaktiviertes Zellsortieren (FACS) oder Konkurrenz-Immunzytochemie bestimmt.

**17.** Spezifisches Bindungselement zur Verwendung oder Verfahren nach einem der Ansprüche 1 bis 16, wobei jeweils ein spezifisches Bindungselement nach Anspruch 1, Teil (b), Anspruch 5, Teil (b), oder Anspruch 6, Teil (b), an dasselbe Epitop auf HER2 wie ein spezifisches Bindungselement nach Anspruch 1, Teil (a), Anspruch 5, Teil (a), oder Anspruch 6, Teil (a), bindet und wobei ein spezifisches Bindungselement nach Anspruch 1, Teil (a), Anspruch 5, Teil (a), oder Anspruch 6, Teil (a), ein Dimer eines Polypeptids von Seq.-ID Nr. 8 oder ein Dimer eines Polypeptids nach Seq.-ID Nr. 8 minus eine oder zwei C-terminale Aminosäuren ist.

**18.** Spezifisches Bindungselement zur Verwendung oder Verfahren nach einem der Ansprüche 1 bis 17, wobei das Verfahren das Bestimmen des HER2-mRNA-Werts in einer vom Patienten erhaltenen Tumorprobe umfasst, wobei ein hoher HER2-mRNA-Wert einer HER2-cDNA-Kopiezahl von größer oder gleich 200 in der Tumorprobe in Bezug auf die cDNA-Kopiezahl eines Bezugsgens in der Probe entspricht und wobei ein hoher HER2-mRNA-Wert anzeigt, dass die mittlere HER2-Genkopiezahl pro Tumorzelle größer oder gleich 10 ist, und wobei gegebenenfalls die cDNA-Kopiezahl von HER2 und des Referenzgens durch Reverse-Transkriptions-PCR und anschließende quantitative PCR bestimmt wird.

**Revendications**

**1.** Élément de liaison spécifique qui se lie au récepteur du facteur de croissance épidermique humain de type 2 (HER2) destiné à être utilisé dans un procédé de traitement du cancer chez un patient, où ledit cancer présente un nombre de copies moyen du gène HER2 supérieur ou égal à 10 par cellule tumorale, et où l'élément de liaison spécifique est :

(a) un élément de liaison spécifique comprenant un site de liaison à un antigène HER2 manipulé dans une région de boucle structurale d'un domaine CH3 de l'élément de liaison spécifique et contenant les séquences d'acides aminés FFTYW (SEQ ID NO: 12), et DRRRWTA (SEQ ID NO: 14) ; ou
(b) un élément de liaison spécifique qui entre en compétition avec un élément de liaison spécifique selon (a) pour une liaison à HER2.

**2.** Élément de liaison spécifique destiné à être utilisé selon la revendication 1, où il a été déterminé qu'un échantillon tumoral obtenu à partir dudit patient présente un nombre de copies moyen du gène HER2 supérieur ou égal à 10 par cellule tumorale.

**3.** Élément de liaison spécifique destiné à être utilisé selon la revendication 1 ou 2, dans lequel le cancer présente, ou il a été déterminé qu'il présente, un taux d'ARNm de HER2 élevé, où un taux d'ARNm de HER2 élevé correspond à un nombre de copies d'ADNc de HER2 supérieur ou égal à 200 dans un échantillon tumoral obtenu à partir dudit patient par rapport au nombre de copies d'ADNc d'un gène de référence dans ledit échantillon, où le nombre de copies d'ADNc de HER2 et du gène de référence dans l'échantillon est déterminé par une RT-PCR suivie d'une PCR quantitative ou, dans lequel le cancer présente, ou il a été déterminé qu'il présente, un taux d'ARNm de HER2 élevé, où un taux d'ARNm de HER2 élevé correspond à un nombre de copies d'ADNc de HER2 supérieur ou égal à 200 et inférieur ou égal à 820 dans un échantillon tumoral obtenu à partir dudit patient par rapport au nombre de copies d'ADNc d'un gène de référence dans ledit échantillon, où le nombre de copies d'ADNc de HER2 et du gène de référence dans l'échantillon est déterminé par une RT-PCR suivie d'une PCR quantitative.

**4.** Élément de liaison spécifique destiné à être utilisé selon la revendication 1, où le procédé comprend :

(i) la détermination du nombre de copies géniques moyen du gène HER2 par cellule tumorale dans un échantillon tumoral obtenu à partir du patient ; et
(ii) le traitement dudit patient avec ledit élément de liaison spécifique si le nombre de copies moyen du gène HER2 est supérieur ou égal à 10 par cellule tumorale.

**5.** Procédé d'identification d'un cancer chez un patient qui est sensible à un traitement avec :

(a) un élément de liaison spécifique comprenant un site de liaison à un antigène HER2 manipulé dans une région de boucle structurale d'un domaine CH3 de l'élément de liaison spécifique et contenant les séquences d'acides aminés FFTYW (SEQ ID NO: 12), et DRRRWTA (SEQ ID NO: 14) ; ou
(b) un élément de liaison spécifique qui entre en compétition avec un élément de liaison spécifique selon (a) pour une liaison à HER2 ; le procédé comprenant :

(i) la détermination du nombre de copies géniques moyen du gène HER2 par cellule tumorale dans un échantillon tumoral obtenu à partir du patient, où un nombre de copies géniques moyen supérieur ou égal à 10 par cellule tumorale indique que le cancer est sensible à un traitement avec l'élément de liaison spécifique, facultativement, où le procédé comprend en outre :
(ii) la sélection dudit patient pour un traitement avec l'élément de liaison spécifique si le nombre de copies moyen du gène HER2 est supérieur ou égal à 10 par cellule tumorale.

**6.** Procédé de prédiction de la réponse d'un cancer à un traitement avec :

(a) un élément de liaison spécifique comprenant un site de liaison à un antigène HER2 manipulé dans une région de boucle structurale d'un domaine CH3 de l'élément de liaison spécifique et contenant les séquences d'acides aminés FFTYW (SEQ ID NO: 12), et DRRRWTA (SEQ ID NO: 14) ; ou
(b) un élément de liaison spécifique qui entre en compétition avec un élément de liaison spécifique selon (a) pour une liaison à HER2 ; le procédé comprenant :

(i) la détermination du nombre de copies géniques moyen du gène HER2 par cellule tumorale dans un échantillon tumoral obtenu à partir d'un patient, où un nombre de copies géniques supérieur ou égal à 10 par cellule tumorale indique que le cancer est sensible à un traitement avec l'élément de liaison spécifique, et où un nombre de copies géniques inférieur à 10 par cellule tumorale indique que le cancer n'est pas sensible à un traitement avec l'élément de liaison spécifique, facultativement, où le procédé comprend en outre :
(ii) la sélection dudit cancer pour un traitement avec l'élément de liaison spécifique si le nombre de copies du gène HER2 est supérieur ou égal à 10 par cellule tumorale.

**7.** Élément de liaison spécifique pour une utilisation ou procédé selon l'une quelconque des revendications 1 à 6, où le nombre de copies du gène HER2 est supérieur ou égal à 11, supérieur ou égal à 12, supérieur ou égal à 13, supérieur ou égal à 14, supérieur ou égal à 15, supérieur ou égal à 16, supérieur ou égal à 17, ou supérieur ou égal à 18.

**8.** Élément de liaison spécifique pour une utilisation ou procédé selon l'une quelconque des revendications 1 à 7, où le cancer est le cancer gastrique, le cancer du sein, le cancer colorectal, le cancer de l'ovaire, le cancer du pancréas, le cancer du poumon, le cancer de l'estomac, ou le cancer de l'endomètre.

**9.** Élément de liaison spécifique pour une utilisation ou procédé selon l'une quelconque des revendications 1 à 8, dans lequel le patient a exhibé une réponse inadéquate au trastuzumab et/ou au trastuzumab plus pertuzumab et/ou, dans lequel le cancer est réfractaire à un traitement par le trastuzumab et/ou le trastuzumab plus pertuzumab.

**10.** Élément de liaison spécifique pour une utilisation ou procédé selon l'une quelconque des revendications 1 à 9, où l'élément de liaison spécifique selon la revendication 1, partie (b), la revendication 5, partie (b), ou la revendication 6, partie (b), comprend un site de liaison à un antigène HER2 manipulé dans une région de boucle structurale d'un domaine CH3 de l'élément de liaison spécifique.

**11.** Élément de liaison spécifique pour une utilisation ou procédé selon la revendication 10, où l'élément de liaison spécifique comprend un site de liaison à un antigène HER2 manipulé dans des régions de boucle structurale AB et EF d'un domaine CH3 de l'élément de liaison spécifique.

**12.** Élément de liaison spécifique pour une utilisation ou procédé selon l'une quelconque des revendications 1 à 11, où l'élément de liaison spécifique selon la revendication 1, partie (a) et/ou (b), la revendication 5, partie (a) et/ou (b), ou la revendication 6, partie (a) et/ou (b), est, ou comprend, un fragment Fc se liant à un antigène.

**13.** Élément de liaison spécifique pour une utilisation ou procédé selon l'une quelconque des revendications 1 à 12, où l'élément de liaison spécifique selon la revendication 1, partie (a) et/ou (b), la revendication 5, partie (a) et/ou (b), ou la revendication 6, partie (a) et/ou (b), comprend le domaine CH3 de SEQ ID NO: 11, ou le domaine CH3 de SEQ ID NO: 11 moins un ou deux acides aminés C-terminaux.

**14.** Élément de liaison spécifique pour une utilisation ou procédé selon la revendication 13, où l'élément de liaison spécifique selon la revendication 1, partie (a) et/ou (b), la revendication 5, partie (a) et/ou (b), ou la revendication 6, partie (a) et/ou (b), comprend en outre le domaine CH2 de SEQ ID NO: 10.

**15.** Élément de liaison spécifique pour une utilisation ou procédé selon l'une quelconque des revendications 1 à 14, où l'élément de liaison spécifique selon la revendication 1, partie (a), la revendication 5, partie (a), ou la revendication 6, partie (a), est un dimère d'un polypeptide de SEQ ID NO: 8, ou un dimère d'un polypeptide de SEQ ID NO: 8 moins un ou deux acides aminés C-terminaux.

**16.** Élément de liaison spécifique pour une utilisation ou procédé selon l'une quelconque des revendications 1 à 15, où l'élément de liaison spécifique selon la revendication 1, partie (b), la revendication 5, partie (b), ou la revendication 6, partie (b), entre en compétition avec un élément de liaison spécifique selon la revendication 1, partie (a), la revendication 5, partie (a), ou la revendication 6, partie (a), respectivement, pour une liaison à HER2 tel que déterminé par résonance plasmonique de surface (SPR), ou un essai d'immunoabsorption enzymatique (ELISA) compétitif, un tri cellulaire activé par fluorescence (FACS), ou une immunocytochimie par compétition.

**17.** Élément de liaison spécifique pour une utilisation ou procédé selon l'une quelconque des revendications 1 à 16, où l'élément de liaison spécifique selon la revendication 1, partie (b), la revendication 5, partie (b), ou la revendication 6, partie (b), se lie au même épitope sur HER2 que l'élément de liaison spécifique selon la revendication 1, partie (a), la revendication 5, partie (a), ou la revendication 6, partie (a), respectivement, et où l'élément de liaison spécifique selon la revendication 1, partie (a), la revendication 5, partie (a), ou la revendication 6, partie (a), est un dimère d'un polypeptide de SEQ ID NO: 8, ou un dimère d'un polypeptide de SEQ ID NO: 8 moins un ou deux acides aminés C-terminaux.

**18.** Élément de liaison spécifique pour une utilisation ou procédé selon l'une quelconque des revendications 1 à 17, où le procédé comprend la détermination du taux d'ARNm de HER2 dans un échantillon tumoral obtenu à partir du patient, où un taux d'ARNm de HER2 élevé correspond à un nombre de copies d'ADNc de HER2 supérieur ou égal à 200 dans l'échantillon tumoral par rapport au nombre de copies d'ADNc d'un gène de référence dans ledit échantillon, et où un taux d'ARNm de HER2 élevé indique que le nombre de copies moyen du gène HER2 par cellule tumorale est supérieur ou égal à 10 et facultativement, où le nombre de copies d'ADNc de HER2 et du gène de référence est déterminé en utilisant une PCR par transcription inverse suivie d'une PCR quantitative.

Figure 1

240 250 260 270 280 290

H561-4 TCPPCPAPELLGGPSVFLFPPKPKDTLMISRTPEVTCVVVDVSHEDPEVKFNW

WT TCPPCPAPELLGGPSVFLFPPKPKDTLMISRTPEVTCVVVDVSHEDPEVKFNW

300 310 320 330 340

H561-4 YVDGVEVHNAKTKPREEQYNSTYRVVSVLTVLHQDWLNGKEYKCKVSNKALPA

WT YVDGVEVHNAKTKPREEQYNSTYRVVSVLTVLHQDWLNGKEYKCKVSNKALPA

350 360 370 381 390 400

H561-4 PIEKTISKAKGQPREPQVYTLPPSRDE**FFTYW**VSLTCLVKGFYPSDIAVEWES

WT PIEKTISKAKGQPREPQVYTLPPSRDE**LTKNQ**VSLTCLVKGFYPSDIAVEWES

410 420 430 440 450 460

H561-4 NGQPENNYKTTPPVLDSDGSFFLYSKLTV**DRRRWTA**GNVFSCSVMHEALHNHY

WT NGQPENNYKTTPPVLDSDGSFFLYSKLTV**DKSRWQQ**GNVFSCSVMHEALHNHY

470 478

H561-4 TQKSLSLSPGK

WT TQKSLSLSPGK

A

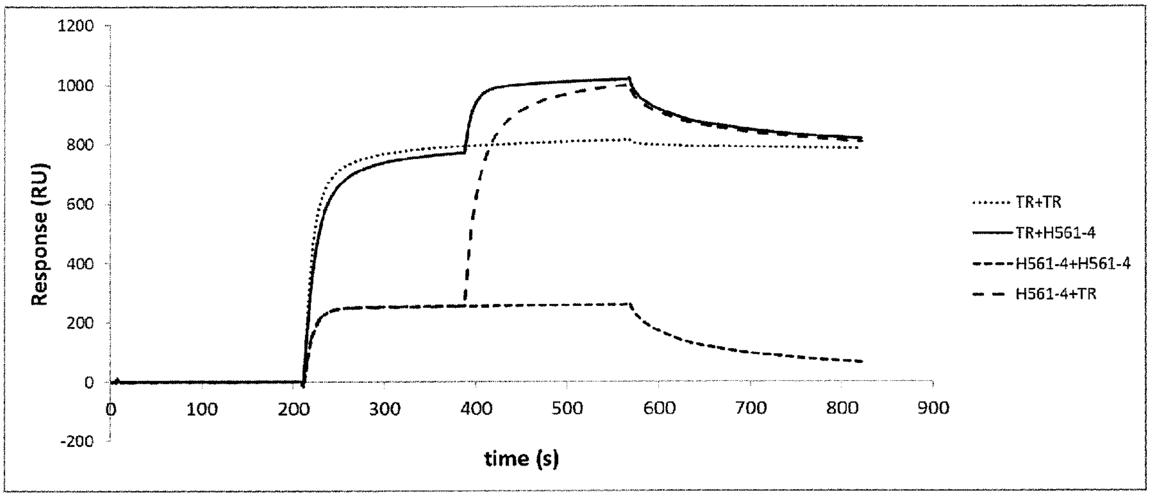

1200
1000
800
600
400
200
0
-200
Response (RU)
0
100
200
300
400
500
600
700
800
900
time (s)
TR+TR
TR+H561-4
H561-4+H561-4
H561-4+TR


B

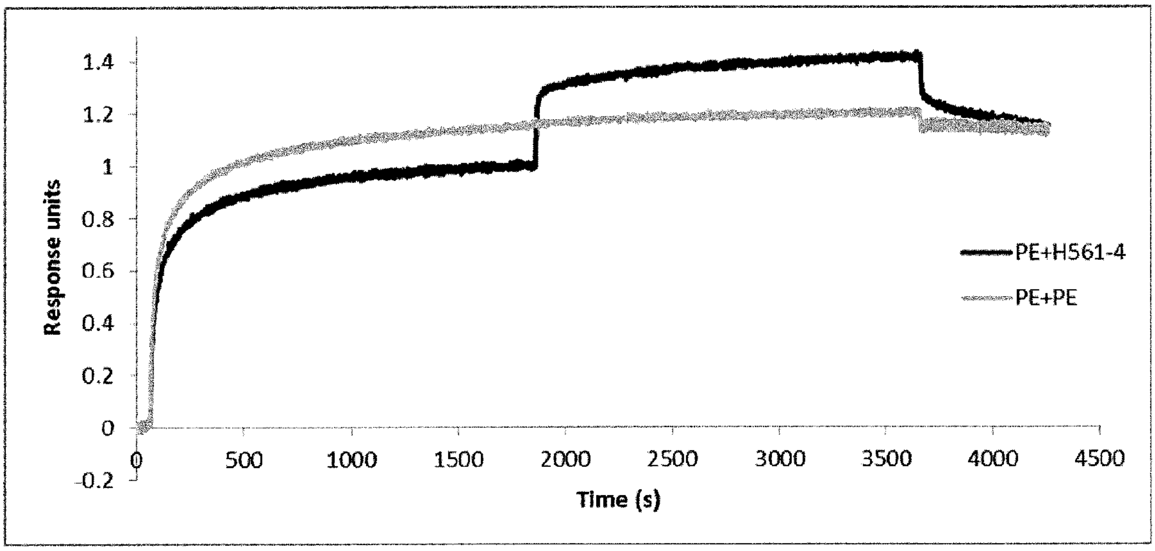

1.4
1.2
1
0.8
0.6
0.4
0.2
0
-0.2
Response units
0
500
1000
1500
2000
2500
3000
3500
4000
4500
Time (s)
PE+H561-4
PE+PE


Figure 2

C

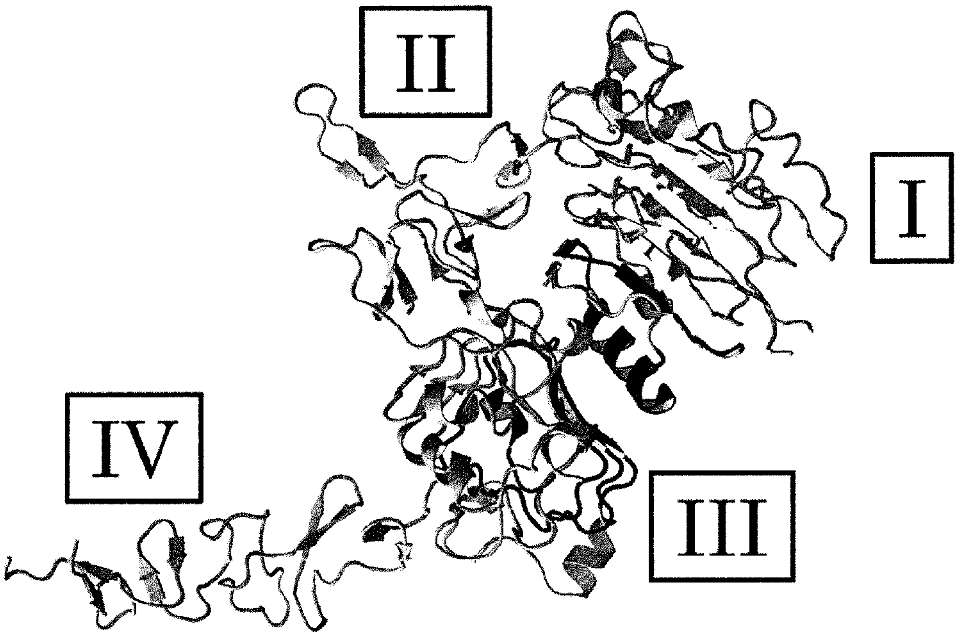


| Binding Area | Domain | Sequence |
|---|---|---|
| 1 | 1 | $_{13}$LPASPETHLDMLRHL$_{27}$ |
| 2 | 1 | $_{31}$CQVVQGNLELTYLPT$_{45}$ |
| 3 | 3 | $_{420}$SLTLQGLGISWLGLRSLRELGSGLALIHHNTHLCFVHTVPWDQLFRNPHQALLHTA$_{475}$ |

Figure 2 continued

A

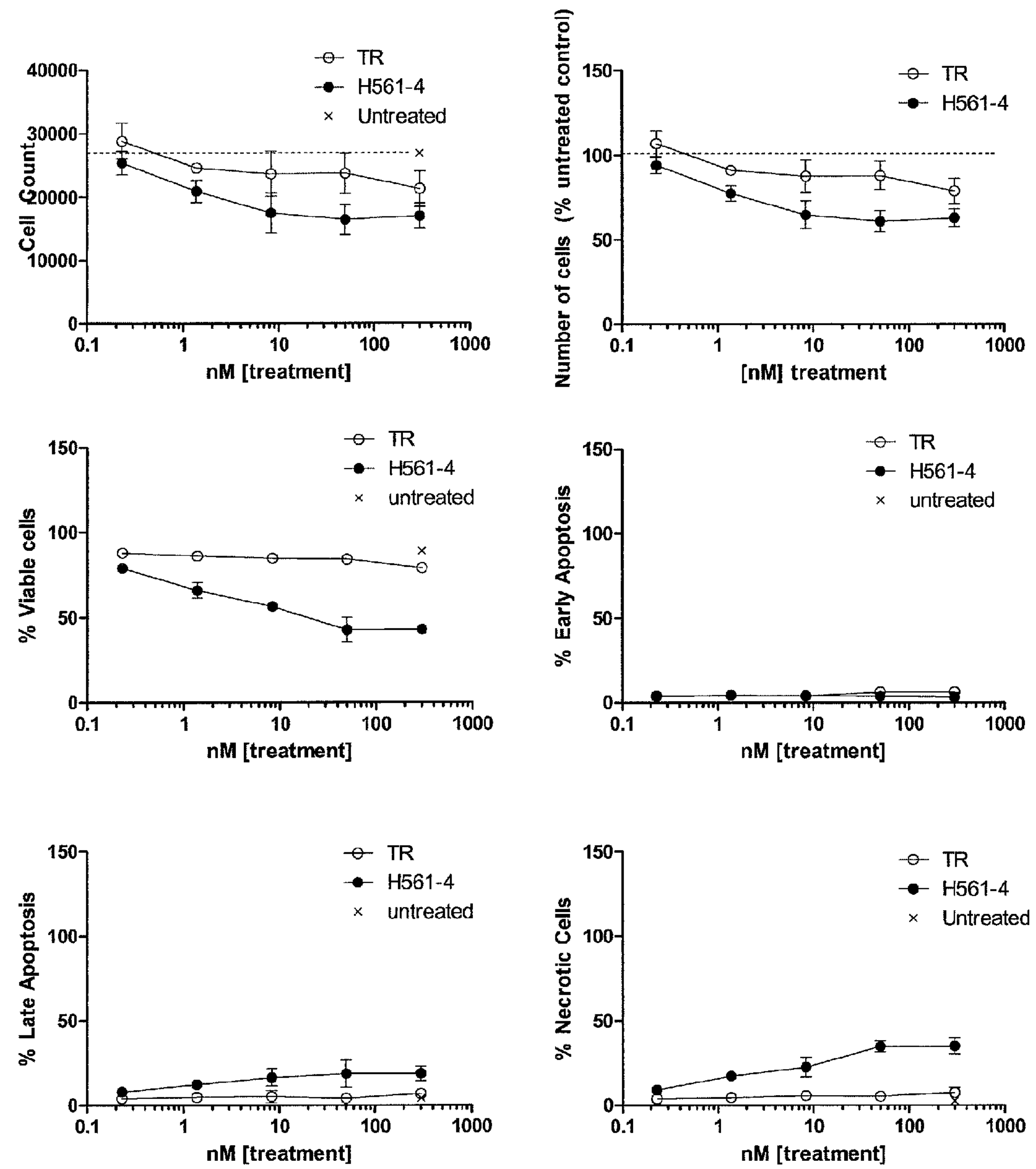
TR
H561-4
Untreated
Cell Count
nM [treatment]
Number of cells (% untreated control)
[nM] treatment
% Viable cells
% Early Apoptosis
% Late Apoptosis
% Necrotic Cells
untreated

Figure 3

B

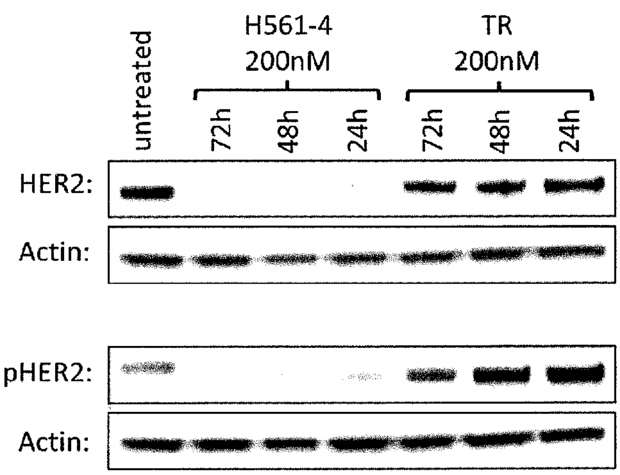


C

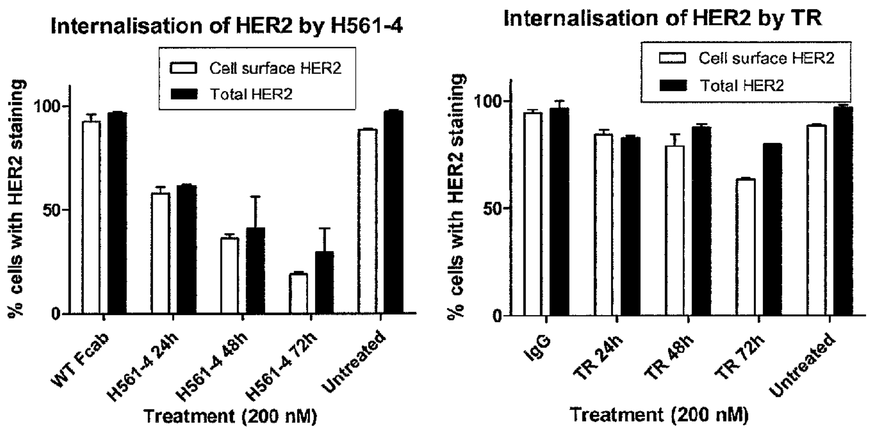


D

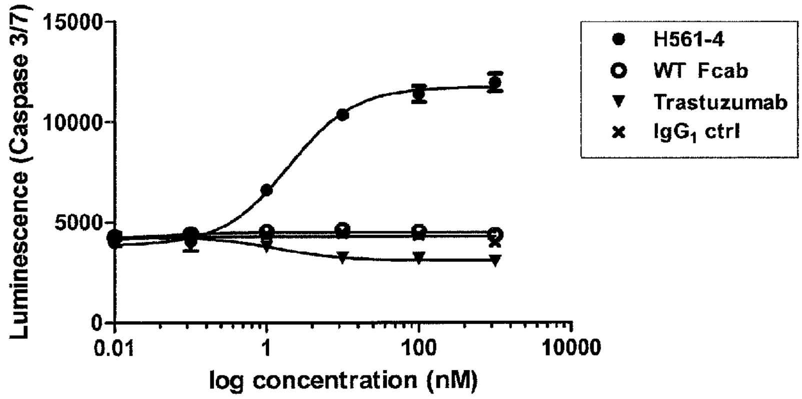


Figure 3 continued

A

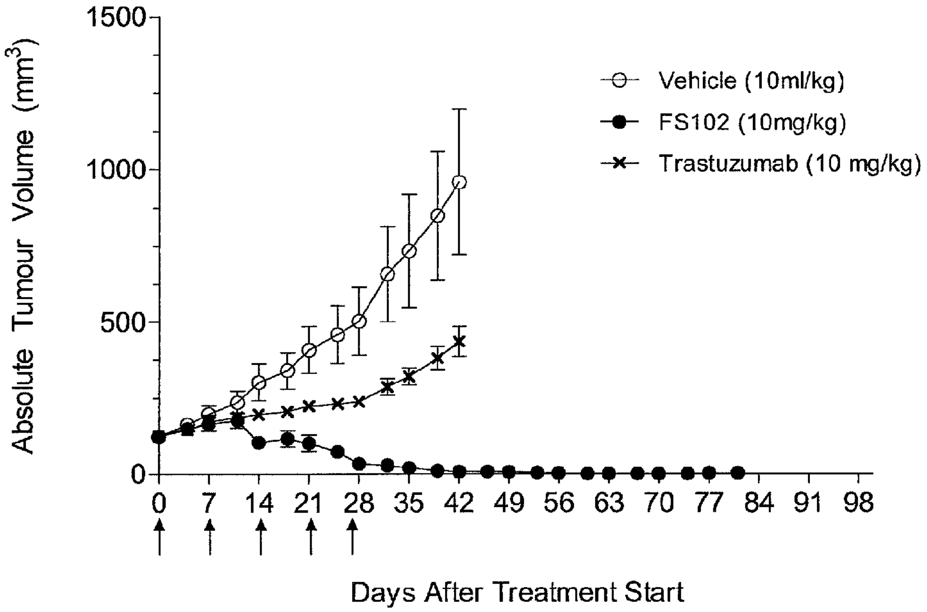
1500
1000
500
0
Absolute Tumour Volume (mm3)
Vehicle (10ml/kg)
FS102 (10mg/kg)
Trastuzumab (10 mg/kg)
0 7 14 21 28 35 42 49 56 63 70 77 84 91 98
Days After Treatment Start

B

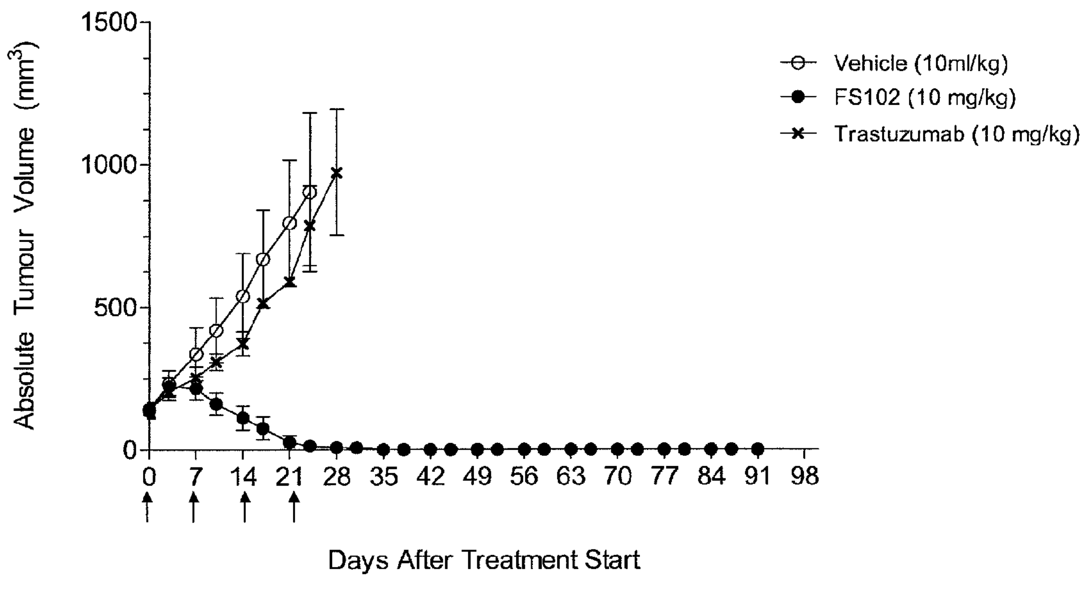
1500
1000
500
0
Absolute Tumour Volume (mm3)
Vehicle (10ml/kg)
FS102 (10 mg/kg)
Trastuzumab (10 mg/kg)
0 7 14 21 28 35 42 49 56 63 70 77 84 91 98
Days After Treatment Start

Figure 4

C

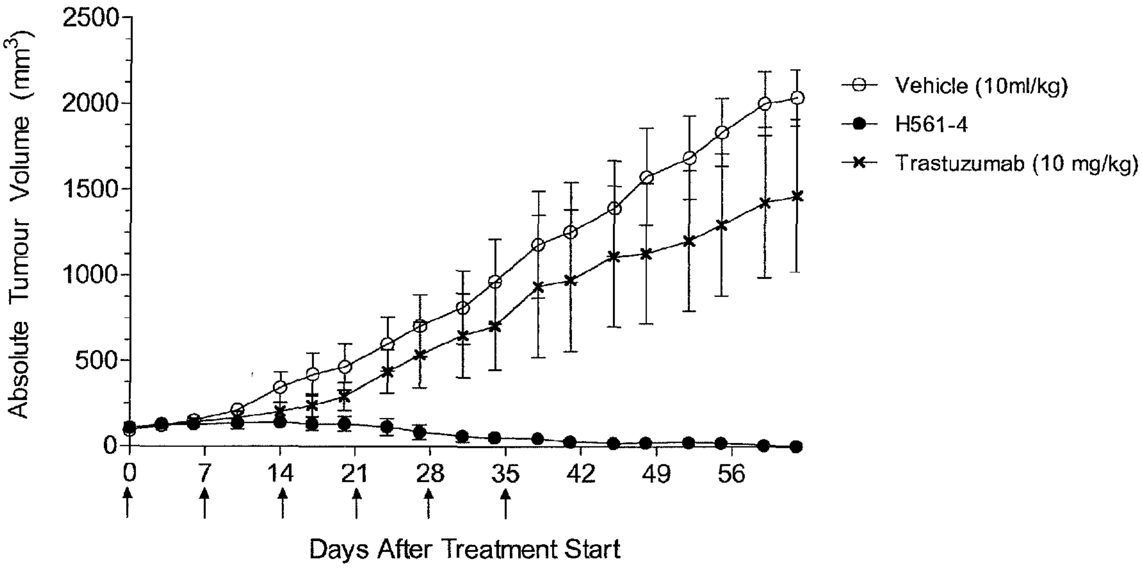


D

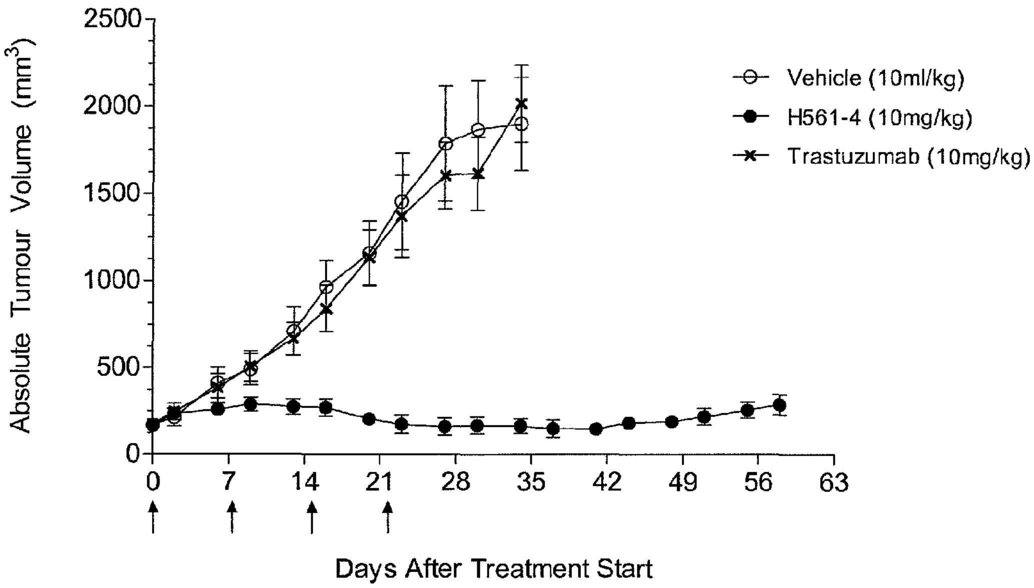


Figure 4 continued

E

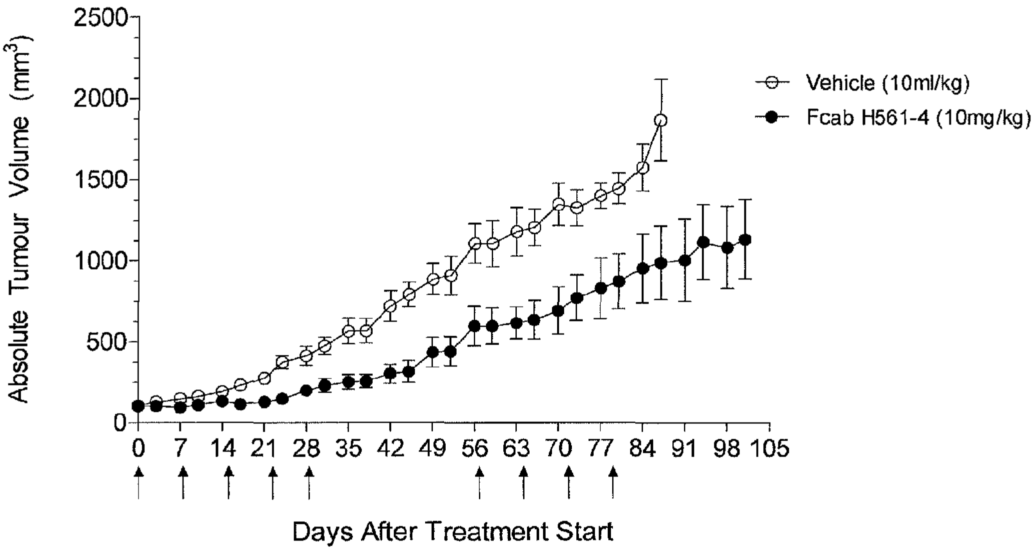


F

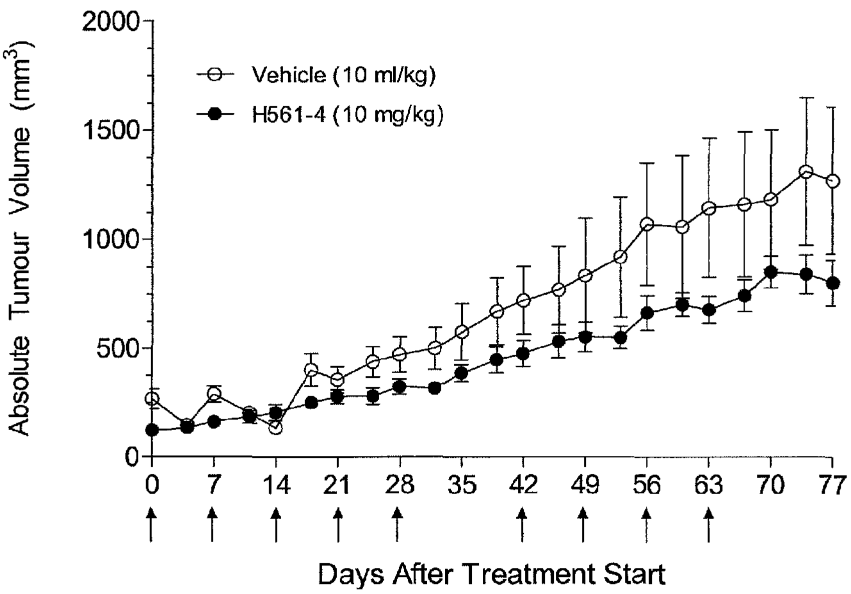


Figure 4 continued

G

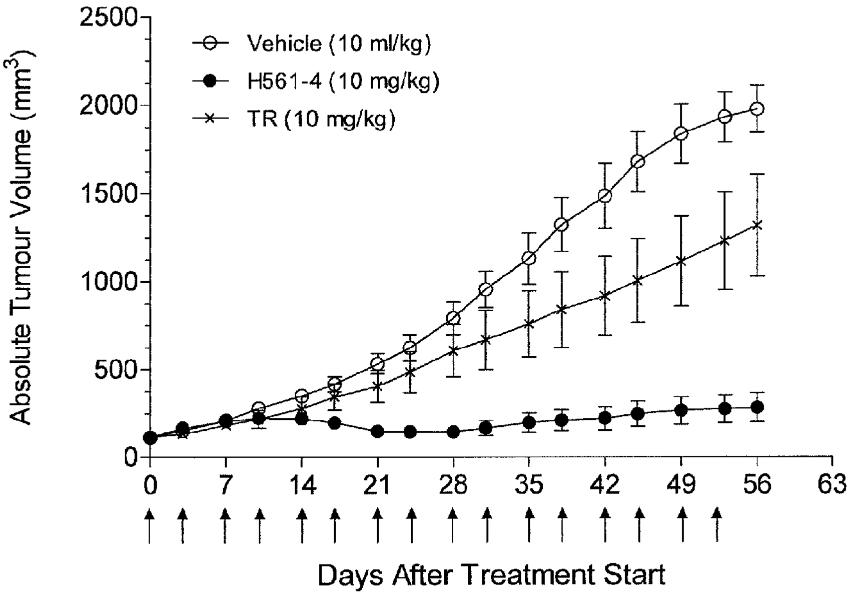


H

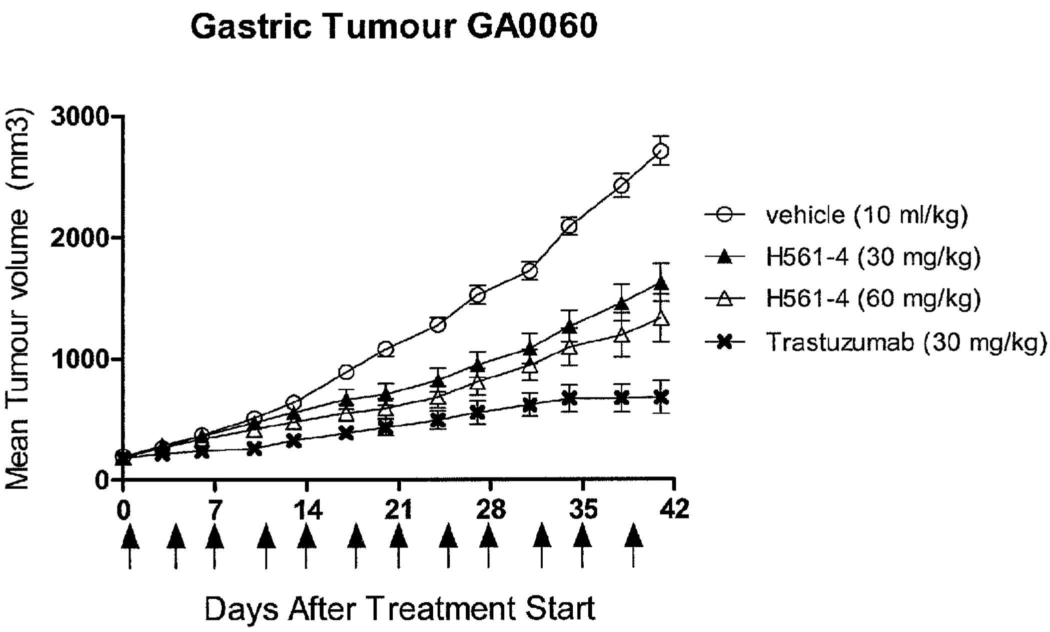


Figure 4 continued

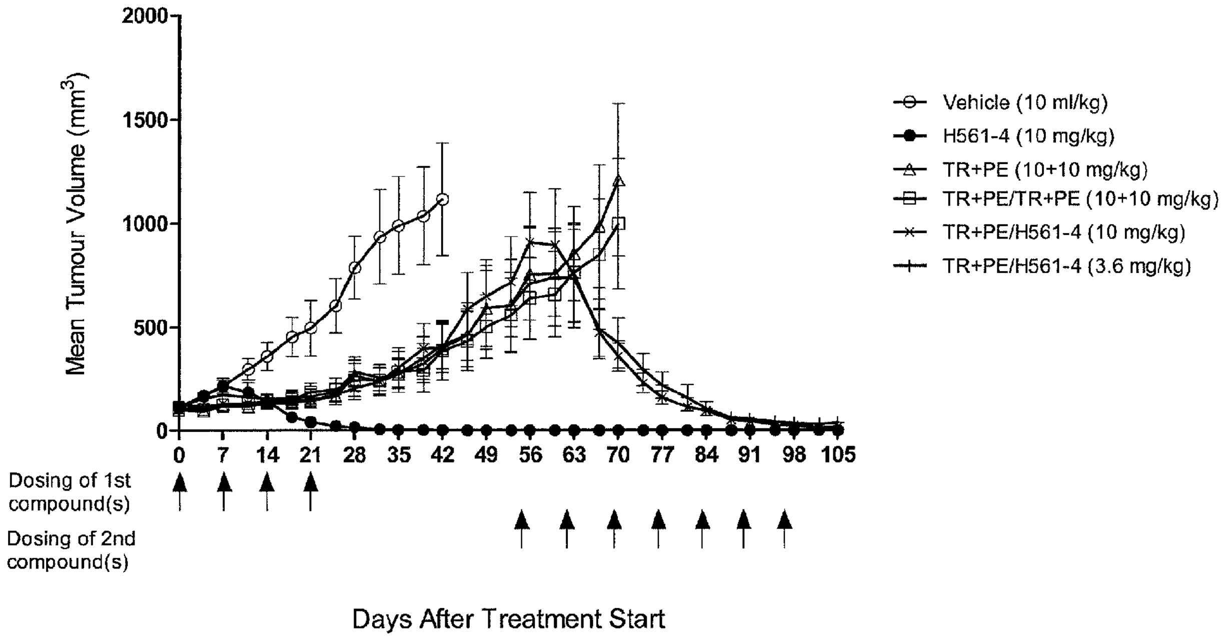
Mean Tumour Volume (mm³)
2000
1500
1000
500
0
0 7 14 21 28 35 42 49 56 63 70 77 84 91 98 105
Dosing of 1st compound(s)
Dosing of 2nd compound(s)
Days After Treatment Start
Vehicle (10 ml/kg)
H561-4 (10 mg/kg)
TR+PE (10+10 mg/kg)
TR+PE/TR+PE (10+10 mg/kg)
TR+PE/H561-4 (10 mg/kg)
TR+PE/H561-4 (3.6 mg/kg)

Figure 5

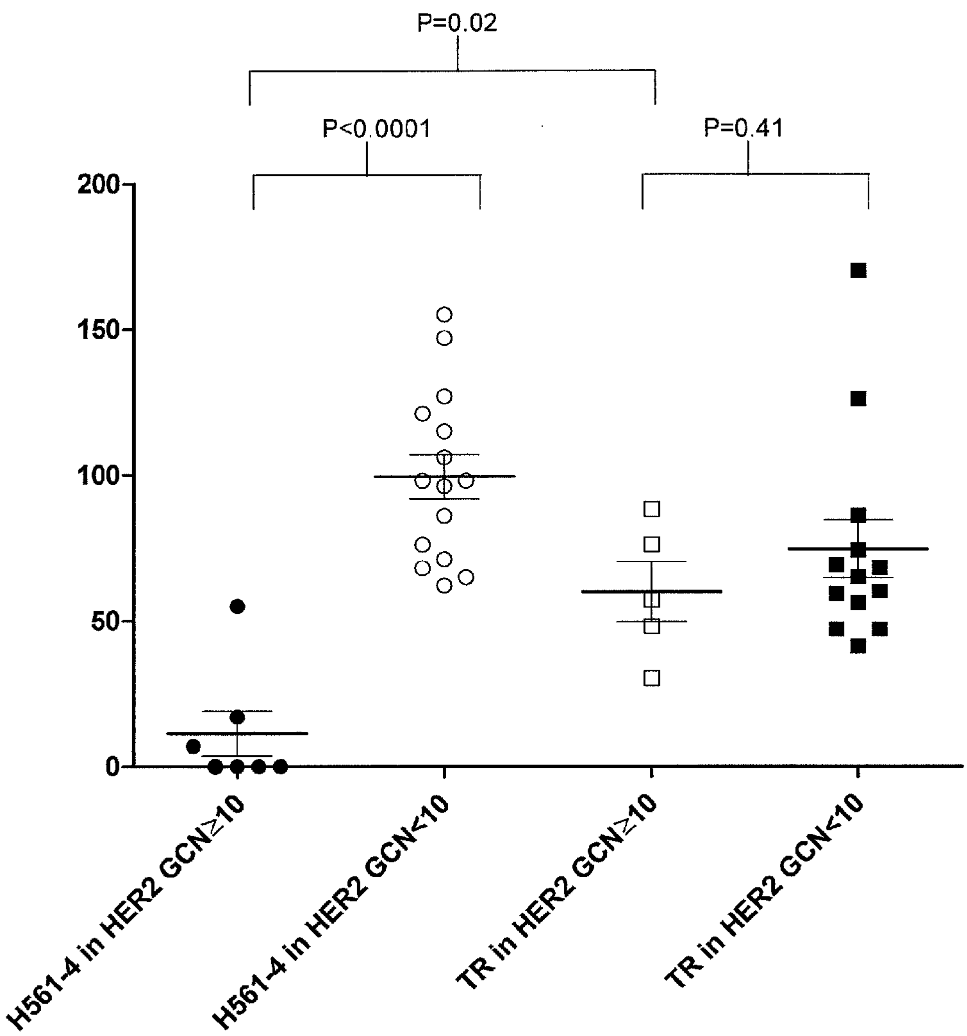
P=0.02
P<0.0001
P=0.41
200
150
100
50
0
H561-4 in HER2 GCN≥10
H561-4 in HER2 GCN<10
TR in HER2 GCN≥10
TR in HER2 GCN<10

Figure 6

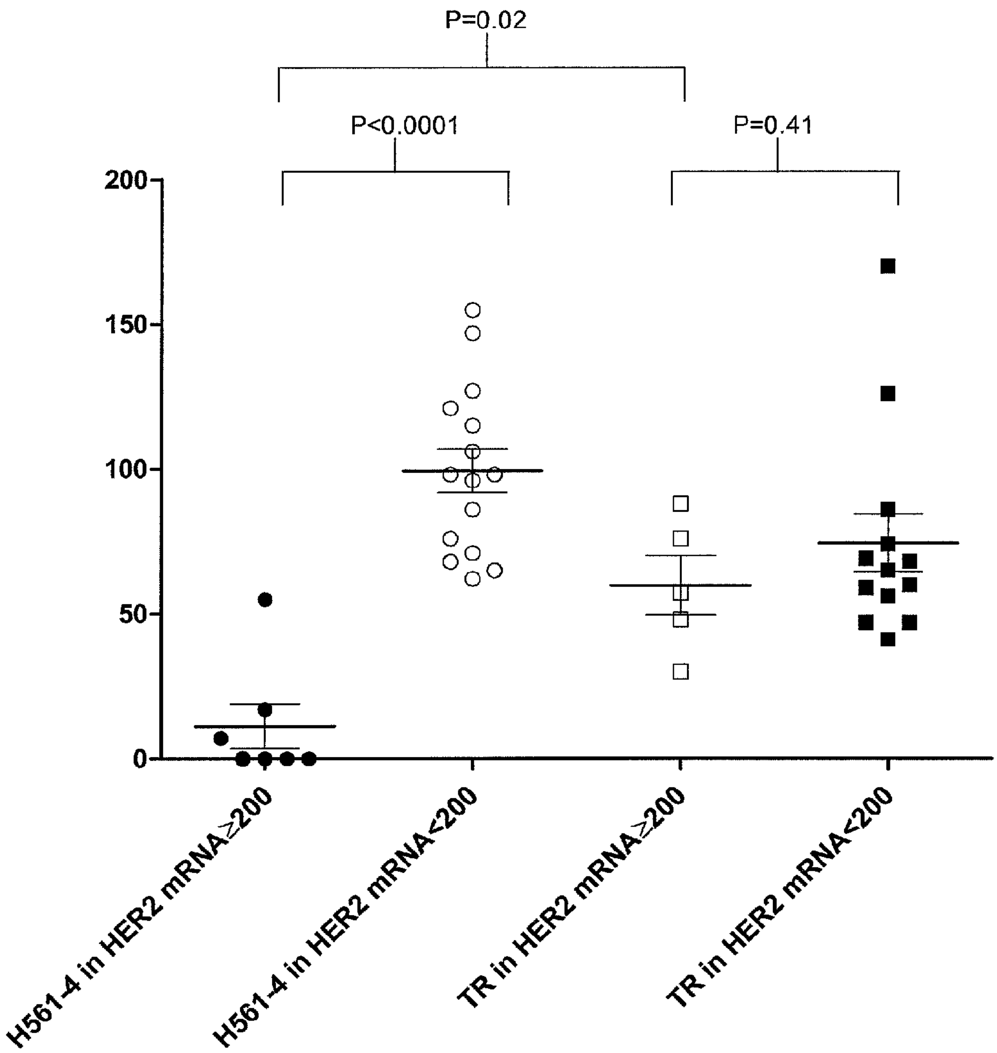
P=0.02
P<0.0001
P=0.41
200
150
100
50
0
H561-4 in HER2 mRNA≥200
H561-4 in HER2 mRNA<200
TR in HER2 mRNA≥200
TR in HER2 mRNA<200

Figure 7

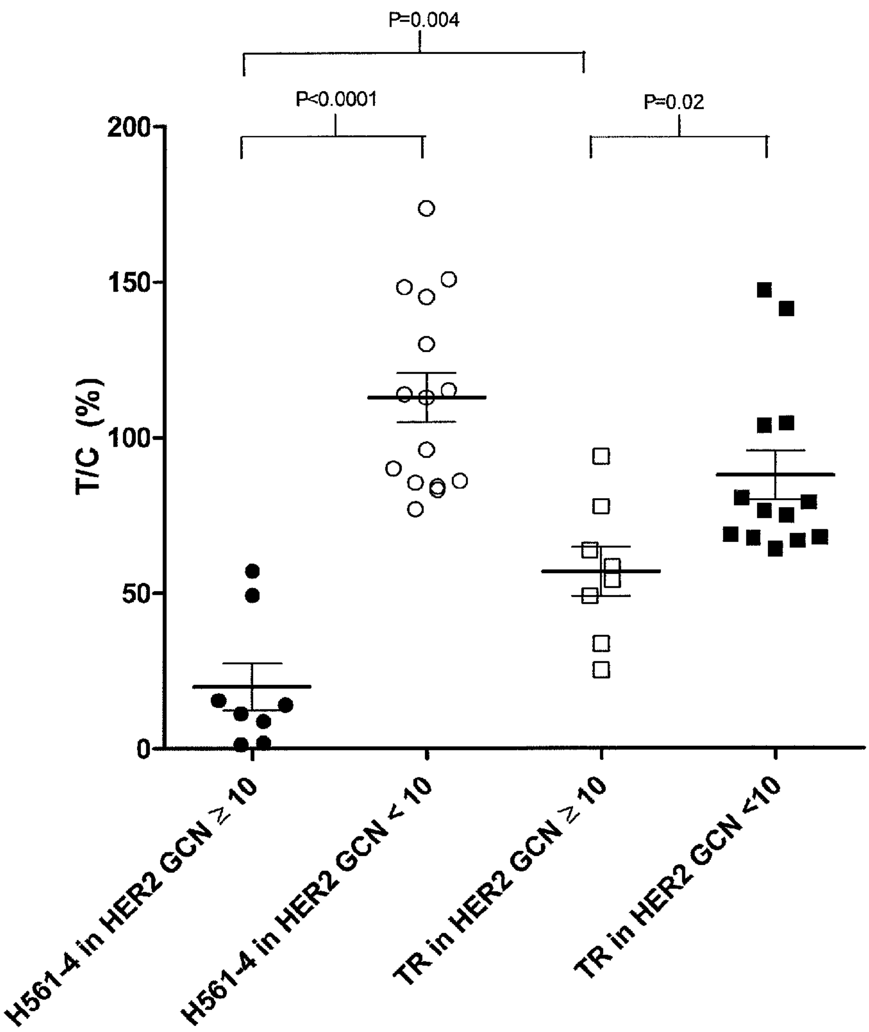
P=0.004
P<0.0001
P=0.02
200
150
100
50
0
T/C (%)
H561-4 in HER2 GCN ≥ 10
H561-4 in HER2 GCN < 10
TR in HER2 GCN ≥ 10
TR in HER2 GCN <10

Figure 8

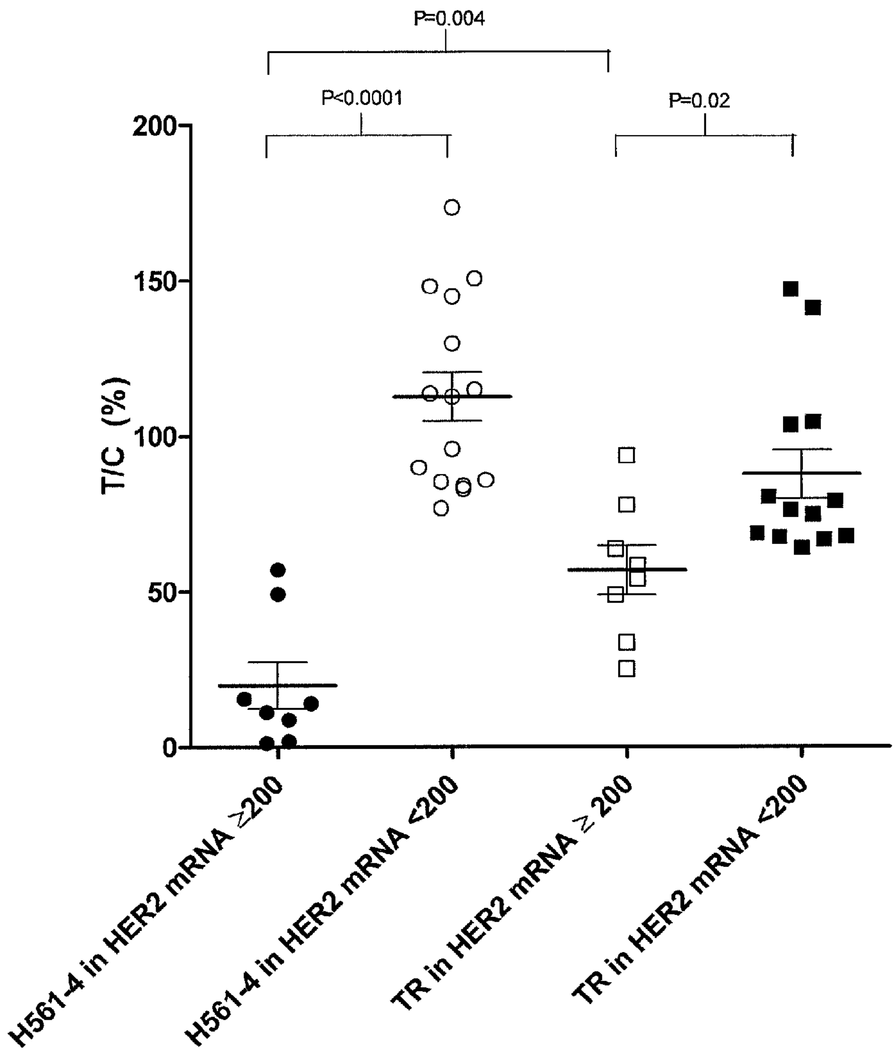
P=0.004
P<0.0001
P=0.02
200
150
100
50
0
T/C (%)
H561-4 in HER2 mRNA ≥200
H561-4 in HER2 mRNA <200
TR in HER2 mRNA ≥ 200
TR in HER2 mRNA <200

Figure 9

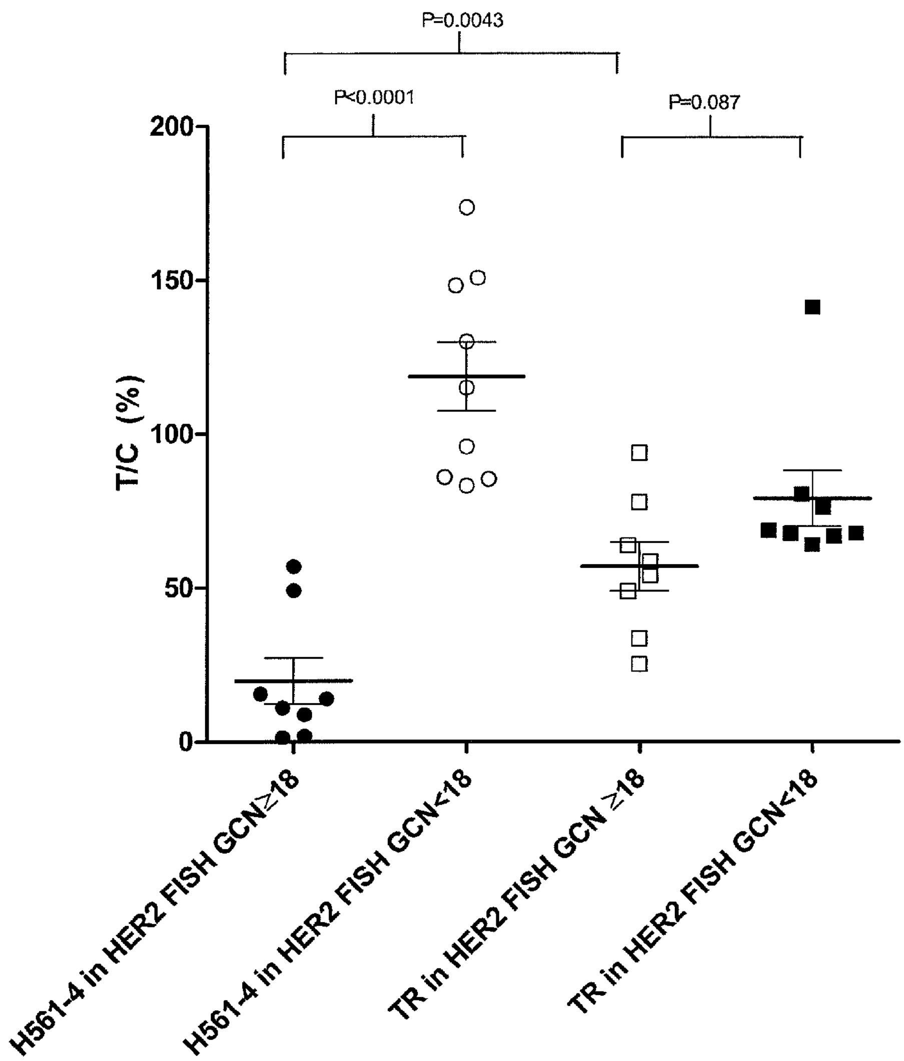
P=0.0043
P<0.0001
P=0.087
200
150
100
50
0
T/C (%)
H561-4 in HER2 FISH GCN≥18
H561-4 in HER2 FISH GCN<18
TR in HER2 FISH GCN ≥18
TR in HER2 FISH GCN<18

Figure 10

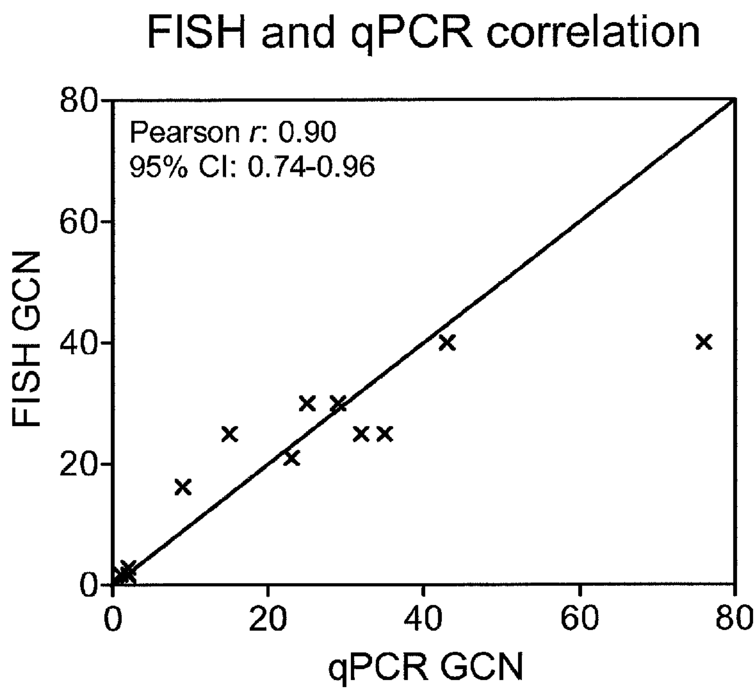
FISH and qPCR correlation
Pearson r: 0.90
95% CI: 0.74-0.96
FISH GCN
qPCR GCN
0
20
40
60
80
0
20
40
60
80

Figure 11

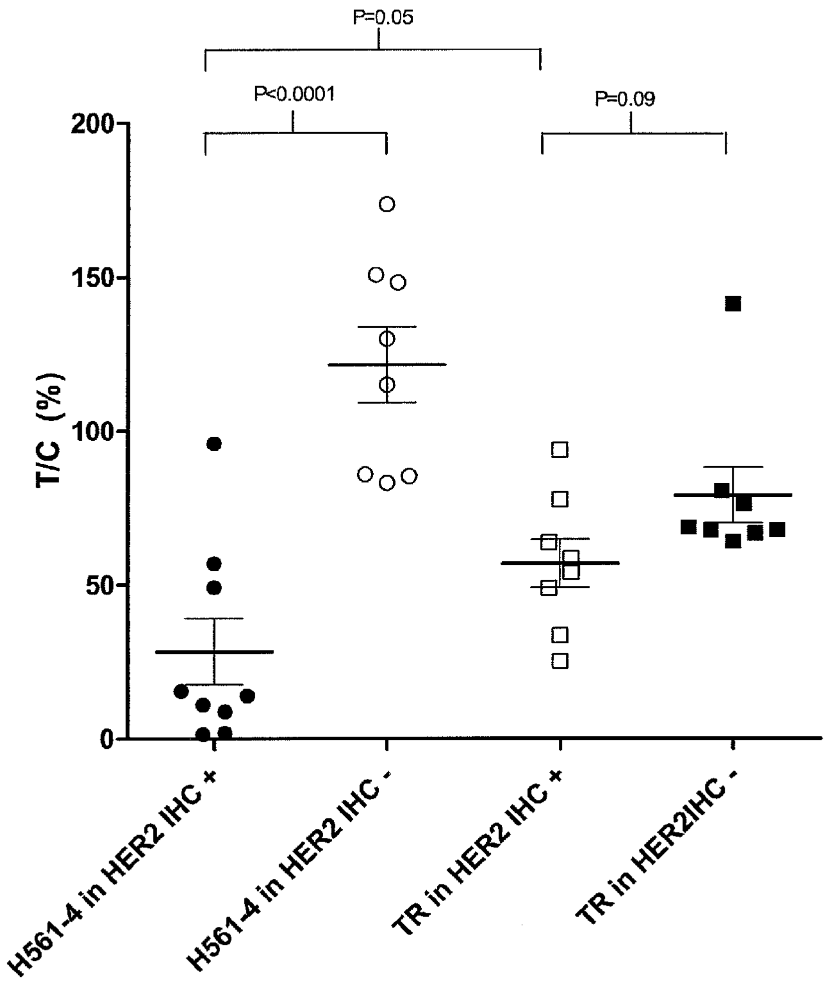
P=0.05
P<0.0001
P=0.09
200
150
100
50
0
T/C (%)
H561-4 in HER2 IHC +
H561-4 in HER2 IHC -
TR in HER2 IHC +
TR in HER2IHC -

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009132876 A1
- WO 2009000006 A1
- US 9209965 W
- WO 9413804 A
- WO 2006072620 A
- WO 2009132876 A
- WO 2006072620 A1
- GB 1317622 A


### Non-patent literature cited in the description


- **BIRD et al.** *Science,* 1988, vol. 242, 423-426
- **HUSTON et al.** *PNAS USA,* 1988, vol. 85, 5879-5883
- **WARD et al.** *Nature,* 1989, vol. 341, 544-546
- **MCCAFFERTY et al.** *Nature,* 1990, vol. 348, 552-554
- **HOLT et al.** *Trends in Biotechnology,* 2003, vol. 21, 484-490
- **HOLLIGER et al.** *Proc. Natl. Acad. Sci. USA,* 1993, vol. 90, 6444-6448
- **KABAT et al.** Sequences of Proteins of Immunological Interest. US Department of Health and Human Services, 1987
- Sustained and Controlled Release Drug Delivery Systems. Marcel Dekker, Inc, 1978
- **LEDERMANN et al.** *Int. J. Cancer,* 1991, vol. 47, 659-664
- **BAGSHAWE et al.** *Antibody, Immunoconjugates and Radiopharmaceuticals,* 1991, vol. 4, 915-922
- **TIMMERMAN et al.** Functional reconstruction and synthetic mimicry of a conformational epitope using CLIPS™ technology. *J MoL Recognit.,* 2007, vol. 20, 283-99
- **SLOOTSTRA et al.** Structural aspects of antibody-antigen interaction revealed through small random peptide libraries. *Molecular Diversity,* 1996, vol. 1, 87-96
- **ARNOULD et al.** Accuracy of HER2 status determination on breast core-needle biopsies (immunohistochemistry, FISH, CISH and SISH vs FISH). *Mod Path.,* 2012, vol. 25, 675-682
- **BECK et al.** Characterization of Therapeutic Antibodies and Related Products. *Anal. Chem.,* 2013, vol. 85, 715-736
- **BERCHUCK, A. et al.** Overexprerssion of HER2/neu in endometrial cancer is associated with advanced stage disease. *AJOG,* 1991, vol. 164 (1), 15-21
- **BRABENDER, J et al.** Epidermal Growth Factor Receptor and HER2-neu mRNA Expression in Non-Small Cell Lung Cancer is Correlated with Survival. *Clin Cancer Res,* 2001, vol. 7, 1850
- CRUK and WHO world cancer report. World Cancer Report. WHO International Agency for Research on Cancer. 2008
- **DOWSETT, M et al.** Disease-free survival according to degree of HER2 amplification for patients treated with adjuvant chemotherapy with or without 1 year of trastuzumab, the HERA trial. *J Clin Oncol,* 2009, vol. 27 (18), 2962-9
- **GARCIA-CABALLERO et al.** Determination of HER2 amplification in primary breast cancer using dual-color chromogenic in situ hybridization is comparable to fluorescence in situ hybridization: a European multicentre study involving 168 specimens. *Histopathology,* 2010, vol. 56, 472-480
- **GRAVALOS, C ; JIMENO, A.** Her2 in gastric cancer: a new prognostic factor and a novel therapeutic target. *Ann Oncol,* 2008, vol. 19 (9), 1523-1529
- **GUN, S et al.** Molecular cytogenetics as a clinical test for prognostic and predictive biomarkers in newly diagnosed ovarian cancer. *J Ovarian Res,* 2013, vol. 6, 2
- **HAMBURG M.A ; COLLINS, F.S.** *The path to personalised medicine,* 15 June 2010
- **HANNA et al.** HER2 in situ hybridization in breast cancer: Clinical implications of polysomy 17 and genetic heterogeneity. *Mod Pathol,* 2014, vol. 27, 4-18
- **HARRIS L et al.** Predictors of Resistance to Preoperative Trastuzumab and Vinorelbine for HER-2 Positive Early Breast Cancer. *Clin Cancer Res,* 2007, vol. 13, 1198-1207
- **HICKS D et al.** Assessment of the HER2 status in breast cancer by Fluorescence In Situ Hybridisation: a technical review with interpretive guidelines. *Hum Pathol,* 2005, vol. 36, 250-261
- **HOFF, E et al.** HER2/neu amplification in breast cancer. *Am J Clin Pathol,* 2002, vol. 117, 916-921

- **HUDIS, C.** Trastuzumab - Mechanism of Action and Use in Clinical Practice. *N Engl J Med,* 2007, vol. 357, 39-51
- **JACOBS, T.W. et al.** Specificity of HercepTest in Determining HER-2/neu Status of Breast Cancers Using the United States Food and Drug Administration-Approved Scoring System. *Journal of Clinical Oncology,* July 1999, vol. 17 (7), 1983-1987
- **LANITIS et al.** Primary Human Ovarian Epithelial Cancer Cells Broadly Express HER2 at Immunologically-Detectable Levels. *PLOSone,* 2012, vol. 7 (11), e49829
- **LEI et al.** Overexpression of HER2/neu oncogene in pancreatic cancer correlated with shortened survival. *International Journal of Pancreatology,* 1995, vol. 17 (1), 15-21
- **MOLLERUP et al.** Dual color chromogenic in situ hybridization for determination of HER2 status in breast cancer: a large comparative study to current state of the art fluorescence in situ hybridization. *BMC Clinical Path.,* 2012, vol. 12, 3
- **OCHS et al.** Expression of Vascular Endothelial Growth Factor and HER2/neu in Stage II Colon Cancer and Correlation with Survival. *Clinical Colorectal Cancer,* 2004, vol. 4 (4), 262-267
- **PERSONS et al.** Quantitation of HER-2/neu and c-myc gene amplification in breast carcinoma using fluorescence in situ hybridization. *Mod Pathol,* 1997, vol. 10, 720-727
- **PRESS et al.** Evaluation of HER-2/neu gene amplification and overexpression: comparison of frequently used assay methods in a molecularly characterized cohort of breast cancer specimens. *JCO,* 2002, vol. 20 (14), 3095-3105
- **RÜSCHOFF et al.** HER2 diagnostics in gastric cancer- guideline validation and development of standardized immunohistochemical testing. *Vichows Arch,* 2010, vol. 457, 299-307
- **ROSS J.** Breast cancer biomarkers and HER2 testing after 10 years of anti-HER2 therapy. *Drug News Perspect,* 2009, vol. 22, 93-106
- **ROSS J.** The HER2/neu Gene and Protein in Breast Cancer 2003: Biomarker and Target of Therapy. *The Oncologist,* 2003, vol. 8, 307-325
- **TIMMERMAN et al.** Functional reconstruction and synthetic mimicry of a conformational epitope using CLIPS™ technology. *J Mol Recognit,* 2007, vol. 20, 283-99
- **VINATZER et al.** Expression of HER2 and the Coamplified Genes GRB7 and MLN64 in Human Breast Cancer: Quantatative Real-time Reverse Transcription PCR as a Diagnostic Alternative to Immunohistochemistry and Fluorescence In situ Hybridisation. *Clin Cancer Res,* 2005, vol. 11 (23), 8348-8357
- **WOLFF A et al.** American Society of Clinical Oncology/College of American Pathologists Guideline Recommendations for Human Epidermal Growth Factors Receptor 2 Testing in Breast Cancer. *J Clin Oncol,* 2007, vol. 25, 118, , 145
- **WOLFF et al.** Recommendations for Human Epidermal Growth Factor Receptor 2 Testing in Breast Cancer: American Society of Clinical Oncology/College of American Pathologists Clinical Practice Guideline Update. *J Clin Onco,* 2013, vol. 31 (31), 3997-4013
- **WONG et al.** Intergrating molecular and clinical evidence in the management of trastuzumab resistant or refractory HER-2+ Metastatic breast cancer. *The Oncologist,* 2011, vol. 16, 1535-1546
- **YARDEN, Y.** Biology of HER2 and Its Importance in Breast Cancer. *Oncology,* 2001, vol. 61 (2), 1-13

SEQUENCE LISTING

<110> F-star Biotechnology Limited
F-star Biotechnologische Forschungs- Und Entwicklungsges
m.b.H

<120> Cancer Biomarkers and Uses Thereof

<130> TEK/CP7067424

<150> GB 1317622.7
<151> 2013-10-04

<160> 19

<170> PatentIn version 3.3

<210> 1
<211> 681
<212> DNA
<213> Artificial sequence

<220>
<223> Synthetic sequence: Nucleotide sequence of antigen-binding Fc fragment H561-4

<400> 1

```
acctgccccc cttgtcctgc ccccgagctg ctgggaggcc cttccgtgtt tctgttcccc      60
ccaaagccca aggacaccct gatgatctcc cggacccccg aagtgacctg cgtggtggtg     120
gacgtgtccc acgaggaccc tgaagtgaag ttcaattggt acgtggacgg cgtggaagtg     180
cacaacgcca agaccaagcc cagagaggaa cagtacaact ccacctaccg ggtggtgtcc     240
gtgctgaccg tgctgcacca ggactggctg aacggcaaag agtacaagtg caaggtgtcc     300
aacaaggccc tgcctgcccc catcgaaaag accatctcca aggccaaggg ccagccccgc     360
gagccccagg tgtacaccct gcccccctagc cgggacgagt tcttcaccta ctgggtgtcc     420
ctgacctgcc tggtcaaggg cttctacccc tccgatatcg ccgtggaatg ggagtccaac     480
ggccagcccg agaacaacta caagaccacc ccccctgtgc tggactccga cggctcattc     540
ttcctgtact ccaagctgac cgtggaccgg cggagatgga ccgccggcaa cgtgttctcc     600
tgcagcgtga tgcacgaggc cctgcacaac cactacaccc agaagtccct gtccctgagc     660
cccggcaagt gatgagaatt c                                               681
```

<210> 2
<211> 18
<212> DNA
<213> Artificial sequence

<220>
<223> Synthetic sequence: Nucleotide sequence of the antigen-binding Fc fragment H561-4 hinge region

<400> 2
acctgccccc cttgtcct 18

<210> 3
<211> 330
<212> DNA
<213> Artificial sequence

<220>
<223> Synthetic sequence: Nucleotide sequence of the antigen-binding Fc fragment H561-4 CH2 domain

<400> 3

```
gcccccgagc tgctgggagg cccttccgtg tttctgttcc ccccaaagcc caaggacacc      60
ctgatgatct cccggacccc cgaagtgacc tgcgtggtgg tggacgtgtc ccacgaggac     120
cctgaagtga agttcaattg gtacgtggac ggcgtggaag tgcacaacgc caagaccaag     180
cccagagagg aacagtacaa ctccacctac cgggtggtgt ccgtgctgac cgtgctgcac     240
caggactggc tgaacggcaa agagtacaag tgcaaggtgt ccaacaaggc cctgcctgcc     300
cccatcgaaa agaccatctc caaggccaag                                      330
```

<210> 4
<211> 345

<212> DNA
<213> Artificial sequence

<220>
<223> Synthetic sequence: Nucleotide sequence of the antigen-binding Fc fragment H561-4 CH3 domain

<400> 4

```
gaaaagacca tctccaaggc caagggccag ccccgcgagc cccaggtgta caccctgccc     60
cctagccggg acgagttctt cacctactgg gtgtccctga cctgcctggt caagggcttc    120
tacccctccg atatcgccgt ggaatgggag tccaacggcc agcccgagaa caactacaag    180
accacccccc ctgtgctgga ctccgacggc tcattcttcc tgtactccaa gctgaccgtg    240
gaccggcgga gatggaccgc cggcaacgtg ttctcctgca gcgtgatgca cgaggccctg    300
cacaaccact acacccagaa gtccctgtcc ctgagccccg gcaag                    345
```

<210> 5
<211> 15
<212> DNA
<213> Artificial sequence

<220>
<223> Synthetic sequence: Nucleotide sequence encoding the part of the H561-4 AB structural loop region believed to be involved in antigen binding

<400> 5
ttcttcacct actgg 15

<210> 6
<211> 15
<212> DNA
<213> Artificial sequence

<220>
<223> Synthetic sequence: Nucleotide sequence encoding part of the H561-4 CD structural loop region

<400> 6
aacggccagc ccgag 15

<210> 7
<211> 21
<212> DNA
<213> Artificial sequence

<220>
<223> Synthetic sequence: Nucleotide sequence encoding the part of the H561-4 EF structural loop region believed to be involved in antigen binding

<400> 7
gaccggcgga gatggaccgc c 21

<210> 8
<211> 223
<212> PRT

<213> Artificial sequence

<220>
<223> Synthetic sequence: Amino acid sequence of antigen-binding Fc fragment H561-4

<400> 8

```
Thr Cys Pro Pro Cys Pro Ala Pro Glu Leu Leu Gly Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Pro Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Phe Phe Thr Tyr Trp Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Arg Arg Arg
            180                 185                 190

Trp Thr Ala Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220
```

<210> 9
<211> 6
<212> PRT
<213> Artificial sequence

<220>
<223> Synthetic sequence: Amino acid sequence of the H561-4 hinge region

<400> 9

```
Thr Cys Pro Pro Cys Pro
1               5
```

<210> 10
<211> 110
<212> PRT
<213> Artificial sequence

<220>
<223> Synthetic sequence: Amino acid sequence of the H561-4 CH2 domain

<400> 10

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110
```

<210> 11
<211> 107
<212> PRT
<213> Artificial sequence

<220>
<223> Synthetic sequence: Amino acid sequence of the H561-4 CH3 domain

<400> 11

```
Gly Gln Pro Arg Glu Pro Gln Val Tyr Thr Leu Pro Pro Ser Arg Asp
1               5                   10                  15

Glu Phe Phe Thr Tyr Trp Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Arg Arg Arg Trp Thr Ala Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105
```

<210> 12
<211> 5
<212> PRT
<213> Artificial sequence

<220>
<223> Synthetic sequence: Amino acid sequence of the part of the H561-4 AB structural loop region believed to be involved in antigen binding

<400> 12

```
Phe Phe Thr Tyr Trp
1               5
```

<210> 13
<211> 5
<212> PRT
<213> Artificial sequence

<220>
<223> Synthetic sequence: Amino acid sequence of part of the H561-4 CD structural loop region

<400> 13

```
Asn Gly Gln Pro Glu
1               5
```

<210> 14
<211> 7
<212> PRT
<213> Artificial sequence

<220>
<223> Synthetic sequence: Amino acid sequence of the part of the H561-4 EF structural loop region believed to be involved in antigen binding

<400> 14

Asp Arg Arg Arg Trp Thr Ala
1               5

<210> 15
<211> 15
<212> PRT
<213> Homo sapiens

<400> 15

Leu Pro Ala Ser Pro Glu Thr His Leu Asp Met Leu Arg His Leu
1               5                   10                  15

<210> 16
<211> 15
<212> PRT
<213> Homo sapiens

<400> 16

Cys Gln Val Val Gln Gly Asn Leu Glu Leu Thr Tyr Leu Pro Thr
1               5                   10                  15

<210> 17
<211> 52
<212> PRT
<213> Homo sapiens

<400> 17

Ser Leu Thr Leu Gln Gly Leu Gly Ile Ser Trp Leu Gly Leu Arg Ser
1               5                   10                  15

Leu Arg Glu Leu Gly Ser Gly Leu Ala Leu Ile His His Asn Thr His
            20                  25                  30

Leu Cys Phe Val His Thr Val Pro Trp Asp Gln Leu Phe Arg Asn Pro
        35                  40                  45

His Gln Ala Leu
    50

<210> 18
<211> 630
<212> PRT
<213> Homo sapiens

<400> 18

```
Thr Gln Val Cys Thr Gly Thr Asp Met Lys Leu Arg Leu Pro Ala Ser
1               5                   10                  15

Pro Glu Thr His Leu Asp Met Leu Arg His Leu Tyr Gln Gly Cys Gln
            20                  25                  30

Val Val Gln Gly Asn Leu Glu Leu Thr Tyr Leu Pro Thr Asn Ala Ser
        35                  40                  45

Leu Ser Phe Leu Gln Asp Ile Gln Glu Val Gln Gly Tyr Val Leu Ile
    50                  55                  60

Ala His Asn Gln Val Arg Gln Val Pro Leu Gln Arg Leu Arg Ile Val
65                  70                  75                  80

Arg Gly Thr Gln Leu Phe Glu Asp Asn Tyr Ala Leu Ala Val Leu Asp
                85                  90                  95

Asn Gly Asp Pro Leu Asn Asn Thr Thr Pro Val Thr Gly Ala Ser Pro
            100                 105                 110

Gly Gly Leu Arg Glu Leu Gln Leu Arg Ser Leu Thr Glu Ile Leu Lys
        115                 120                 125
```

```
Gly Gly Val Leu Ile Gln Arg Asn Pro Gln Leu Cys Tyr Gln Asp Thr
    130                 135                 140

Ile Leu Trp Lys Asp Ile Phe His Lys Asn Asn Gln Leu Ala Leu Thr
145                 150                 155                 160

Leu Ile Asp Thr Asn Arg Ser Arg Ala Cys His Pro Cys Ser Pro Met
                165                 170                 175

Cys Lys Gly Ser Arg Cys Trp Gly Glu Ser Ser Glu Asp Cys Gln Ser
            180                 185                 190

Leu Thr Arg Thr Val Cys Ala Gly Gly Cys Ala Arg Cys Lys Gly Pro
        195                 200                 205

Leu Pro Thr Asp Cys Cys His Glu Gln Cys Ala Ala Gly Cys Thr Gly
    210                 215                 220

Pro Lys His Ser Asp Cys Leu Ala Cys Leu His Phe Asn His Ser Gly
225                 230                 235                 240

Ile Cys Glu Leu His Cys Pro Ala Leu Val Thr Tyr Asn Thr Asp Thr
                245                 250                 255

Phe Glu Ser Met Pro Asn Pro Glu Gly Arg Tyr Thr Phe Gly Ala Ser
            260                 265                 270

Cys Val Thr Ala Cys Pro Tyr Asn Tyr Leu Ser Thr Asp Val Gly Ser
        275                 280                 285

Cys Thr Leu Val Cys Pro Leu His Asn Gln Glu Val Thr Ala Glu Asp
    290                 295                 300

Gly Thr Gln Arg Cys Glu Lys Cys Ser Lys Pro Cys Ala Arg Val Cys
305                 310                 315                 320

Tyr Gly Leu Gly Met Glu His Leu Arg Glu Val Arg Ala Val Thr Ser
                325                 330                 335

Ala Asn Ile Gln Glu Phe Ala Gly Cys Lys Lys Ile Phe Gly Ser Leu
            340                 345                 350

Ala Phe Leu Pro Glu Ser Phe Asp Gly Asp Pro Ala Ser Asn Thr Ala
        355                 360                 365

Pro Leu Gln Pro Glu Gln Leu Gln Val Phe Glu Thr Leu Glu Glu Ile
    370                 375                 380
```

```
Thr Gly Tyr Leu Tyr Ile Ser Ala Trp Pro Asp Ser Leu Pro Asp Leu
385                 390                 395                 400

Ser Val Phe Gln Asn Leu Gln Val Ile Arg Gly Arg Ile Leu His Asn
                405                 410                 415

Gly Ala Tyr Ser Leu Thr Leu Gln Gly Leu Gly Ile Ser Trp Leu Gly
            420                 425                 430

Leu Arg Ser Leu Arg Glu Leu Gly Ser Gly Leu Ala Leu Ile His His
        435                 440                 445

Asn Thr His Leu Cys Phe Val His Thr Val Pro Trp Asp Gln Leu Phe
    450                 455                 460

Arg Asn Pro His Gln Ala Leu Leu His Thr Ala Asn Arg Pro Glu Asp
465                 470                 475                 480

Glu Cys Val Gly Glu Gly Leu Ala Cys His Gln Leu Cys Ala Arg Gly
                485                 490                 495

His Cys Trp Gly Pro Gly Pro Thr Gln Cys Val Asn Cys Ser Gln Phe
            500                 505                 510

Leu Arg Gly Gln Glu Cys Val Glu Glu Cys Arg Val Leu Gln Gly Leu
        515                 520                 525

Pro Arg Glu Tyr Val Asn Ala Arg His Cys Leu Pro Cys His Pro Glu
    530                 535                 540

Cys Gln Pro Gln Asn Gly Ser Val Thr Cys Phe Gly Pro Glu Ala Asp
545                 550                 555                 560

Gln Cys Val Ala Cys Ala His Tyr Lys Asp Pro Pro Phe Cys Val Ala
                565                 570                 575

Arg Cys Pro Ser Gly Val Lys Pro Asp Leu Ser Tyr Met Pro Ile Trp
            580                 585                 590

Lys Phe Pro Asp Glu Glu Gly Ala Cys Gln Pro Cys Pro Ile Asn Cys
        595                 600                 605

Thr His Ser Cys Val Asp Leu Asp Asp Lys Gly Cys Pro Ala Glu Gln
    610                 615                 620

Arg Ala Ser Pro Leu Thr
625                 630
```

<210> 19
<211> 223
<212> PRT
<213> Homo sapiens

<400> 19

```
Thr Cys Pro Pro Cys Pro Ala Pro Glu Leu Leu Gly Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Pro Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220
```